# FASCICULE DE BREVET EUROPEEN

(11) **EP 2 036 892 B1**
(45) Date de publication et mention de la délivrance du brevet: **10.03.2010**
(21) Numéro de dépôt: 08290851.8
(22) Date de dépôt: 11.09.2008
(51) Int. Cl.: C07D 223/16, A61K 31/55, A61P 9/00

(54) **Dérivés de 1,2,4,5-tétrahydro-3H-benzazépines, leur procédé de préparation et les compositions pharmaceutiques qui les contiennent**
1,2,4,5-Tetrahydro-3H-Benzazepinderivate, Verfahren zu ihrer Herstellung und diese enthaltende pharmazeutische Zusammensetzungen
1,2,4,5-tetrahydro-3H-benzazepine derivatives, process for their preparation and pharmaceutical compositions containing them

(30) Priorité: 11.09.2007 FR 0706346
(43) Date de publication de la demande: 18.03.2009
(73) Titulaire: Les Laboratoires Servier, 92415 Courbevoie Cedex (FR)
(72) Inventeur: Peglion, Jean-Louis, 78110 Le Vesinet (FR); Goument, Bertrand, 78220 Viroflay (FR); Dessinges, Aimée, 92500 Rueil-Malmaison (FR); Caignard, Pascal, 93800 Epinay-sur-Seine (FR); Vilaine, Jean-Paul, 92290 Chatenay Malabry (FR); Thollon, Catherine, 75011 Paris (FR); Villeneuve, Nicole, 92500 Rueil Malmaison (FR); Chimenti, Stefano, 75011 Paris (FR)
(74) Mandataire: Giudicelli, Cathy

(56) Documents cités:
- EP-A- 0 161 604
- EP-A- 0 534 859
- WO-A-2006/092491
- US-B1- 6 514 964

## Description

La présente invention a pour objet de nouveaux dérivés de 1,2,4,5-tétrahydro-3*H-*benzazépines, leur procédé de préparation et les compositions pharmaceutiques qui les contiennent.

Les composés de l'invention bloquent les canaux "HCN, hyperpolarization-activated cyclic nucleotide-gated channels".

Le blocage des canaux HCN induit par les dérivés de l'invention permet l'utilisation de ceux-ci dans les traitements curatifs ou préventifs de toutes les pathologies dans lesquelles l'activité ou l'expression des canaux HCN est exacerbée et anormale.

Ces composés de l'invention peuvent notamment être utiles dans le traitement curatif ou préventif des douleurs, en particulier des douleurs neuropathiques et inflammatoires (Chaplan SR, Guo HQ, Lee DH, Luo L, Liu C, Kuei C, Velumian AA, Butler MP, Brown SM, Dubin AE. Neuronal hyperpolarization-activated pacemaker channels drive neuropathic pain. J Neurosci 2003; 23(4):1169-78*;* Luo L, Chang L, Brown SM, Ao H, Lee DH, Higuera ES, Dubin AE, Chaplan SR. Role of peripheral hyperpolarization-activated cyclic nucleotide-modulated channel pacemaker channels in acute and chronic pain models in the rat. Neuroscience 2007; 144(4):1477-85*;* Jiang YQ, Sun Q, Tu HY, Wan Y Characteristics of HCN Channels and Their Participation in Neuropathic Pain. Neurochem Res. 2008 May 7), de la vessie hyperactive (Stamatiou K, Heretis I, Skoumbourdis E. Does ivabradine exhibit a role in the reduction of bladder overactivity? Int Urol Nephrol. 2008 Feb 1*.* ), et de la sensation de sécheresse oculaire (Ingram SL, Williams JT. Modulation of the hyperpolarization-activated current (Ih) by cyclic nucleotides in guinea-pig primary afferent neurons. J Physiol. 1996; 492:97-106*;* Belmonte C. Eye dryness sensations after refractive surgery: impaired tear secretion or "phantom" cornea? J Refract Surg 2007; 23(6):598-602*).*

Les composés de l'invention réduisent de façon directe et sélective l'activité pacemaker cardiaque.

La réduction sélective de la fréquence cardiaque induite par les dérivés de l'invention permet l'utilisation de ceux ci à titre curatif ou préventif dans les différentes pathologies dans lesquelles une accélération de la fréquence cardiaque joue un rôle déclenchant ou aggravant. Ces produits peuvent en particulier améliorer le traitement et le pronostic à long terme des cardiopathies ischémiques (Dyer AR, Persky V, Stamler J, et al. Heart rate as a prognostic factor for coronary heart disease and mortality: findings in three Chicago epidemiologic studies. Am J Epidemiol. 1980; 112: 736-749*;* Kannel WB, Kannel C, Paffengarger RS Jr, et al. Heart rate and cardiovascular mortality: the Framingham Study. Am Heart J. 1987; 113: 1489-1494*;* Gillum RF, Makuc DM, Feldman JJ. Pulse rate, coronary heart disease, and death: the NHANES I epidemiologic follow-up study. Am Heart J. 1991; 121: 172-177*;* Greenland P, Daviglus ML, Dyer AR, et al. Resting heart rate is a risk factor for cardiovascular and noncardiovascular mortality: the Chicago Heart Association Detection Project in Industry. Am J Epidemiol 1999; 149: 853-862*;* Kristal-Boneh E, Silber H, Harari G, et al. The association of resting heart rate with cardiovascular, cancer and all-cause mortality. Eight year follow-up of 3527 male Israeli employees (the CORDIS Study). Eur Heart J 2000; 21: 116-124*;* Palatini P. Heart rate as a cardiovascular risk factor: do women differ from men? Ann Med. 2001; 33: 213-221*;* Aronow WS, Ahn C, Mercando AD, et al. Association of average heart rate on 24-hour ambulatory electrocardiograms with incidence of new coronary events at 48-month follow-up in 1,311 patients (mean age 81 years) with heart disease and sinus rhythm. Am J Cardiol. 1996; 78: 1175-1176*)* dans leurs différentes expressions cliniques : angor stable (Borer JS, Fox K, Jaillon P, et al. Antianginal and antiischemic effects of ivabradine, an If inhibitor, in stable angina. A randomized, double-blind, multicentered, placebo-controlled trial. Circulation 2003;107: 817-23*;* Diaz A, Bourassa MG, Guertin MC, Tardif JC: Long-term prognostic value of resting heart rate in patients with suspected or proven coronary artery disease. Eur Heart J 2005; 26: 967-974*),* angor instable, infarctus du myocarde (Hjalmarson A, Gilpin EA, Kjekshus J, Schieman G, Nicod P, Henning H, Ross J Jr: Influence of heart rate on mortality after acute myocardial infarction. Am J Cardiol 1990; 65: 547-553*;* Disegni E, Goldbourt U, Reicher-Reiss H, Kaplinsky E, Zion M, Boyko V, Behar S: The predictive value of admission heart rate on mortality in patients with acute myocardial infarction. SPRINT Study Group. Secondary Prevention Reinfarction Israeli Nifedipine Trial. J Clin Epidemiol 1995; 48:1197-1205*;* Zuanetti G, Mantini L, Hernandez-Bernal F, Barlera S, di Gregorio D, Latini R, Maggioni AP: Relevance of heart rate as a prognostic factor in patients with acute myocardial infarction: insights from the GISSI-2 study. Eur Heart J 1998;19(suppl F):F19-F26*;* Lee KL, Woodlief LH, Topol EJ, Weaver WD, Betriu A, Col J, Simoons M, Aylward P, Van de Werf F, Califf RM: Predictors of 30-day mortality in the era of reperfusion for acute myocardial infarction. Results from an international trial of 41, 021 patients. GUSTO-I Investigators. Circulation 1995; 91:1659-1668*;* Steffenino G, Santoro GM, Maras P, Mauri F, Ardissino D, Violini R, Chiarella F, Lucci D, Marini M, Baldasseroni S, Maggioni AP: In-hospital and one-year outcomes of patients with high-risk acute myocardial infarction treated with thrombolysis or primary coronary angioplasty. Ital Heart J 2004; 5:136-145*;* Mauss O, Klingenheben T, Ptaszynski P, Hohnloser SH: Bedside risk stratification after acute myocardial infarction: prospective evaluation of the use of heart rate and left ventricular function. J Electrocardiol 2005; 38:106-112*),* post infarctus; des insuffisances cardiaques tant systolique que diastolique *(*Aaronson KD, Schwartz JS, Chen TM, Wong KL, Goin JE, Mancini DM: Development and prospective validation of a clinical index to predict survival in ambulatory patients referred for cardiac transplant evaluation. Circulation 1997; 95:2660-2667*;* CIBIS-II Investigators and Committees: The cardiac Insufficiency Bisoprolol Study II (CIBIS-II): a randomised trial. Lancet 1999; 353:9-13*;* Mulder P, Barbier S, Chagraoui A, et al. Long-term heart rate reduction induced by the selective If current inhibitor ivabradine improves left ventricular function and intrinsic myocardial structure in congestive heart failure. Circulation 2004; 109: 1674-9); des troubles du rythme ventriculaires ou supra ventriculaires (James R, Arnold J, Allen J, et al. The effects of heart rate, myocardial ischemia and vagal stimulation on the threshold of ventricular fibrillation. Circulation. 1977; 55: 311-7*;* Bernier M, Curtis JM, Hearse DJ. Ischemia-induced and reperfusion-induced arrhythmias: importance of heart rate. Am J Physiol 1989; 256: H21-H31), des pathologies constituant un facteur de risque vasculaire : hypertension artérielle, diabète, hypercholestérolémie, en réduisant notamment le développement des lésions d'athéroscléroses et leurs complications (Beere PA, Glagov S, Zarins CK. Retarding effect of lowered heart rate on coronary atherosclerosis. Science 1984; 226: 180-2*;* Kaplan JR, Manuck SB, Clarkson TB. The influence of heart rate on coronary atherosclerosis. J Cardiovasc Pharmacol 1987; 10: S100-S102*;* Beere PA, Glagov S, Zarins CK Experimental atherosclerosis at the carotid bifurcation of the cynomolgus monkey. Localization, compensatory enlargement, and the sparing effect of lowered heart rate. Atheroscler Thromb 1992; 12, 1245-53*;* Perski A, Hamsten A, Linvall K et al. Heart rate correlates with severity of coronary artery atherosclerosis in young postinfarction patients. Am Heart J 1988; 116: 1369-73; Perski A Ollson G, Landou C et al. Minimum heart rate and coronary atherosclerosis: Independent relations to global severity and rate of progression of angiographic lesions in men with myocardial infarction at a young age. Am Heart J 1992; 123: 609-16; Heidland UE, Strauer BE. Left ventricular muscle mass and elevated heart rate are associated with coronary plaque disruption. Circulation. 2001; 104:1477-82).

Des composés réduisant la fréquence cardiaque de façon directe et sélective sont notamment connus de la demande EP 0 534 859.

Des dérivés de benzazépines ont été décrits dans la demande EP 0 161 604 pour le traitement d'affections cardiovasculaires et dans le brevet US 6,514,964 pour le traitement d'affections en relation avec les récepteurs des intégrines.

Le problème de la présente invention était d'obtenir de nouveaux produits réduisant la fréquence cardiaque, à la fois puissants, sélectifs et sûrs d'emploi.
A cet égard, il est intéressant d'avoir des composés présentant peu de risques d'interaction médicamenteuse.

La présente invention concerne plus spécialement les composés de formule (I) : 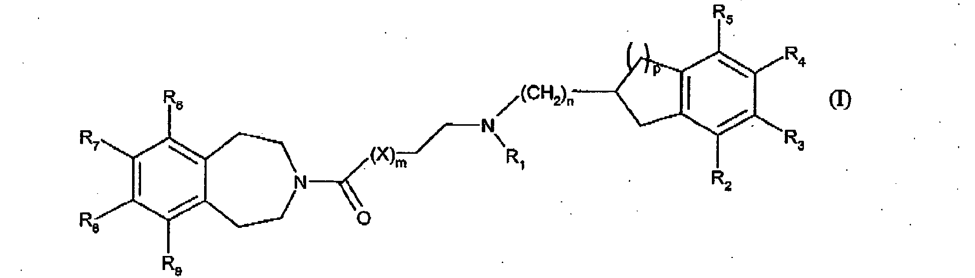 dans laquelle:
- R₁ représente un atome d'hydrogène ou un groupement choisi parmi cycloalkyle C₃-C₇, benzyle et alkyle C₁-C₆ linéaire ou ramifié, le groupement alkyle étant saturé ou insaturé et éventuellement substitué par un groupement hydroxy, cycloalkyle C₃-C₇ ou par un ou plusieurs atomes d'halogène,
- R₂, R₃, R₄ et R₅, identiques ou différents, représentent chacun un atome d'hydrogène ou un groupement hydroxy, méthyle, -OSO₂R₁₀, -OCOR₁₀, alkoxy C₁-C₆ linéaire ou ramifié saturé ou insaturé éventuellement substitué par un groupement méthoxy ou -(CO)-N R₁₂R'₁₂, ou bien R₂ et R₃, ou R₃ et R₄, ou R₄ et R₅ forment ensemble un groupement -O-(CH₂)_{q}-O-, -O-CH=CH-O- ou -O-CH=CH-,
- R₆, R₇, R₈ et R₉, identiques ou différents, représentent chacun un atome d'hydrogène ou un groupement alkoxy C₁-C₆ linéaire ou ramifié saturé ou insaturé, ou bien R₆ et R₇, ou R₇ et R₈, ou R₈ et R₉ forment ensemble un groupement -0-(CH₂)_{q}-O-,
- R₁₀ représente un groupement choisi parmi alkoxy C₁-C₆ linéaire ou ramifié, NR₁₁R'₁₁, et alkyle C₁-C₆ linéaire ou ramifié éventuellement substitué par un ou plusieurs atomes d'halogène,
- R₁₁ et R'₁₁, identiques ou différents, représentent chacun un atome d'hydrogène ou un groupement alkyle C₁-C₆ linéaire ou ramifié, ou bien R₁₁ et R'₁₁ forment ensemble, avec l'atome d'azote qui les porte, un hétérocycle azoté mono-ou bicyclique comportant de 5 à 8 chaînons et comportant éventuellement un autre hétéroatome choisi parmi O et N, ledit hétérocycle étant éventuellement substitué par un ou plusieurs atomes d'halogène,
- R₁₂ et R'₁₂, identiques ou différents, représentent chacun un atome d'hydrogène ou un groupement alkyle C₁-C₆ linéaire ou ramifié,
- X représente O, NH ou CH₂,
- m et p, identiques ou différents, représentent chacun 0 ou 1,
- n et q, identiques ou différents, représentent chacun 1 ou 2,
leurs isomères optiques lorsqu'ils existent, ainsi que leurs sels d'addition à un acide pharmaceutiquement acceptable.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, oxalique, méthanesulfonique, benzènesulfonique, camphorique, pamoïque, 1,5-naphtalènedisulfonique.

Un aspect de la présente invention concerne les composés de formule (I) pour lesquels R₁ représente un atome d'hydrogène ou un groupement alkyle C₁-C₆ linéaire ou ramifié, leurs isomères optiques lorsqu'ils existent, ainsi que leurs sels d'addition à un acide pharmaceutiquement acceptable.

Un autre aspect de l'invention concerne les composés de formule (I) pour lesquels R₁ représente un groupement cycloalkyle C₃-C₇ ou cycloalkylalkyle dans lequel la partie cycloalkyle comprend de 3 à 7 atomes de carbone et la partie alkyle comprend de 1 à 6 atomes de carbone et est linéaire ou ramifié, leurs isomères optiques lorsqu'ils existent, ainsi que leurs sels d'addition à un acide pharmaceutiquement acceptable.

Un autre aspect de l'invention concerne les composés de formule (I) pour lesquels R₂, R₃, R₄ et R₅, identiques ou différents, représentent chacun un atome d'hydrogène ou un groupement alkoxy C₁-C₆ linéaire ou ramifié saturé ou insaturé ou -OCOR₁₀ dans lequel R₁₀ représente un groupement NR₁₁R'₁₁ tel que défini précédemment, leurs isomères optiques lorsqu'ils existent, ainsi que leurs sels d'addition à un acide pharmaceutiquement acceptable.

R₁₁ et R'₁₁ représentent chacun préférentiellement un groupement alkyle C₁-C₆ linéaire ou ramifié.

Un autre aspect de l'invention concerne les composés de formule (I) pour lesquels R₆, R₇, R₈ et R₉, identiques ou différents, représentent chacun un atome d'hydrogène ou un groupement alkoxy C₁-C₆ linéaire ou ramifié saturé ou insaturé, leurs isomères optiques lorsqu'ils existent, ainsi que leurs sels d'addition à un acide pharmaceutiquement acceptable.

Un autre aspect de l'invention concerne les composés de formule (I) pour lesquels m représente 0, leurs isomères optiques lorsqu'ils existent, ainsi que leurs sels d'addition à un acide pharmaceutiquement acceptable.

Un autre aspect de l'invention concerne les composés de formule (I) pour lesquels m représente 1 et X représente CH₂, leurs isomères optiques lorsqu'ils existent, ainsi que leurs sels d'addition à un acide pharmaceutiquement acceptable.

Un autre aspect de l'invention concerne les composés de formule (I) pour lesquels p représente 0, leurs isomères optiques, ainsi que leurs sels d'addition à un acide pharmaceutiquement acceptable.

Un autre aspect de l'invention concerne les composés de formule (I) pour lesquels p représente 1, leurs isomères optiques lorsqu'ils existent, ainsi que leurs sels d'addition à un acide pharmaceutiquement acceptable.

Un autre aspect de l'invention concerne les composés pour lesquels R₁ représente un atome d'hydrogène ou un groupement alkyle C₁-C₆ linéaire ou ramifié, R₂, R₃, R₄ et R₅, identiques ou différents, représentent chacun un atome d'hydrogène ou un groupement alkoxy C₁-C₆ linéaire ou ramifié saturé ou insaturé, R₆, R₇, R₈ et R₉, identiques ou différents, représentent chacun un atome d'hydrogène ou un groupement alkoxy C₁-C₆ linéaire ou ramifié saturé ou insaturé, m représente 0, n représente 1 et p représente 0, leurs isomères optiques, ainsi que leurs sels d'addition à un acide pharmaceutiquement acceptable.

Un autre aspect de l'invention concerne les composés suivants :
- la N-{[3,4-diméthoxybicyclo[4.2.0]octa-1,3,5-trién-7-yl]méthyl} 3-(7,8-diméthoxy-1,2,4,5-tétrahydro-3H-3-benzazépin-3-yl) N-méthyl-3-oxopropan-1-amine, ses isomères optiques, ainsi que ses sels d'addition à un acide pharmaceutiquement acceptable;
- la N-{[3,4-diméthoxybicyclo[4.2.0]octa-1,3,5-trién-7-yl]méthyl}-3-(7,8-diméthoxy-1,2,4,5-tétrahydro-3H-3-benzazépin-3-yl)-3-oxopropan-1-amine, ses isomères optiques, ainsi que ses sels d'addition à un acide pharmaceutiquement acceptable;
- la N-[2-(5,6-diméthoxy-2,3-dihydro-1*H*-indén-2-yl)méthyl]-3-(7,8-diméthoxy-1,2,4,5-tétrahydro-3*H*-3-benzazépin-3-yl)-3-oxopropan-1-amine, ainsi que ses sels d'addition à un acide pharmaceutiquement acceptable;
- la N-{[3,4-diméthoxybicyclo[4.2.0]octa-1,3,5-trién-7-yl]méthyl} 3-(7,8-diméthoxy-1,2,4,5-tétrahydro-3H-3-benzazépin-3-yl) N-méthyl-4-oxobutan-1-amine, ses isomères optiques, ainsi que ses sels d'addition à un acide pharmaceutiquement acceptable;
- la N-{[3,4-diméthoxybicyclo[4.2.0]octa-1,3,5-trién-7-yl]méthyl} 3-(7,8-diméthoxy-1,2,4,5-tétrahydro-3H-3-benzazépin-3-yl)-4-oxobutan-1-amine, ses isomères optiques, ainsi que ses sels d'addition à un acide pharmaceutiquement acceptable;
- le 7-{[[3-(7,8-diméthoxy-1,2,4,5-tétrahydro-3*H*-3-benzazépin-3-yl)-3-oxopropyl]-(méthyl)amino]méthyl}bicyclo[4.2.0]octa-1,3,5-trién-3-yl diméthylcarbamate, ses isomères optiques, ainsi que ses sels d'addition à un acide pharmaceutiquement acceptable.

La présente invention a également pour objet le procédé de préparation des composés de formule (I), à partir d'un composé de formule (II) : 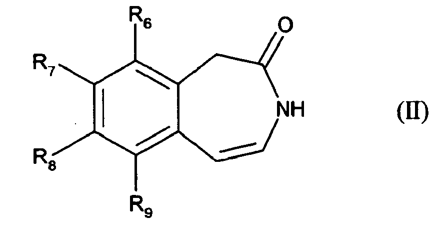 dans laquelle R₆, R₇, R₈ et R₉ sont tels que définis pour la formule (I),
que l'on soumet à une réaction d'hydrogénation, pour conduire au composé de formule (III): 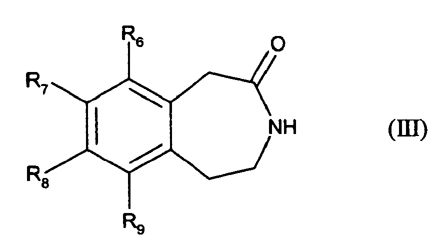 dans laquelle R₆, R₇, R₈ et R₉ sont tels que définis précédemment,
que l'on réduit, pour conduire au composé de formule (IV) : 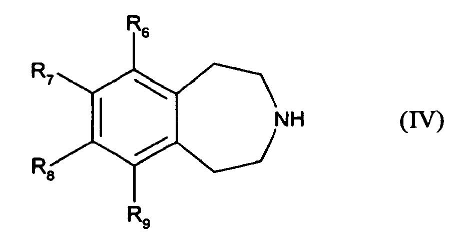 dans laquelle R₆, R₇, R₈ et R₉ sont tels que définis précédemment,
que l'on met en réaction :
- soit, lorsque l'on souhaite accéder aux composés de formule (I) pour lesquels m représente zéro, avec le chlorure d'acryloyle, pour conduire au composé de formule (V) : 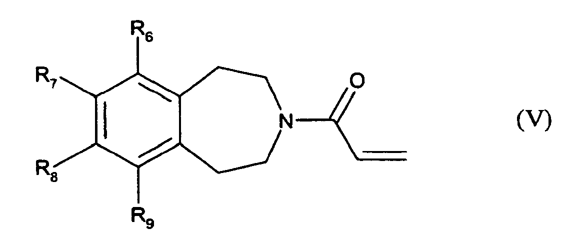 dans laquelle R₆, R₇, R₈ et R₉ sont tels que définis précédemment,
   que l'on soumet à une réaction de couplage avec un composé de formule (VI) : 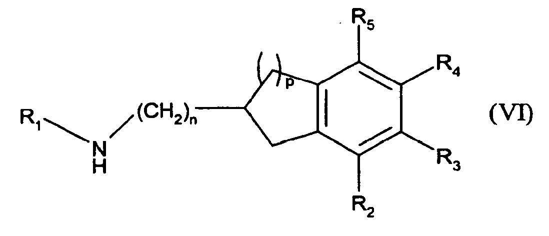 dans laquelle n, p, R₁, R₂, R₃, R₄ et R₅ sont tels que définis pour la formule (I),
   pour conduire aux composés de formule (Ia), cas particulier des composés de formule (I) pour lesquels m représente zéro : 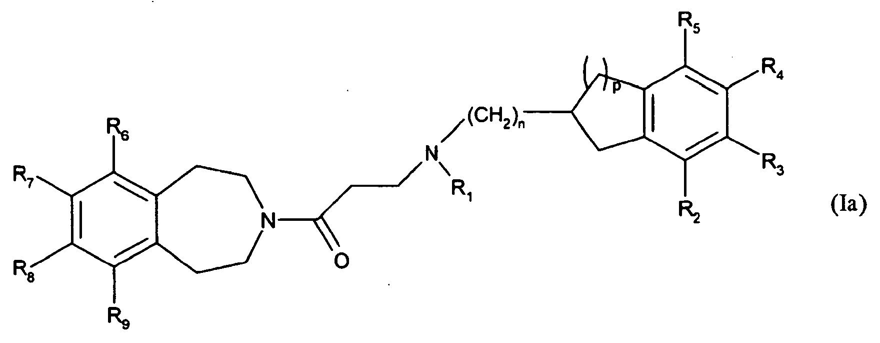 dans laquelle n, p, R₁, R₂, R₃, R₄, R₅, R₆, R₇, R₈ et R₉ sont tels que définis pour la formule (I),
- soit, lorsque l'on souhaite accéder aux composés de formule (I) pour lesquels (X)ₘ représente O ou NH, avec du diphosgène, pour conduire au composé de formule (VII) : 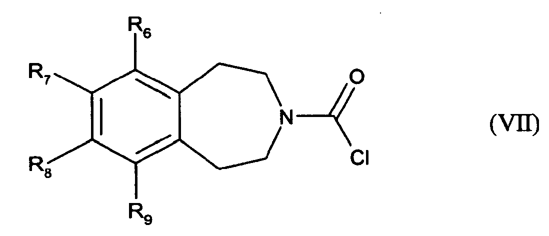 dans laquelle R₆, R₇, R₈ et R₉ sont tels que définis précédemment,
   que l'on met en réaction avec un composé de formule (VIII) : 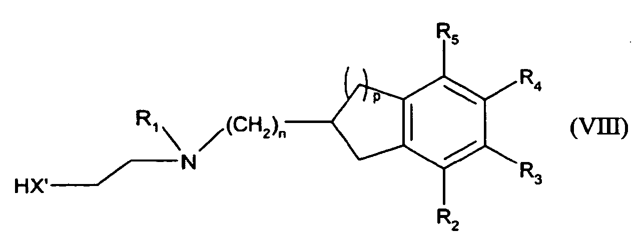 dans laquelle n, p, R₁, R₂, R₃, R₄ et R₅ sont tels que définis pour la formule (I), et
   X' représente O ou NH,
   pour conduire aux composés de formule (Ib), cas particulier des composés de formule (I) pour lesquels m représente 1 et X représente O ou NH : 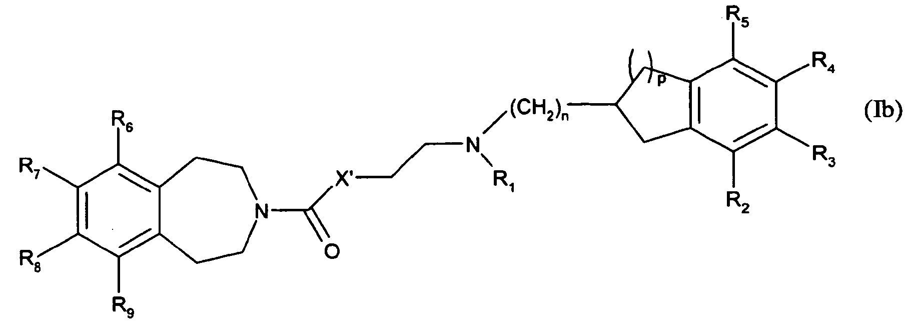 dans laquelle n, p, R₁, R₂, R₃, R₄, R₅, R₆, R₇, R₈ et R₉ sont tels que définis pour la formule (I), et X' représente O ou NH,
- soit, lorsque l'on souhaite accéder aux composés de formule (I) pour lesquels (X)ₘ représente CH₂, avec de la gamma-butyrolactone, pour conduire au composé de formule (IX) : 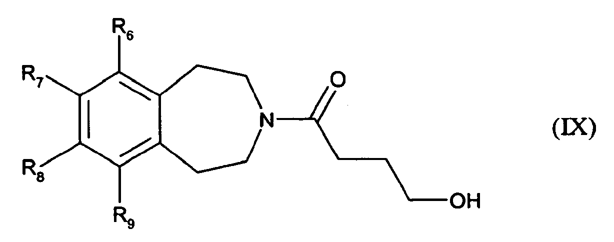 dans laquelle R₆, R₇, R₈ et R₉ sont tels que définis précédemment,
   qui est oxydé en composé de formule (X) : 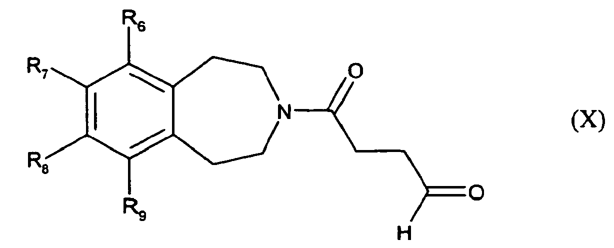 dans laquelle R₆, R₇, R₈ et R₉ sont tels que définis précédemment,
   qui est mis en réaction avec un composé de formule (VI), pour conduire aux composés de formule (Ic), cas particulier des composés de formule (I) pour lesquels m représente 1 et X représente CH₂ : 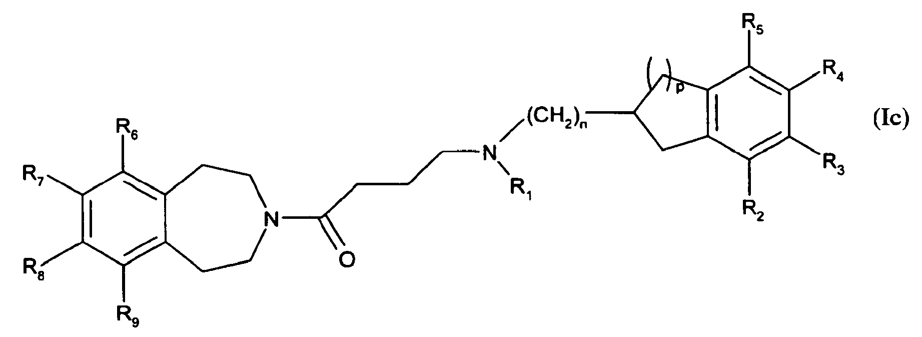 dans laquelle n, p, R₁, R₂, R₃, R₄, R₅, R₆, R₇, R₈ et R₉ sont tels que définis pour la formule (I).

Les formes optiquement actives des composés de formule (I) sont obtenues, soit à partir de formes optiquement actives des intermédiaires de synthèse de formule (VI) et (VIII), soit par dédoublement des formes racémiques des composés de formule (I), selon des méthodes connues de la littérature.

Les composés de l'invention bloquent les canaux "HCN, hyperpolarization-activated cyclic nucleotide-gated channels".
Le blocage des canaux HCN induit par les dérivés de l'invention permet l'utilisation de ceux-ci dans les traitements curatifs ou préventifs de toutes les pathologies dans lesquelles l'activité ou l'expression des canaux HCN est exacerbée et anormale.
Ces composés de l'invention peuvent notamment être utiles dans le traitement curatif ou préventif des douleurs, en particulier des douleurs neuropathiques et inflammatoires, de la vessie hyperactive, et de la sensation de sécheresse oculaire.

Les composés de l'invention réduisent de façon directe et sélective l'activité pacemaker cardiaque.
La réduction sélective de la fréquence cardiaque induite par les dérivés de l'invention permet l'utilisation de ceux ci à titre curatif ou préventif dans les différentes pathologies dans lesquelles une accélération de la fréquence cardiaque joue un rôle déclenchant ou aggravant. Ces produits peuvent en particulier améliorer le traitement et le pronostic à long terme des cardiopathies ischémiques dans leurs différentes expressions cliniques: angor stable, syndromes coronaires aigus : angor instable, syndromes de menace et infarctus du myocarde, post infarctus; des insuffisances cardiaques tant systolique que diastolique et dans leurs formes chronique ou aiguë; des troubles du rythme ventriculaires ou supra ventriculaires, des pathologies constituant un facteur de risque vasculaire : hypertension artérielle, diabète, hypercholestérolémie, en réduisant notamment la dysfonction endothéliale, le développement des lésions d'athéroscléroses et leurs complications. Ces produits peuvent permettre d'assurer une protection myocardique, chez des patients à risque, au cours de la chirurgie ou au cours du choc septique. La réduction de fréquence cardiaque peut par ailleurs faire partie du traitement de maladies, comme l'hyperthyroïdie, s'accompagnant d'une tachycardie sinusale.

La présente invention a également pour objet les compositions pharmaceutiques contenant comme principe actif un composé de formule (I), ou son sel d'addition à un acide pharmaceutiquement acceptable, en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales.

Outre le composé de formule (I), les compositions pharmaceutiques selon l'invention contiennent un ou plusieurs excipients ou véhicules tels que des diluants, des lubrifiants, des liants, des agents de désintégration, des absorbants, des colorants, des édulcorants.

A titre d'exemple d'excipients ou véhicules, on peut citer :
◆ *pour les diluants :* le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants :* le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.

Le pourcentage de principe actif de formule (I) dans la composition pharmaceutique est préférentiellement compris entre 5% et 50% en poids.

La posologie utile varie selon l'âge et le poids du patient, la voie d'administration, la nature et la sévérité de l'affection, et la prise de traitements éventuels associés et s'échelonne de 0,5 mg à 500 mg en une ou plusieurs prises par jour.

Les exemples suivants illustrent la présente invention. Les structures des composés décrits dans les exemples ont été déterminées selon les techniques spectrophotométriques usuelles (infrarouge, résonance magnétique nucléaire, spectrométrie de masse).

Les préparations décrivent la préparation des intermédiaires de synthèse.

| Abréviations : | |
|---|---|
| BINAP | 2,2'-bis(diphénylphosphino)-1,1'-binaphtyle |
| C.C.M | chromatographie sur couche mince |
| DMF | N,N-diméthylformamide |
| DMSO | diméthylsulfoxyde |
| éq. | équivalent |
| IR | infra-rouge |
| PEG 300 | polyéthylèneglycol ayant un poids moléculaire moyen de 300 g/mol |
| THF | tétrahydrofurane |

### PREPARATION 1 : [(4,5-Diméthoxybicyclo[4.2.0]octa-1,3,5-trièn-7-yl)-méthyl]-amine

### Stade 1 : 8,8-Diéthoxy 2,3-diméthoxybicyclo[4.2.0]octa 1,3,5-triène

Sur 8,4 g (215 mmol.)/ 2éq.) d'amidure de sodium dans 10 mL de toluène, on coule 24 g (107 mmol.) de bromovératrol , puis 25 g (215 mmol./ 2 éq.) de 1,1-diéthoxy éthylène. On porte à 80°C pendant 4h30, refroidit à -10°C et coule 50 mL d'eau, puis 50 mL de toluène. On sépare les phases et réextrait la phase aqueuse au toluène. Les phases toluéniques jointes sont lavées à l'eau jusqu'à pH neutre, puis concentrées. Le résidu est chromatographié sur 600 g de silice (éluant : dichlorométhane) pour conduire au produit désiré.

### Stade 2 : 4,5-Diméthoxybicyclo[4.2.0] octa 1,3,5-trièn-7-one

Sur 7,5 g (29,7 mmol.) du composé précédent en solution dans 120 mL de THF, on ajoute 30 mL d'une solution aqueuse molaire d'acide chlorhydrique. On maintient sous agitation à température ambiante pendant 19 h, puis évapore au maximum le THF. La masse blanche obtenue est reprise par 150 mL d'eau et agitée pendant 1 h à 5°C. Après filtration, 3 lavages par 15 mL d'eau et séchage sous vide, on récupère le produit désiré.

### Stade 3 : 4,5-Diméthoxybicyclo[4.2.0] octa 1,3,5-trièn-7-ol

Sur 4,73 g (26,5 mmol.) du composé précédent en suspension dans 138 mL de méthanol à 0°C, on ajoute, en 2 fois, en 5 mn 1,21 g (31,8 mmol.) de borohydrure de sodium. Après 1 h sous agitation à 0°C, on verse dans 350 mL d'eau, puis extrait 3 fois par 100 mL de dichlorométhane. Les phases organiques jointes sont lavées 2 fois par 50 mL d'eau, puis séchées sur sulfate de magnésium et concentrées pour conduire au produit désiré.

### Stade 4 : 8-Bromo 2,3-diméthoxybicyclo[4.2.0] octa 1,3,5-triène

On porte au reflux pendant 2h30 4,55 g (25,3 mmol.) du composé précédent, 11,9 g (45,5 mmol. / 1,8 éq.) de triphénylphosphine et 10,05 g (30,3 mmol./ 1,2 éq.) de tétrabromométhane dans 150 mL d'éther. Après refroidissement, l'insoluble est filtré sur fritté et rincé 5 fois par 50 mL d'éther. Après concentration des filtrats, le résidu est chromatographié sur 550 g de silice (éluant : dichlorométhane) pour conduire au produit désiré.

### Stade 5 : 4,5-Diméthoxybicyclo[4.2.0] octa 1,3,5-trièn-7-carbonitrile

6,0 g (24,8 mmol.) du composé précédent et 8,64 g (32,2 mmol./ 1,3 éq.) de cyanure de tétrabutylammonium dans 125 mL de THF sont agités à température ambiante pendant 21 h. Après concentration du milieu réactionnel, le résidu est chromatographié sur 350 g de silice (éluant : dichlorométhane) pour conduire au produit désiré.

### Stade 6 : [(4,5-Diméthoxybicyclo[4.2.0] octa 1,3,5-trièn-7-yl)méthyl]amine

A 4,01 g (21,2 mmol.) du composé précédent en solution dans 60 mL de méthanol et 60 mL de méthanol ammoniacal 7N, on ajoute 2 mL de Nickel de Raney. On hydrogène à température ambiante et pression ordinaire pendant 6h30. On filtre alors sur célite, rince au méthanol et concentre pour conduire au produit désiré.

### PREPARATION 2 : ((2-Méthoxybicyclo[4.2.0]octa-1,3,5-trièn-7-yl)méthyl]amine

### Stade 1 : 3-(2, 6-Dibromophényl) 2-chloro-propanenitrile

Sur 62,4 g (464 mmol./1,2 éq.) de chlorure cuivrique, 76 mL (580 mmol./ 1,5 éq.) de tert-butyl nitrite et 483 mL (7,34 moles/ 19 éq.) d'acrylonitrile dans 475 mL d'acétonitrile, à température ambiante, on coule goutte à goutte en 1h 100g (386 mmol.) de 2,6-dibromo aniline en solution dans 380 mL d'acétonitrile. On maintient encore 1h sous agitation à température ambiante, puis verse le milieu réactionnel sur 1 L d'une solution aqueuse à 20% d'acide chlorhydrique. La phase aqueuse est extraite au toluène et les phases toluéniques jointes sont lavées par une solution aqueuse saturée de chlorure de sodium. Après concentration, on obtient le produit désiré qui est engagé tel quel dans l'étape suivante.

### Stade 2 : 3-(2,6-Dibromophényl)-propanenitrile

Le composé précédent est dissout dans 1400 mL d'acide acétique. On ajoute en une fois 68 g (1,04 moles/ 2,7 éq.) de zinc en poudre. Un exotherme jusqu'à 58°C se développe lentement. Après 1h de contact, on verse le milieu réactionnel sur 3 kg de glace. La phase aqueuse obtenue est extraite par 1 L, puis 500 mL de toluène. Les phases toluéniques jointes sont lavées par une solution aqueuse saturée d'hydrogénocarbonate de sodium, puis une solution aqueuse saturée de chlorure de sodium. Après concentration, le résidu est chromatographié sur silice pour conduire au produit désiré.

### Stade 3 : 2-Bromobicyclo[4.2.o]octa 1,3,5-triène 7-carbonitrile

Sur l'amidure de sodium (1,91 moles / 4 éq.) dans 3 L d'ammoniac liquide, on ajoute 138g (477 mmol.) du composé précédent sous forme solide. Après 2 h sous agitation au reflux de l'ammoniac, on bloque le réaction en ajoutant 102g (4 éq.) de chlorure d'ammonium solide, puis on laisse l'ammoniac s'évaporer. Le résidu est repris par 1 L d'éther, on agite, puis filtre le solide et le rince à l'éther. Après concentration des filtrats joints, le résidu est chromatographié sur 2 kg de silice (éluant : dichlorométhane) pour donner le produit désiré.

### Stade 4 : Acide 2-bromobicyclo[4.2.0]octa 1,3,5-triène 7-carboxylique

Sur 75 g (360 mmol.) du composé précédent dans 375 mL d'éthanol, à température ambiante, on ajoute une solution de 38 g (612 mmol. : 1,7 éq.) de potasse en solution dans 125 mL d'eau. On porte au reflux pendant 16 h, puis évapore l'éthanol. On ajoute 1 L d'eau, lave la phase aqueuse 2 fois par 250 mL d'éther, puis l'acidifie jusqu'à pH 1 avec de l'acide chlorhydrique concentré. Le précipité formé est filtré, rincé à l'eau et séché pour conduire au produit désiré.

### Stade 5 : 2-(2-Bromobicyclo[4.2.0]octa 1,3,5-trien-7-yl) 4,4-diméthyl 4,5-dihydro oxazole

On mélange 79 g (348 mmol.) du composé précédent et 39 g (417 mmol./ 1,2 éq.) de 1,1-diméthyl 2-hydroxy éthylamine dans 800 mL de xylène. On chauffe à 140°C pendant 6 h en réalisant un entraînement azéotropique de l'eau. On laisse ensuite refroidir et maintient sous agitation à température ambiante pendant la nuit. Le précipité qui s'est formé (essentiellement de l'acide de départ) est filtré et lavé au toluène. On concentre les filtrats joints pour conduire au produit désiré.

### Stade 6 : [7-(4,4-Diméthyl-4,5-dihydro-1,3-oxazol-2-yl)bicyclo[4.2.0]octa-1,3,5-trién-2-yl](diphénylméthylène) amine

Dans 800 mL de toluène, à température ambiante, on mélange 48 g (171 mmol.) du composé précédent, 37,3 g (205 mmol./ 1,2 éq.) de benzophénone-imine, 1,57 g (1,71 mmol./ 0,01 éq.) de di-palladium tris-(dibenzylidène acétone), 3,2 g (5,14 mmol./ 0,03 éq.) de BINAP et 23 g (240 mmol./ 1,4 éq.) de tert-butylate de sodium. On chauffe à 85°C pendant 24 h , en ajoutant 9,3 g (0,3 éq.) de benzophénone-imine après 4 h. Après arrêt du chauffage, on filtre les insolubles vers 50°C, puis concentre le filtrat pour obtenir le produit désiré, qui est engagé tel quel dans l'étape suivante.

### Stade 7 : Ester éthylique de l'acide 2-aminobicyclo[4.2.0]octa 1,3,5-triène 7-carboxylique

On porte au reflux pendant 1h30 117 g (environ 171 mmol.) du composé précédent dans 2400 mL d'éthanol contenant 200 mL d'acide sulfurique concentré. On concentre au maximum le solvant, puis reprend par 1,5 L d'eau. La phase aqueuse est lavée à l'éther, basifiée par du carbonate de potassium solide, puis extraite à l'éther. Après séchage sur sulfate de magnésium et concentration des phases éthérées jointes, on recueille le produit désiré.

### Stade 8 : Acide 2-hydroxybicyclo[4.2.0]octa 1,3,5-triene 7-carboxylique

A froid, sur 11 g (57,5 mmol.) du composé précédent en suspension dans 220 mL d'eau, on coule 18,6 mL (345 mmol./ 6 éq.) d'acide sulfurique concentré en maintenant la température inférieure à 15°C. On refroidit vers 0 à 5°C et coule goutte à goutte en 15 mn une solution de 4,36 g (63,3 mmol./ 1,1 éq.) de nitrite de sodium dans 25 mL d'eau, agite pendant 5 mn à 0°C, puis coule goutte à goutte en 10 mn et en maintenant la température inférieure à 10°C une solution de 144 mg (0,57 mmol./ 0,01 éq.) de sulfate de cuivre pentahydraté dans 166 mL d'acide sulfurique aqueux 1M. On chauffe ensuite à 80°C pendant 1h, puis maintient sous agitation la nuit à température ambiante. La phase aqueuse est extraite à l'éther, les phases éthérées jointes sont lavées par une solution aqueuse saturée de chlorure de sodium, séchées sur sulfate de magnésium et concentrées. Le résidu est chromatographié sur 500 g de silice (éluant : dichlorométhane / éthanol : 90/10) pour donner le produit désiré.

### Stade 9: Ester éthylique de l'acide 2-méthoxybicyclo[4.2.0]octa 1,3,5-triene 7-carboxylique

On porte à 130°C pendant 45 h, 6 g (33 mmol.) du composé précédent et 2,7 g (8,3 mmol./0,25 éq.) de carbonate de césium dans 330 mL de carbonate de diméthyle. Après filtration et concentration du milieu réactionnel, on recueille le produit désiré.

### Stade 10 : 2-Méthoxybicyclo[4.2.0]octa 1,3,5-triene 7-carboxamide

Sur 6 g (31 mmol.) du composé précédent dans 50 mL de THF à 0°C, on ajoute goutte à goutte 120 mL d'une solution aqueuse à 28% d'ammoniaque. On maintient sous agitation à température ambiante pendant 2 jours, puis évapore au maximum le THF. La phase aqueuse est extraite à l'acétate d'éthyle. Les phases organiques jointes sont séchées sur sulfate de magnésium et concentrées pour donner le produit désiré.

### Stade 11 : 1-(2-Méthoxybicyclo[4.2.0]octa 1,3,5-trién-7-yl)méthanamine

Sur 0,87 g (4,9 mmol.) du composé précédent en solution dans 20 mL de THF, à température ambiante, on coule goutte à goutte en 10 mn 1,39 mL (14,7 mmol./ 3 éq.) de borane-diméthylsulfure, puis porte à 70°C pendant 24 h. On ramène à température ambiante, solvolyse par 7,5 mL de méthanol anhydre et porte de nouveau à reflux pendant 1h. Après évaporation, le résidu (0,99 g) est chromatographié sur une colonne de silice de 50g (éluant : dichlorométhane / éthanol / ammoniaque: 95/5/0,5) pour donner une première fraction du produit désiré et une autre fraction du même produit sous forme de complexes de bore. Cette dernière fraction, après traitement par l'éthanol chlorhydrique, puis passage acide-base, donne une fraction supplémentaire du produit désiré.

### PREPARATION 3 : 7-(Aminométhyl)bicyclo[4.2.0]octa-1,3,5-trièn-3-ol

### Stade 1 : 3-Hydroxybicyclo[4.2.0]octa 1,3,5-triène 7-carbonitrile

68,7 g (431,6 mmol.) de 3-méthoxybicyclo[4.2.0]octa 1,3,5-triène 7-carbonitrile en solution dans 1500 mL de dichlorométhane sont amenés à 0°C. On ajoute alors goutte à goutte en 1h15, 863 mL d'une solution molaire de tribromure de bore dans le dichlorométhane (863 mmol./ 2 éq.). On laisse ensuite remonter à température ambiante pendant la nuit. On ramène vers 0°C et verse sur 1 kg de glace. On agite pendant 30 mn à température ambiante, sépare les phases et réextrait la phase aqueuse par 2 fois 500 mL de dichlorométhane. Les phases organiques jointes sont lavées par 600 mL d'eau, puis séchées sur sulfate de magnésium. Après filtration et concentration, on obtient le produit recherché.

### Stade 2 : 7-(Aminométhyl)bicyclo[4.2.0]octa-1,3,5-trièn-3-ol

A 8,0 g (55,1 mmol.) du nitrile précédent en solution dans 170 mL de méthanol et 170 mL de méthanol ammoniacal 7N, on ajoute 8 mL de Nickel de Raney. On hydrogène à température ambiante et pression ordinaire jusqu'à absorption du volume théorique d'hydrogène. On filtre alors sur célite, rince au méthanol et concentre pour obtenir l'amine recherchée.

### PREPARATION 4 : 8-(Aminométhyl)bicyclo[4.2.0]octa-1,3,5-trién-3-ol

On procède de la même manière que pour le produit de la préparation 3, mais à partir du 4-méthoxybicyclo[4.2.0]octa 1,3,5-triène 7-carbonitrile.

### PREPARATION 5 : [(3-Ethoxybicyclo[4.2.0]octa 1,3,5-trién-7-yl)méthyl]amine

### Stade 1 : 3-Ethoxybicyclo[4.2.0]octa 1,3,5-triène 7-carbonitrile :

On solubilise 1,5 g (10,19 mmoles) du produit du stade 1 de la préparation 3 dans 15 mL de diméthylformamide (DMF). On ajoute 2,8 g (2 équivalents) de carbonate de potassium, puis 2,3 mL (3 équivalents) de bromure d'éthyle et 100 mg (0,06 équivalent) d'iodure de potassium. On chauffe à 80°C pendant 3 heures le milieu réactionnel. Puis on refroidit et on évapore la DMF à l'évaporateur rotatif. Le résidu obtenu est repris à l'eau et extrait au dichlorométhane (CH₂Cl₂). La phase organique est séchée sur MgSO₄, filtrée puis évaporée pour donner 1,8 g d' huile.
Cette huile est purifiée par flash-chromatographie sur 100 g de silice (éluant : CH₂Cl₂ 100 %) pour conduire au produit attendu qui cristallise à température ambiante.
*Point de fusion* = 50-52°C

### Stade 2: [(3-Ethoxybicyclo[4.2.0]octa 1,3,5-trièn-7-yl)méthyl]amine

On met en solution 1,5 g (8,65 mmoles) du produit du stade 1 dans 30 mL de méthanol (MeOH). On ajoute 30 mL d'une solution de méthanol-ammoniacal (7N) et 0,5 g de Nickel de Raney. On hydrogène le milieu réacionnel sous une pression de 1 bar, à température ambiante pendant une nuit. Puis on filtre le catalyseur et on évapore à sec le filtrat. On obtient le produit attendu sous forme d'huile.

### PREPARATION 6: [(3-tert-Butoxybicyclo[4.2.0]octa 1,3,5-trien-7-yl)méthyl]amine

### Stade 1: 3-tert-Butoxybicyclo[4.2.0]octa 1,3,5-triène 7-carbonitrile

On solubilise 2,0 g (13,59 mmoles) du produit du stade 1 de la préparation 3 dans 100 mL de dichlorométhane (CH₂Cl₂). On refroidit à 0°C et on fait buller de l'isobutène au travers de la solution jusqu'à saturation. Puis on ajoute 0,2 mL d'acide sulfurique concentré et on agite à température ambiante pendant 24 heures. La solution est neutralisée par ajout de NaOH (1N). On extrait à CH₂Cl₂ , on lave à l'eau , on sèche sur MgSO₄, puis on filtre et on évapore à sec le filtrat. On obtient un résidu qui est purifié par flash-chromatographie sur 150 g de silice (éluant : CH₂Cl₂ 100 %) pour conduire au produit attendu sous forme d'huile.

### Stade 2 : [(3-tert-Butoxybicyclo[4.2.0]octa 1,3,5-trien-7-yl)méthyl]amine

On met en solution 1,1 g (5,46 mmoles) du produit du stade 1 dans 30 mL de méthanol (MeOH). On ajoute 30 mL d'une solution de méthanol-ammoniacal (7N) et 0,5 g de Nickel de Raney. On hydrogène le milieu réactionnel sous une pression de 1 bar, à température ambiante pendant une nuit. Puis on filtre le catalyseur et on évapore à sec le filtrat. On obtient le produit attendu sous forme d'huile.

### PREPARATION 7 : 5,6-Dihydrocyclobuta[f][1,3]benzodioxol-5-ylméthylamine

### Stade 1: (5,6-Dihydrocyclobuta[4,5] benzo [1,2-d][1,3]dioxol 5-yl-méthyl)carbamate d'éthyle

On met en solution 7,9g (44,6 mmoles) de (5,6-dihydrocyclobuta[4,5] benzo [1,2-*d*][1,3]dioxol 5-ylméthyl)amine dans 80 mL de dichlorométhane. On ajoute 80 mL d'eau et 6 mL (1,6 équivalent) d'une solution aqueuse de soude (12N). On ajoute en 15 minutes, 3,95 mL (1,1 équivalent) de chloroformiate d'éthyle et on agite à 25°C pendant 2 heures. On décante, lave la phase organique avec une solution aqueuse saturée en NaHCO₃. On sèche sur MgSO₄, filtre et évapore à sec. On obtient le produit attendu sous la forme d'un solide brun.

### Stade 2 : 5,6-Dihydrocyclobuta[f][1,3]benzodioxol-5-ylméthylamine

On solubilise 7,5 g (30 mmoles) du produit du stade 1 dans 15 mL de tétrahydrofurane anhydre. Cette solution est versée goutte à goutte sur une suspension de 1,48 g (1,3 équivalents) de AlLiH₄ en suspension dans 38 mL de tétrahydrofurane anhydre. On agite 2 heures à 25°C. On hydrolyse à froid par 2,7 mL d'eau, 2,7 mL de NaOH (20%) puis 2,7 mL d'eau. On filtre et évapore à sec le filtrat. On obtient 2,4 g d'un résidu qui est purifié par colonne chromatographie sur silice (éluant : CH₂Cl₂ / Éthanol / NH₄OH : 95/5/0,5. On obtient le produit attendu sous la forme d'huile marron.

### PREPARATION 8 : [2-(5,6-Diméthoxy 2,3-dihydro 1H indén-2-yl) éthyl]amine

On solubilise 5 g (2,3 mmoles) de (5,6-diméthoxy 2,3-dihydro 1H inden-2-yl) acétonitrile dans 50 ml de méthanol. On ajoute 50 mL d'une solution MeOH-NH₃ (7 N) et 0,5 g de Nickel de Raney. On hydrogène sous 5 bar de pression, à 25°C, pendant 4 jours. On filtre sur célite, évapore à sec le filtrat. Le résidu est repris par 50 mL d'HCl (1N), lavé par de l'acétate d'éthyle, puis la phase aqueuse est amenée à pH = 10 en ajoutant NaOH (20%). On extrait alors par Et₂O, lave à l'eau, sèche sur MgSO₄, filtre et évapore à sec. On obtient le produit attendu sous forme d'huile.

### PREPARATION 9 : 2-(5-Méthoxybicyclo[4.2.0]octa 1,3,5-trién-7-yl)méthylamine

3g (19mmol) de 5-méthoxybicyclo[4.2.0]octa 1,3,5-triène 7-carbonitrile dans 45 mL de méthanol et 45 mL d'une solution 7M de méthanol ammoniacal sont hydrogénés en présence de 1,5g de nickel de Raney à 50% dans l'eau à pression atmosphérique. Après deux heures de réaction, le milieu réactionnel est filtré et évaporé pour conduire au produit attendu.

### PREPARATION 10: 2-(2,3-Diméthoxybicyclo[4.2.0]octa 1,3,5-trien-7-yl)méthylamine

Obtenu de la même façon que le produit de la préparation 9 mais en utilisant le 2,3-diméthoxybicyclo[4.2.0]octa 1,3,5-triène 7-carbonitrile.

### PREPARATION 11 :2-(2,3,4-Triméthoxybicyclo[4.2.0]octa 1,3,5-trièn-7-yl)méthylamine

Obtenu de la même façon que le produit de la préparation 9 mais en utilisant le 2,3,4-triméthoxybicyclo[4.2.0]octa 1,3,5-triène 7-carbonitrile.

### PREPARATION 12 : (Cyclopropylméthyl){[(7S)- 3,4-diméthoxybicyclo[4.2.0]octa 1,3,5-trién-7-yl]méthyl}amine

### Stade 1 : {[(7S)-3,4-Diméthoxybicyclo[4.2.0]octa 1,3,5-trién-7-yl]méthyl}cyclopropane carboxamide

A 4,6 g (20 mmol.) de chlorhydrate de {[(7S) 3,4-diméthoxybicyclo[4.2.0]octa-1,3,5-trien-7-yl]méthyl}amine dans 60 mL de dichlorométhane, à température ambiante, on ajoute d'un trait 6,1 mL (44 mmol./ 2,2 éq.) de triéthylamine et laisse agiter 30 mn environ. On ajoute alors goutte à goutte en 40 mn, 2 mL (22 mmol./ 1,1 éq.) du chlorure de l'acide cyclopropane carboxylique. On laisse agiter à température ambiante pendant 1h30, puis transvase dans une ampoule à décanter, ajoute 60 mL de dichlorométhane et lave successivement par 40 mL d'eau, 2 fois 40 mL d'acide chlorhydrique 1N, 2 fois 40 mL d'une solution aqueuse saturée d'hydrogénocarbonate de sodium et 40 mL d'eau. Après séchage sur sulfate de magnésium, filtration et concentration, on recueille le produit désiré.

### Stade 2 : (Cyclopropylméthyl){[(7S)-3,4-diméthoxybicyclo[4.2.0]octa-1,3,5-trién-7-yl]-méthyl}-amine

A 1,2 g (30 mmol.) d'aluminohydrure de lithium en suspension dans 40 mL de THF, à température ambiante, on ajoute goutte à goutte une solution de 5,2 g (20 mmol.) du composé précédent dans 80 mL de THF, puis porte au reflux pendant 4h30. On refoidit vers 0°C, puis ajoute avec précautions successivement 0,79 mL d'eau, 0,63 mL d'une solution aqueuse à 20% de soude et 2,9 mL d'eau. On laisse agiter la nuit à température ambiante, puis filtre les sels sur fritté et rince au THF. Les filtrats joints sont concentrés pour conduire au produit désiré.

### PREPARATION 13: {[(7S)-3,4-Diméthoxybicyclo[4.2.0]octa-1,3,5-trién-7-yl]méthyl} éthanamine

On procède comme à la préparation 12, stades A et B mais en utilisant au stade A le chlorure d'acétyle au lieu du chlorure de l'acide cyclopropane carboxylique.

### PREPARATION 14 : N-Benzyl-1-[(7S)-3,4-diméthoxybicyclo[4.2.0]octa-1,3,5-trién-7-yl] méthanamine

On procède comme à la préparation 12, stades A et B mais en utilisant au stade A le chlorure de benzoyle au lieu du chlorure de l'acide cyclopropane carboxylique.

### PREPARATION 15 : (Cyclopentylméthyl){[(7S)-3,4-diméthoxybicyclo[4.2.0]octa-1,3,5-trién-7-yl]méthyl}amine

On procède comme à la préparation 12, stades A et B mais en utilisant au stade A le chlorure de l'acide cyclopentane carboxylique au lieu du chlorure de l'acide cyclopropane carboxylique

### PREPARATION 16 : (Cyclobutylméthyl){[(7S)-3,4-diméthoxybicyclo[4.2.0]octa-1,3,5-trién-7-yl]méthyl}amine

On procède comme à la préparation 12, stades A et B mais en utilisant au stade A le chlorure de l'acide cyclobutane carboxylique au lieu du chlorure de l'acide cyclopropane carboxylique.

### PREPARATION 17: -N-{[(7S)-3,4-Diméthoxybicyclo[4.2.0]octa-1,3,5-trién-7-yl]-méthyl} 2,2,2-trifluoroéthanamine

### Stade 1 : N-{[(7S)-3,4-Diméthoxybicyclo[4.2.0]octa-1,3,5-trién-7-yl]-méthyl}-2,2,2-trifluoroacétamide

A 2,9 g (15 mmol.) de {[(7*S*) 3,4-diméthoxybicyclo[4.2.0]octa-1,3,5-trién-7-yl]méthyl}amine dans 7,5 mL de méthanol, à température ambiante, on ajoute d'un trait 2,1 mL (15 mmol./ 1 éq.) de triéthylamine, puis 2,3 mL (19 mmol./ 1,25 éq.) de trifluoroacétate d'éthyle, goutte à goutte en 20 mn. On laisse agiter à température ambiante pendant 2h, puis évapore à sec. On reprend par 100 mL de dichlorométhane, lave par 50 mL d'acide chlorhydrique 1N, puis 50 mL d'eau et sèche sur sulfate de magnésium. Après filtration et concentration, on recueille le produit désiré.

### Stade 2 : N-{[(7S)-3,4-Diméthoxybicyclo[4.2.0]octa-1,3,5-trién-7-yl]-méthyl}-2,2,2-trifluoroéthanamine

A 4,07 g (14,0 mmol.) du composé du stade 1 dans 28 mL de THF, vers 0°C, on ajoute goutte à goutte en 10 mn, 21 mL(21 mmol./ 1,5 éq.) d'une solution molaire de borane dans le THF, puis porte au reflux pendant 18 h. On laisse refroidir et ajoute goutte à goutte 14 mL d'une solution d'éthanol chlorhydrique 2,6 N, puis porte de nouveau au reflux 2 h. On laisse refroidir, filtre le solide sur fritté, le rince à l'éther et le sèche sous vide pour obtenir le chlorhydrate, et après traitement en milieu basique, le produit attendu.

### PREPARATION 18 : N-{[(7S)-3,4-Diméthoxybicyclo[4.2.0]octa-1,3,5-trién-7-yl]-méthyl} prop-2-ène-1-amine

A 3,9 g (20 mmol.) de {[(7*S*) 3,4-diméthoxybicyclo[4.2.0]octa-1,3,5-trién-7-yl]méthyl}amine dans 40 mL d'acétone, à température ambiante, on ajoute d'un trait 4,1 g (30mmol./ 1,5 éq.) de carbonate de potassium, puis on ajoute goutte à goutte rapidement 1,9 mL (22 mmol./ 1,1 éq.) de bromure d'allyle. On laisse agiter à température ambiante pendant 60 h, puis filtre les sels et concentre le filtrat. Le résidu (4,8 g) est chromatographié sur 300 g de silice (éluant : dichlorométhane / éthanol / ammoniaque : 98/2/0,2) pour conduire au produit désiré.

### PREPARATION 19 : -N-{[(7S)-3,4-Diméthoxybicyclo[4.2.0]octa-1,3,5-trién-7-yl] méthyl}cyclopentanamine

Dans 60 mL de dichlorométhane, à température ambiante, on mélange 2,9 g (15 mmol.) de {[(7*S*) 3,4-diméthoxybicyclo[4.2.0]octa-1,3,5-trién-7-yl]méthyl}amine et 1,3 mL (15 mmol./ 1 éq.) de cyclopentanone. On ajoute ensuite 4,8 g (22,5 mmol./ 1,5 éq.) de triacétoxyborohydrure de sodium, puis 0,86 mL (15 mmol./ 1 éq.) d'acide acétique, et on laisse agiter à température ambiante pendant 4 h. On verse alors 90 mL d'une solution aqueuse normale de soude, puis extrait 2 fois par 120 mL d'éther. Les phases organiques jointes sont lavées par 90 mL d'eau, puis 90 mL d'une solution aqueuse saturée de chlorure de sodium, puis séchées sur sulfate de magnésium. Après filtration et concentration, on recueille le produit désiré.

### PREPARATION 20 : N-{[(7S)-3,4-Diméthoxybicyclo[4.2.0]octa-1,3,5-trién-7-yl]-méthyl}cyclobutanamine

On procède comme à la préparation 19, mais en utilisant la cyclobutanone au lieu de la cyclopentanone.

### PREPARATION 21 : (7-Aminométhyl) bicyclo[4.2.0]octa 1,3,5-trién-3-yl triflurométhylsulfonate

### Stade 1: 7-Cyanobicyclo[4.2.0]octa 1,3,5-trién-3-yl trifluorométhane sulfonate

On coule à 0°C en 1 heure, 2,4 mL d'anhydride triflique (1,2 équivalents) sur une solution de 2 g (13,8 mmoles) de 3-hydroxy bicyclo[4.2.0]octa 1,3,5-triène 7- carbonitrile dans 40 mL de dichlorométhane et 3,9 mL (2 équivalents) de triéthylamine. On laisse agiter 3 jours à 25°C. On verse le milieu réactionnel sur de l'eau, on extrait au dichlorométhane, sèche sur MgSO₄, filtre et évapore pour obtenir 3,8 g d'une huile. Ce résidu est purifié par flash-chromatographie sur 150 g de silice ; éluant = 100 % toluène. On obtient le produit attendu sous forme d'huile.

### Stade 2 : (7-Aminométhyl) bicyclo[4.2.0]octa 1,3,5-trién-3-yl triflurométhylsulfonate

On solubilise 2,7 g (9,74 mmoles) du produit du stade 1 dans 40 mL de méthanol. On ajoute 40 mL d'une solution MeOH-NH₃ (7 N) et 1 g de Nickel de Raney. On hydrogène sous 5 bar de pression , à 25°C, pendant 4 jours. On filtre sur célite, évapore à sec le filtrat. On obtient le produit attendu sous forme d'huile.

### PREPARATION 22 : (7-Aminométhyl) bicyclo[4.2.0]octa 1,3,5-trién-3-yl diméthylsulfamate

### Stade 1: 7-Cyanobicyclo[4.2.0]octa 1,3,5-trién-3-yl diméthylsulfamate

On mélange à 25°C, 2g (13,8 mmoles) de 3-hydroxy bicyclo[4.2.0]octa 1,3,5-triène 7-carbonitrile dans 40 mL de dichlorométhane , 3,9 g (2 équivalents) de triéthylamine et 1,63 mL (1,1 équivalent) de chlorure de diméthylsulfamoyle. On laisse agiter 4 jours à 25°C. On verse le milieu réactionnel sur de l'eau, on extrait au dichlorométhane, sèche sur MgSO₄, filtre et évapore pour obtenir une huile. Ce résidu est purifié par flash-chromatographie sur 70 g de silice (éluant : dichorométhane / toluène : 50/50). On obtient le produit attendu sous la forme d'un solide blanc.
*Point de fusion* (M.K.) = 78-79°C.

### Stade 2 : (7-Aminométhyl) bicyclo[4.2.0]octa 1,3,5-trién-3-yl diméthylsulfamate

On solubilise 1,8 g (7,1 mmoles) du produit du stade 1 dans 40 mL de méthanol. On ajoute 40 mL d'une solution MeOH-NH₃ (7 N) et 1 g de Nickel de Raney. On hydrogène sous 5 bar de pression, à 25°C, pendant 4 jours. On filtre sur célite, évapore à sec le filtrat. On obtient une huile qui est purifiée par flash-chromatographie sur 50 g de silice (éluant : dichorométhane / Éthanol : 90/10). On obtient le produit attendu sous forme d'huile.

### PREPARATION 23 : (5,6-Dihydrocyclobuta[4,5]benzo[1,2-b]furan-6-yl-méthyl)-amine

On solubilise 7 g (41,3 mmoles) de 5,6-dihydrocyclobuta[4,5]benzo[1,2-b]furan-6-carbonitrile dans 490 mL d'éthanol. On ajoute 70 mL d'une solution à 28% de NH₄OH et 1 g de Nickel de Raney. On hydrogène sous 5 bar de pression, à 25°C, pendant 3 jours. On filtre sur célite, évapore à sec le filtrat. On obtient le produit attendu sous la forme d'une huile jaune.

### PREPARATION 24 : 2-(Bromométhyl)-5,6-diméthoxyindane

On solubilise 1 g (4,8 mmoles) de (5,6-diméthoxy 2,3-dihydro-1*H-*indén-2-yl)méthanol dans 20 mL de dichlorométhane et 1 mL de triéthylamine. On refroidit à 0°C, et on coule 0,41 mL (1,1 équivalent) de chlorure de mésyle. On agite 1 heure à 25°C. On hydrolyse par 30g de glace. On décante, lave avec HCl (1N), puis H₂O. On sèche sur MgSO₄, filtre et évapore. On obtient 1,2 g d'un solide beige qui est dissout dans 30 mL d'acétone. On ajoute 0,83g de LiBr (2 équivalents) et on agite 12 heures à 25°C. On évapore l'acétone, le résidu est repris par de l'eau et extrait à l'éther diéthylique. La phase organique est décantée, séchée sur MgSO₄, filtrée et évaporée. On obtient le produit attendu sous forme d'huile.

### PREPARATION 25 : 2-[([(7S)- 3,4-Diméthoxybicyclo[4.2.0]octa 1,3,5-trién-7-yl] méthyl}(méthyl)amino]éthanol

A température ambiante, on mélange dans 10 mL d'acétonitrile 1,04 g (5 mmol.) de {[(7*S*)-3,4-diméthoxybicyclo[4.2.0]octa-1,3,5-trién-7-yl]méthyl}méthylamine, 0,44 mL (6 mmol./1,2 éq.) de 2-bromoéthanol et 2,07 g (15 mmol./ 3 éq.) de carbonate de potassium. On porte au reflux la nuit, puis amène à sec. Le résidu est repris au dichlorométhane, lavé à l'eau et séché sur sulfate de magnésium pour donner, après concentration, le produit recherché.

### PREPARATION 26 : 2-[{[(7R)-3,4-Diméthoxybicyclo[4.2.0]octa 1,3,5-trién-7-yl] méthyl}(méthyl)amino]éthanol

Obtenu comme à la préparation 25 mais en remplaçant la {[(7*S*) 3,4-diméthoxybicyclo[4.2.0]octa-1,3,5-trién-7-yl]méthyl}méthylamine par la {[(7R)-3,4-diméthoxybicyclo[4.2.0]octa-1,3,5-trién-7-yl]méthyl}méthylamine.

### PREPARATION 27 : N-{[(7S)-3,4-Diméthoxybicyclo[4.2.0]octa 1,3,5-trién-7-yl] méthyl}N-méthyl-éthane 1,2-diamine

### Stade 1 : [{[{7S)-3,4-Diméthoxybicyclo[4.2.0]octa 1,3,5-trién-7-yl] méthyl} (méthyl)amino]acétonitrile.

A température ambiante, on mélange dans 16 mL de méthylisobutylcétone 1,04 g (5 mmol.) {[(7*S*)-3,4-diméthoxybicyclo[4.2.0]octa-1,3,5-trién-7-yl]méthyl}méthylamine, 0,35 mL (5 mmol./l éq.) de bromoacétonitrile et 2,16 g (20 mmol./ 4 éq.) de carbonate de sodium. On porte au reflux la nuit, puis amène à sec. Le résidu est repris à l'eau et la phase aqueuse est extraite au dichlorométhane. Les phases organiques jointes sont séchées sur sulfate de magnésium pour donner, après concentration, le produit attendu.

### Stade 2 : N-{[(7S)-3,4-Diméthoxybicyclo[4.2.0]octa 1,3,5-trién-7-yl] méthyl} N-méthyl-éthane 1,2-diamine

Sur 0,21 g (5,4 mmol.) d'aluminohydrure de lithium en suspension dans 5 mL de THF, à température ambiante, on coule goutte à goutte une solution de 1,1 g (4,5 mmol.) du composé du stade 1 dans 10mL de THF, puis maintient sous agitation à température ambiante pendant 5h. On refroidit vers 0°C, puis ajoute avec précaution successivement 0,14 mL d'eau, 0,11 mL d'une solution aqueuse à 20% de soude et 0,51 mL d'eau. On laisse agiter la nuit à température ambiante, puis filtre les sels sur fritté et rince au THF. Les filtrats joints sont concentrés pour donner le produit désiré.

### PREPARATION 28 : N-{[(7R)- 3,4-diméthoxybicyclo[4.2.0]octa 1,3,5-trién-7-yl] méthyl} N-méthyl-éthane 1,2-diamine

Obtenu comme à la préparation 27 mais en remplaçant la {[(7S)-3,4-diméthoxybicyclo[4.2.0]octa-1,3,5-trién-7-yl]méthyl}méthylamine par la {[(7R)-3,4-diméthoxybicyclo[4.2.0]octa-1,3,5-trién-7-yl]méthyl}méthylamine.

### PREPARATION 29 : [(4-Méthoxy 3-méthyl-bicyclo[4.2.0]octa 1,3,5-trién-7-yl) méthyl] amine

### Stade 1 : 3-(3-Bromo 4-méthoxy 5-méthylphényl) propanenitrile

On dissout 62,8 g (358,4 mmol.) du 3-(4-méthoxy 5-méthylphényl) propanenitrile dans 390 mL d'acide acétique. On ajoute alors d'un trait 58 ,8 g (716,8 mmol. / 2éq.) d'acétate de sodium, puis goutte à goutte 20,2 mL (394,2 mmol./ 1,1éq.) de brome, et laisse agiter à température ambiante pendant la nuit. On verse alors le milieu réactionnel sur 2 L d'eau, et extrait la phase aqueuse 3 fois par 500 mL de dichlorométhane. Les phases organiques jointes sont lavées successivement par 500 mL d'eau, 500 mL d'une solution aqueuse saturée d'hydrogénocarbonate de sodium et 500 mL d'eau, et séchées sur sulfate de magnésium. Après concentration, on recueille le produit attendu.

### Stade 2 : 4-Méthoxy 3-méthylbicyclo[4.2.0]octa 1,3,5-triène 7-carbonitrile

Sur l'amidure de sodium (925 mmol. / 2,5 éq.) dans 1850 mL d'ammoniac liquide, on coule 94 g (370 mmol.) du composé précédent en solution dans 230 mL d'éther. Après 3 h sous agitation au reflux de l'ammoniac, on bloque le réaction en ajoutant 99g (5 éq.) de chlorure d'ammonium solide, puis on laisse l'ammoniac s'évaporer. Le résidu est repris par 500 mL d'eau et 500 mL de dichlorométhane, on agite, sépare les phases et ré-extrait la phase aqueuse 2 fois par 500 mL de dichlorométhane. Les phases organiques jointes sont séchées sur sulfate de magnésium et concentrées. Le résidu (68 g) est chromatographié sur 800 g de silice (éluant : dichlorométhane / cyclohexane : 50/50) pour donner, après recristallisation dans 150 mL d'éther isopropylique, le produit désiré.

### Stade 3 : [(4-Méthoxy 3-méthyl-bicyclo[4.2.0]octa 1,3,5-trién-7-yl) méthyl] amine

A 4,0 g (23,1 mmol.) du composé précédent en solution dans 75 mL de méthanol et 75 mL de méthanol ammoniacal 7N, on ajoute 3,3 mL de nickel de Raney. On hydrogène à température ambiante et pression ordinaire pendant 2h. On filtre alors sur célite, rince au méthanol et concentre pour obtenir le produit désiré.

### PREPARATION 30 : [(3-Méthoxy-4-méthyl-bicyclo[4.2.0]octa 1,3,5-trién-7-yl)-méthyl]amine

### Stade 1 : 5-Méthoxy 4-méthylbenzaldéhyde

A température ambiante, on mélange 36,35 g (238,8 mmol.) de (3-méthoxy-4-méthylphényl)-méthanol et 207,6 g (2,4 moles / 10 éq.) d'oxyde de manganèse dans 730 mL de dichlorométhane. On maintient la nuit sous agitation à température ambiante, puis filtre sur célite et rince au dichlorométhane. Après concentration des filtrats joints, on obtient le produit recherché.

### Stade 2 : 2-Bromo 5-méthoxy 4-méthylbenzaldéhyde

A température ambiante, on dissout 35,5 g (238,7 mmol.) du composé précédent dans 290 mL de dichlorométhane, ajoute d'un trait 29,4 g (358 mmol./ 1,5 éq.) d'acétate de sodium, puis coule goutte à goutte une solution de 15 mL (286,4 mmol./ 1,2 éq.) de brome dans 130 mL de dichlorométhane. On laisse agiter à température ambiante pendant 4h, filtre l'insoluble, lave le filtrat par une solution normale de thiosulfate de sodium et le sèche sur sulfate de magnésium. Après concentration, le résidu est recristallisé dans 130 mL d'heptane pour conduire au produit désiré.

### Stade 3 : 3-(2-Bromo-5-méthoxy-4-méthylphényl)acrylonitrile

On dissout 4,22 g (183,6 mmol./ 1 éq.) de sodium dans 180 mL d'éthanol anhydre, puis amène vers 0°C. On ajoute alors goutte à goutte 29,7 mL (183,6 mmol./ 1 éq.) de diéthoxyphosphonoacétonitrile. On maintient sous agitation à 0°C pendant 30 mn, ajoute par fractions 42,05 g (183,6 mmol.) du composé précédent, puis laisse agiter à nouveau 30 mn à 0°C, puis à température ambiante pendant 1h. Le milieu réactionnel est versé sur 1800 mL d'eau, on laisse agiter 30 mn, puis filtre le solide, le lave à l'eau et le sèche sous vide pour obtenir le produit désiré.

### Stade 4 : 3-(2-Bromo-5-méthoxy-4-méthylphényl)propanenitrile

A température ambiante, on mélange 46 g (182,5 mmol.) du composé précédent et 27,7 g (730 mmol. / 4 éq.) de borohydrure de sodium dans 380 mL d'isopropanol, puis on porte au reflux pendant 2 jours (on ajoute 1 éq. de borohydrure de sodium après 1 journée). On laisse alors refroidir, puis concentre. Le résidu est repris dans 630 mL d'un mélange eau et glace, puis acidifié jusqu'à pH 1 par addition avec précaution de 90 mL d'acide chlorhydrique concentré. On extrait cette phase aqueuse 2 fois à l'acétate d'éthyle. Les phases organiques jointes sont lavées par une solution aqueuse saturée de chlorure de sodium, puis séchées sur sulfate de magnésium. Après concentration, le résidu est recristallisé de 100 mL d'heptane pour donner le produit désiré.

### Stade 5 : 3-Méthoxy-4-méthylbicyclo[4.2.0]octa 1,3,5-triène 7-carbonitrile

On procède comme au stade 2 de la préparation 29 pour conduire au produit attendu.

### Stade 6 : [(3-Méthoxy-4-méthyl-bicyclo[4.2.0]octa 1,3,5-trién-7-yl)méthyl] amine

On procède comme au stade 3 de la préparation 29 pour conduire au produit attendu.

### EXEMPLE 1 : N-[(3,4-Diméthoxybicyclo[4.2.0]octa-1,3,5-trién-7-yl)méthyl]3-(7,8-diméthoxy-1,2,4,5-tétrahydro-3H-3-benzazépin-3-yl)-N-méthyl-3-oxopropan-1-amine, chlorhydrate

### Stade 1: 7,8-Diméthoxy 1,3,4,5-tetrahydro-2H-3-benzazépin-2-one

A température ambiante, on solubilise 35g (159,6 mmol.) de 7,8-diméthoxy 1,3-dihydro-2*H*-3-benzazépin-2-one dans un mélange de 525 mL d'éthanol et 175 mL d'acétate d'éthyle. On ajoute alors 7 g (20% massique) de palladium sur charbon à 10% et hydrogène à 65°C sous 3,5 bars pendant 22 h. On filtre le catalyseur sur Célite, rince par un mélange éthanol/acétate d'éthyle et évapore à sec pour conduire au produit désiré.

### Stade 2 : Chlorhydrate de la 7,8-diméthoxy 2,3,4,5-tetrahydro-1H-3-benzazépine

On refroidit à 0°C 22,13 g (100 mmol.) du produit obtenu au stade 1 en suspension dans 250 mL de THF. On ajoute alors goutte à goutte en 15 mn, 250 mL de borane/THF (1M dans le THF) et on chauffe à 80°C pendant 24 h. On refroidit et ajoute goutte à goutte 250 mL d'éthanol, puis 150 mL d'éthanol chlorhydrique 2,6N. On porte au reflux 40 mn, puis filtre un léger insoluble et évapore à sec. Le résidu ainsi obtenu est recristallisé dans un mélange de 150 mL d'isopropanol et 20 mL d'eau pour conduire au produit recherché.

### Stade 3 : 3-Acryloyl-7,8-diméthoxy-2,3,4,5-tétrahydro-1H-3-benzazépine

On met en solution à température ambiante 70 g (287,2 mmol.) du produit obtenu au stade 2 dans 1200 mL de dichlorométhane, puis ajoute d'un trait 100 mL (718 mmol. / 2,5 éq.) de triéthylamine. On amène à 0°C et coule goutte à goutte une solution de 25,8 mL (315,9 mmol. / 1,1éq.) de chlorure d'acryloyle dans 270 mL de dichlorométhane, en maintenant la température vers 0°C. Après 2 h à 0°C, on maintient sous agitation la nuit à température ambiante. On lave alors par 750 mL d'eau, 750 mL d'acide chlorhydrique 1N et 750 mL d'eau, puis sèche sur sulfate de magnésium. Après filtration et évaporation, le résidu est chromatographié sur 3,2 kg de silice (éluant : dichlorométhane / éthanol : 95/5), puis recristallisé dans l'isopropanol. Après filtration sur fritté et séchage sous vide à 50°C, on obtient le produit désiré.

### Stade 4 : N-[(3,4-Diméthoxybicyclo[4.2.0]octa-1,3,5-trién-7-yl)méthyl] 3-(7,8-diméthoxy-1,2,4,5-tétrahydro-3H-3-benzazépin-3-yl) N-méthyl 3-oxopropan-1-amine, chlorhydrate

On met en solution dans 5 mL de THF 1,05 g (4,0 mmol.) du produit obtenu au stade 3 et 0,82 g (4,0 mmol.) de {[(7*R*,*S*) 3,4-diméthoxybicyclo[4.2.0]octa-1,3,5-trién-7-yl]méthyl}méthylamine. On porte au reflux (bain d'huile à 90°C) la nuit, sous courant d'azote de sorte que le solvant s'évapore progressivement. Le résidu obtenu est chromatographié directement sur silice (éluant : dichlorométhane / éthanol / ammoniaque : 95/5/0,5). Après solubilisation dans l'isopropanol, on traite par 1,1 éq. d'éther chlorhydrique et maintient sous agitation jusqu'à précipitation. Après séchage, le solide obtenu est concrétisé dans un mélange acétate d'éthyle (10 mL) et acétonitrile (20 mL) pour conduire, après filtration et séchage, au produit attendu.
*Point de fusion* (M. K.) : 176-179°C.

### EXEMPLE 2 : N-{[(7R)-3,4-Diméthoxybicyclo[4.2.0]octa-1,3,5-trién-7-yl]méthyl} 3-(7,8-diméthoxy-1,2,4,5-tétrahydro-3H-3-benzazépin-3-yl) N-méthyl-3-oxopropan-1-amine, chlorhydrate

Obtenu comme le produit de l'exemple 1, mais en remplaçant au stade 4 la [(3,4-diméthoxybicyclo[4.2.0]octa-1,3,5-trién-7-yl)méthyl]méthylamine racémique par la {[(7*R*)-3,4-diméthoxybicyclo[4.2.0]octa-1,3,5-trién-7-yl]méthyl}méthylamine.
*Point de fusion* (M.K.) : 155-158°C.
*Pouvoir rotatoire :* solvant: DMSO, C=0.01g/cm³, T=20°C, L=589nm, α_{D}=-1,46.

### EXEMPLE 3a : N-{[(7S)-3,4-Diméthoxybicyclo[4.2.0]octa-1,3,5-trién-7-yl]méthyl} 3-(7,8-diméthoxy-1,2,4,5-tétrahydro-3H-3-benzazépin-3-yl) N-méthyl-3-oxopropan-1-amine, chlorhydrate

Obtenu comme le produit de l'exemple 1, mais en remplaçant au stade 4 la [(3,4-diméthoxybicyclo[4.2.0]octa-1,3,5-trién-7-yl)méthyl]méthylamine racémique par la {[(7*S*)-3,4-diméthoxybicyclo[4.2.0]octa-1,3,5-trién-7-yl]méthyl}méthylamine.
*Point de fusion* (M.K.): 157-160°C.
*Pouvoir rotatoire :* solvant: DMSO, C=0.01g/cm³, T=20°C, L=589nm, α_{D} =+1,6.
*IR* (cm⁻¹): 2438 (NH⁺), 1625 (C=O), 1234-1203-1179 (C-O-C), 865-846 (CH-Ar).

### EXEMPLE3b:N-{[(7S)-3,4-Diméthoxybicyclo[4.2.0]octa-1,3,5-trién-7-yl]méthyl} 3-(7,8-diméthoxy-1,2,4,5-tétrahydro-3H-3-benzazépin-3-yl) N-méthyl-3-oxopropan-1-amine, fumarate

Le produit attendu est obtenu par salification de la N-{[(7*S*)-3,4-diméthoxybicyclo[4.2.0]octa-1,3,5-trién-7-yl]méthyl} 3-(7,8-diméthoxy-1,2,4,5-tétrahydro-3H-3-benzazépin-3-yl)-N-méthyl-3-oxopropan-1-amine (1,32 g; 2,82 mmol), obtenue par retour base du chlorhydrate obtenu à l'exemple 3a, par l'acide fumarique.

| *Microanalyse élémentaire :* | | | |
|---|---|---|---|
| | *%C* | *%H* | *%N* |
| *Calculé* | *63.68* | *6.90* | *4.79* |
| *Trouvé* | *63.40* | *6.88* | *4.90* |

### EXEMPLE 3c : N-{[(7S)-3,4-Diméthoxybicyclo[4.2.0]octa-1,3,5-trién-7-yl]méthyl} 3-(7,8-diméthoxy-1,2,4,5-tétrahydro-3H-3-benzazépin-3-yl) N-méthyl-3-oxopropan-1-amine, hémipamoate

Le produit attendu est obtenu par salification de la N-{[(7*S*)-3,4-diméthoxybicyclo[4.2.0]octa-1,3,5-trién-7-yl]méthyl} 3-(7,8-diméthoxy-1,2,4,5-tétrahydro-3H-3-benzazépin-3-yl)-N-méthyl-3-oxopropan-1-amine, obtenue par retour base du chlorhydrate obtenu à l'exemple 3a, par l'acide pamoïque.

| *Microanalyse élémentaire :* | | | |
|---|---|---|---|
| | *%C* | *%H* | *%N* |
| *Calculé* | *69.77* | *6.69* | *4.23* |
| *Trouvé* | *69.38* | *6.56* | *3.91* |

### EXEMPLE 3d : N-{[(7S)-3,4-Diméthoxybicyclo[4.2.0]octa-1,3,5-trién-7-yl]méthyl} 3-(7,8-diméthoxy-1,2,4,5-tétrahydro-3H-3-benzazépin-3-yl) N-méthyl-3-oxopropan-1-amine, héminapadisylate

Le produit attendu est obtenu par salification de la N-{[(7*S*)-3,4-diméthoxybicyclo[4.2.0]octa-1,3,5-trién-7-yl]méthyl} 3-(7,8-diméthoxy-1,2,4,5-tétrahydro-3H-3-benzazépin-3-yl)-N-méthyl-3-oxopropan-1-amine, obtenue par retour base du chlorhydrate obtenu à l'exemple 3a, par l'acide 1,5-naphtalènedisulfonique.

| *RMN ¹H :* | | | | |
|---|---|---|---|---|
| (aa) | δ = 10.77 ppm (1H; bs) | (l,l') | δ = 3.29 et 2.77 ppm (2H; dd) | |
| (a) | δ = 6.74 ppm (1H; s) | | J_{ll'} = 13.4 Hz ; | Jₗₖ = 4.7 Hz ; J_{l'k} = 1.9 Hz |
| (b) | δ = 6.70 ppm (1H; s) | (m) | δ = 2.87 ppm (2H; m) | |
| (c) | δ = 6.68 ppm (1H; s) | (n) | δ = 2.86 ppm (2H; m) | |
| (d) | δ = 6.65 ppm (1H; s) | (o) | δ = 2.85 ppm (2H; m) | |
| (e) | δ = 3.874 ppm (3H; s) | (p) | δ = 2.81 ppm (1H; dd) | |
| (f) | δ = 3.871 ppm (3H; s) | | J_{pp'} = 12.5 Hz ; | Jₚₖ = 5.9 Hz |
| (g) | δ = 3.858 ppm (3H; s) | (p') | δ = 2.63 ppm (1H; m) | |
| (h) | δ = 3.851 ppm (3H; s) | (q) | δ = 2.65 ppm (2H; m) | |
| (i) | δ = 3.73 ppm (2H; m) | (r) | δ = 2.40 ppm (3H; s) | |
| (j) | δ = 3.60 ppm (2H; m) | (x) | δ = 2.40 ppm (1H; d) | J_{xz} = 8.7 Hz |
| (k) | δ = 3.59 ppm (1H; m) | (y) | δ = 2.40 ppm (1H; d) | J_{yz} = 7.2 Hz |
| | | (z) | δ= 2.40 ppm (1H; dd) | |

### EXEMPLE4 : N-{[(7S)-3,4-Diméthoxybieyclo[4.2.0]oeta-1,3,5-trién-7-yl]méthyl}-3-(7,8-diméthoxy-1,2,4,5-tétrahydro-3H-3-benzazépin-3-yl)-3-oxopropan-1-amine, chlorhydrate

Obtenu comme le produit de l'exemple 1, mais en remplaçant au stade 4 la [(3,4-diméthoxybicyclo[4.2.0]octa-1,3,5-trién-7-yl)méthyl]méthylamine racémique par la {[(7*S*)-3,4-diméthoxybicyclo[4.2.0]octa-1,3,5-trién-7-yl]méthyl}amine et le THF par le dioxanne.
*Point de fusion* (M.K.) : 177-180°C.
*Pouvoir rotatoire:* solvant: MeOH, C=0.011g/cm³, T=20°C, L=589nm, α_{D} =-4.15.
*IR* (cm⁻¹) : 2723 (NH⁺), 1645 (C=O).

### EXEMPLE 5 : N-[(4,5-Diméthoxybicyclo[4.2.0]octa-1,3,5-trién-7-yl)méthyl]-3-(7,8-diméthoxy-1,2,4,5-tétrahydro-3H-3-benzazépin-3-yl)-3-oxopropan-1-amine, chlorhydrate

Obtenu comme le produit de l'exemple 1, mais en remplaçant au stade 4 la [(3,4-diméthoxybicyclo[4.2.0]octa-1,3,5-trién-7-yl)méthyl]méthylamine par le produit de la préparation 1 et le THF par le dioxanne.
*Point de fusion* (M.K.) : 199-201°C.
*IR* (cm⁻¹): 1645 (C=O), 3000 à 2400 (NH₂⁺).

### EXEMPLE 6 : N-[2-(5,6-Diméthoxy-2,3-dihydro-1H-indén-2-yl)éthyl]-3-(7,8-diméthoxy-1,2,4,5-tétrahydro-3H-3-benzazépin-3-yl)-N-méthyl-3-oxopropan-1-amine, chlorhydrate

Obtenu comme le produit de l'exemple 1, mais en remplaçant au stade 4 la [(3,4-diméthoxybicyclo[4.2.0]octa-1,3,5-trién-7-yl)méthyl]méthylamine par la [2-(5,6-diméthoxy-2,3-dihydro-1*H-*indén-2-yl)éthyl]méthylamine.
*Point de fusion* (M.K.) = 193-199°C

### EXEMPLE 7 : N-[(5,6-Diméthoxy-2,3-dihydro-1H-indén-2-yl)méthyl]-3-(7,8-diméthoxy-1,2,4,5-tétrahydro-3H-3-benzazépin-3-yl)-N-méthyl-3-oxopropan-1-amine, chlorhydrate

Obtenu comme le produit de l'exemple 1, mais en remplaçant au stade 4, la [(3,4-diméthoxybicyclo[4.2.0]octa-1,3,5-trién-7-yl)méthyl]méthylamine par la [(5,6-diméthoxy-2,3-dihydro-1*H* indén-2-yl)méthyl]méthylamine.
*Point de fusion* (M.K.) = 194-197°C

### EXEMPLE 8 : N-[(2,3-Diméthoxybicyclo[4.2.0]octa-1,3,5-trién-7-yl)méthyl] 3-(7,8-diméthoxy-1,2,4,5-tétrahydro-3H-3-benzazépin-3-yl)-3-oxopropan-1-amine, chlorhydrate

Obtenu comme le produit de l'exemple 1, mais en remplaçant au stade 4, la [(3,4-diméthoxybicyclo[4.2.0]octa-1,3,5-trién-7-yl)méthyl]méthylamine par le produit de la préparation 10.
*Point de fusion* (M.K.) = 220-222°C

### EXEMPLE 9 : N-[(2,3,4-Triméthoxybicyclo[4.2.0]octa-1,3,5-trién-7-yl)méthyl] 3-(7,8-diméthoxy-1,2,4,5-tétrahydro-3H-3-benzazépin-3-yl)-3-oxopropan-1-amine, hémifumarate

Obtenu comme le produit de l'exemple 1, mais en remplaçant au stade 4, la [(3,4-diméthoxybicyclo[4.2.0]octa-1,3,5-trién-7-yl)méthyl]méthylamine par le produit de la préparation 11. La salification est effectuée en présence d'acide fumarique.
*Point de fusion* (M.K.) = 155-157°C

### EXEMPLE 10 : 3-(7,8-Diméthoxy-1,2,4,5-tétrahydro-3H-3-benzazépin-3-yl)-N-[(5-méthoxybicyclo[4.2.0]octa-1,3,5-trién-7-yl)méthyl] 3-oxopropan-1-amine, chlorhydrate

Obtenu comme le produit de l'exemple 1, mais en remplaçant au stade 4 la [(3,4-diméthoxybicyclo[4.2.0]octa-1,3,5-trién-7-yl)méthyl]méthylamine par le produit de la préparation 9 et le THF par le dioxanne.
*Point de fusion* (M.K.)= 185-187°C

### EXEMPLE 11 : N-{[(7S)-3,4-Diméthoxybicyclo[4.2.0]octa-1,3,5-trién-7-yl]méthyl}-3-(7-méthoxy-1,2,4,5-tétrahydro-3H-3-benzazépin-3-yl)-3-oxopropan-1-amine, chlorhydrate

Obtenu comme le produit de l'exemple 1, mais en remplaçant au stade 1 la 7,8-diméthoxy-1,3-dihydro-2*H-*3-benzazépin-2-one par la 8-méthoxy-1,3-dihydro-2H-3-benzazépin-2-one et au stade 4 la (3,4-diméthoxybicyclo[4.2.0]octa-1,3,5-trién-7-yl)méthyl]méthylamine racémique par la {[(7*S*)-3,4-diméthoxybicyclo[4.2.0]octa-1,3,5-trién-7-yl]méthyl}amine et le THF par le dioxanne.
*Point de fusion* (M.K.)= 171-173°C
*IR* (cm⁻¹): 1636 (C=O), 3000 à 2400 (NH₂⁺).

### EXEMPLE 12 : N-[2-(5,6-Diméthoxy-2,3-dihydro-1H-indén-2-yl)méthyl]-3-(7,8-diméthoxy-1,2,4,5-tétrahydro-3H-3-benzazépin-3-yl)-3-oxopropan-1-amine, chlorhydrate

### Stade 1 : N-Benzyl 3-(7,8-diméthoxy 1,2,4,5-tétrahydro-3H-3-benzazépin-3-yl) 3-oxopropan-1-amine

On procède comme au stade 4 de l'exemple 1, mais en remplaçant la [(3,4-diméthoxybicyclo[4.2.0]octa-1,3,5-trién-7-yl)méthyl]méthylamine par la benzylamine.
*Point de fusion* (M.K.) = 100-102°C.

### Stade 2 : N-Benzyl N-[(5,6-diméthoxy-2,3-dihydro-1H-indén-2-yl)méthyl] 3-(7,8-diméthoxy 1,2,4,5-tétrahydro-3H-3-benzazépin-3-yl) 3-oxopropan-1-amine

On solubilise dans 20 mL d'acétonitrile 1,7 g (4,6 mmoles) du produit du stade 1 et 1,25 g (1 équivalent) du produit de la préparation 24. On ajoute 5,1 g (8 équivalents) de K₂CO₃ et on chauffe à reflux pendant 24 heures. On refroidit, filtre et évapore le filtrat. On obtient un résidu qui est purifié par chromatographie sur silice (éluant : CH₂Cl₂ / Éthanol : 95/5) pour conduire au produit attendu sous forme d'huile.

### Stade 3 : N-[2-(5,6-Diméthoxy-2,3-dihydro-1H-indén-2-yl)méthyl]-3-(7,8-diméthoxy-1,2,4,5-tétrahydro-3H-3-benzazépin-3-yl)-3-oxopropan-1-amine, chlorhydrate

On solubilise 2,3 g (4,1 mmoles) du produit obtenu au stade 2 dans 46 mL d'éthanol. On ajoute 230 mg de Pd/C à 5%, et on hydrogène sous 1 bar de pression et à température ambiante pendant 24 heures. On filtre sur Célite, rince à l'éthanol et évapore à sec le filtrat. On obtient 1,95 g d'un résidu qui est solubilisé dans 15 mL d' éthanol. On ajoute 2,5 mL d'une solution Et₂O-HCl (2M). Un produit cristallise qui est filtré et séché. On obtient le produit attendu sous la forme d'un solide blanc.
*Point de fusion* (M.K.) = 198-204°C

### EXEMPLE 13 : 3-(7,8-Diméthoxy-1,2,4,5-tétrahydro-3H-3-benzazépin-3-yl)-N-[(2-méthoxybicyclo[4.2.0]octa-1,3,5-trién-7-yl)méthyl]-3-oxopropan-1-amine, chlorhydrate

On mélange à température ambiante 0,93 g (3,6 mmol./ 1 éq.) du produit du stade 3 de l'exemple 1, 0,58 g (3,6 mmol.) du produit de la préparation 2 et 0,11 g (0,18 mmol./ 0,05 éq.) de triflate d'ytterbium dans 10 mL de toluène. On chauffe à 100°C jusqu'à la fin de la réaction (estimée par C.C.M), puis évapore à sec. Le résidu obtenu est chromatographié directement sur silice (éluant : dichlorométhane / éthanol / ammoniaque : 95/5/0,5) pour donner le produit attendu sous forme base. Après solubilisation dans 10 mL d'acétonitrile, on traite par 0,56 mL (1,1 éq.) d'éthanol chlorhydrique 3,5N pour obtenir, après filtration et séchage, le produit attendu.
*Point de fusion* (M.K.) : 202-204°C.
*IR* (cm⁻¹): 1641 (C=O), 3000 à 2500 (NH₂⁺).

### EXEMPLE 14 : 3-(7,8-Diméthoxy-1,2,4,5-tétrahydro-3H-3-benzazépin-3-yl)-N-[(3-éthoxybicyclo[4.2.0]octa-1,3,5-trién-7-yl)méthyl]-3-oxopropan-1-amine, chlorhydrate

Obtenu de la même façon que le produit de l'exemple 13, mais en remplaçant le produit de la préparation 2 par celui de la préparation 5.
*Point de fusion* (M.K.): 221-225°C

### EXEMPLE 15 : N-[(3-tert-Butoxybicyclo[4.2.0]octa-1,3,5-trién-7-yl)méthyl]- 3-(7,8-diméthoxy-1,2,4,5-tétrahydro-3H-3-benzazépin-3-yl)-3-oxopropan-1-amine, hémifumarate

Obtenu de la même façon que le produit de l'exemple 13, mais en remplaçant le produit de la préparation 2 par celui de la préparation 6 et en utilisant comme agent de salification l'acide fumarique à la place de l'acide chlorhydrique.
*Point de fusion* (M .K.) =189-191°C

### EXEMPLE 16 : N-(5,6-Dihydrocyclobuta[f][1,3]benzodioxol-5-ylméthyl)-3-(7,8-diméthoxy-1,2,4,5-tétrahydro-3H-3-benzazépin-3-yl)-N-méthyl-3-oxopropan-1-amine, chlorhydrate

Obtenu de la même façon que le produit de l'exemple 13, mais en remplaçant le produit de la préparation 2 par celui de la préparation 7.
*Point de fusion =* 180-184°C

### EXEMPLE 17 : N-(5,6-Dibydrocyclobuta[f][1,3]benzodioxol-5-ylméthyl)-3-(7,8-diméthoxy-1,2,4,5-tétrahydro-3H-3-benzazépin-3-yl)-3-oxopropan-1-amine, chlorhydrate

Obtenu de la même façon que le produit de l'exemple 13, mais en remplaçant le produit de la préparation 2 par la (5,6-dihydrocyclobuta[4,5] benzo [1,2-*d*][1,3]dioxol 5-ylméthyl)amine.
*Point de fusion* (M.K.) = 201-207°C.

### EXEMPLE 18 : N-[2-(5,6-Diméthoxy-2,3-dihydro-1H-indén-2-yl)éthyl]-3-(7,8-diméthoxy-1,2,4,5-tétrahydro-3H-3-benzazépin-3-yl)-3-oxopropan-1-amine, chlorhydrate

Obtenu de la même façon que le produit de l'exemple 13, mais en remplaçant le produit de la préparation 2 par celui de la préparation 8.
*Point de fusion* (M.K.) = 186-189°C

### EXEMPLE 19 : 7-({[3-(7,8-Diméthoxy-1,2,4,5-tétrahydro-3H-3-benzazépin-3-yl)-3-oxopropyl]amino}méthyl)bicyclo[4.2.0]octa-1,3,5-trién-3-yl trifluorométhanesulfonate, chlorhydrate

Obtenu de la même façon que le produit de l'exemple 13, mais en remplaçant le produit de la préparation 2 par celui de la préparation 21.
*Point de fusion* (M.K.): 162-168°C

### EXEMPLE 20 : 7-({[3-(7,8-Diméthoxy-1,2,4,5-tétrahydro-3H-3-benzazépin-3-yl)-3-oxopropyl]amino}méthyl)bieyclo[4.2.0]oeta-1,3,5-trién-3-yl diméthylsulfamate, chlorhydrate

Obtenu de la même façon que le produit de l'exemple 13, mais en remplaçant le produit de la préparation 2 par celui de la préparation 22.
*Point de fusion* (M.K.): 228-235°C

### EXEMPLE 21 : N-(5,6-Dihydrocyclobuta[f][1]benzofuran-6-ylméthyl)-3-(7,8-diméthoxy-1,2,4,5-tétrahydro-3H-3-benzazépin-3-yl)-3-oxopropan-1-amine, chlorhydrate

Obtenu de la même façon que le produit de l'exemple 13, mais en remplaçant le produit de la préparation 2 par celui de la préparation 23.
*Point de fusion* (M .K .) : 245-255°C

### EXEMPLE 22 : N-(Cyclopropylméthyl)-N-{[(7S)-3,4-diméthoxybieyclo[4.2.0]oeta-1,3,5-trién-7-yl]méthyl}-3-(7,8-diméthoxy-1,2,4,5-tétrahydro-3H-3-benzazépin-3-yl)-3-oxopropan-1-amine, chlorhydrate

Obtenu de la même façon que le produit de l'exemple 13, mais en remplaçant le produit de la préparation 2 par le produit de la préparation 12, et le toluène par le dioxanne.
*Point de fusion* (M.K.) = 88-92°C
*Pouvoir rotatoire:* solvant: MeOH, C=0.01g/cm³, T=20°C, L=589nm, α_{D} =-13.25
*IR* (cm⁻¹): 1632 (C=O), 3420 (H₂O), 3000 à 2200 (NH⁺).

### EXEMPLE 23 : N-Allyl-N-{[(7S)-3,4-diméthoxybieyclo[4.2.0]oeta-1,3,5-trién-7-yl]méthyl}-3-(7,8-diméthoxy-1,2,4,5-tétrahydro-3H-3-benzazépin-3-yl)-3-oxopropan-1-amine, chlorhydrate

Obtenu de la même façon que le produit de l'exemple 13, mais en remplaçant le produit de la préparation 2 par le produit de la préparation 18, et le toluène par le dioxanne.
*Point de fusion* (M.K.) = 74-80°C.
*Pouvoir rotatoire:* solvant: MeOH, C=0.01g/cm³, T=20°C, L=589nm, α_{D} =-4.6
*IR* (cm⁻¹) : 1631 (C=O), 3400 (H₂O), 3000 à 2000 (NH⁺).

### EXEMPLE 24 : N-{[(7S)-3,4-Diméthoxybicyclo[4.2.0]octa-1,3,5-trién-7-yl]méthyl}-3-(7,8-diméthoxy-1,2,4,5-tétrahydro-3H-3-benzazépin-3-yl)-N-éthyl-3-oxopropan-1-amine, hémifumarate

Obtenu de la même façon que le produit de l'exemple 13, mais en remplaçant le produit de la préparation 2 par le produit de la préparation 13, et le toluène par le dioxanne. La salification est effectuée par l'acide fumarique.
*Point de fusion* (M.K.) = 71-73°C.
*Pouvoir rotatoire:* solvant: MeOH, C=0.01g/cm³, T=20°C, L=589nm, α_{D} =-6.67 *IR* (cm⁻¹) : 2200 à 2700 (NH⁺/OH), 1701-1631 (C=O), 1276-1203 (C-O-C).

### EXEMPLE 25 : N-{[(7S)-3,4-Diméthoxybieyclo[4.2.0]oeta-1,3,5-trién-7-yl]méthyl}-3-(7,8-diméthoxy-1,2,4,5-tétrahydro-3H-3-benzazépin-3-yl)-N-(2,2,2-trifluoroéthyl)-3-oxopropan-1-amine, chlorhydrate

Obtenu de la même façon que le produit de l'exemple 13, mais en remplaçant le produit de la préparation 2 par le produit de la préparation 17, et le toluène par le dioxanne.
*Point de fusion* (M.K.) = 68-70°C.
*Pouvoir rotatoire:* solvant: MeOH, C=0.01g/cm³, T=20°C, L=589nm, α_{D} =-8.04
*IR* (cm⁻¹): 1633 (C=O), 2800 à 1900 (-NH⁺), 1107 à 1206 (-CF₃).

### EXEMPLE 26 : N-Benzyl-N-{[(7S)-3,4-diméthoxybieyclo[4.2.0]oeta-1,3,5-trién-7-yl]méthyl}-3-(7,8-diméthoxy-1,2,4,5-tétrahydro-3H-3-benzazépin-3-yl)-3-oxopropan-1-amine, fumarate

Obtenu de la même façon que le produit de l'exemple 13, mais en remplaçant le produit de la préparation 2 par le produit de la préparation 14, et le toluène par le dioxanne. La salification est effectuée par l'acide fumarique.
*Point de fusion* (M .K.): 146-148°C
*Pouvoir rotatoire:* solvant: MeOH, C=0.011g/cm³, T=20°C, L=589nm, α_{D} =+2.07
*IR* (cm⁻¹): 1708 et 1646 (C=O), 3000 à 2500 (-OH).

### EXEMPLE 27 : N-Cyclopentyl-N-{[(7S)-3,4-diméthoxybieyclo[4.2.0]oeta-1,3,5-trién-7-yl]méthyl}-3-(7,8-diméthoxy-1,2,4,5-tétrahydro-3H-3-benzazépin-3-yl)-3-oxopropan-1-amine, fumarate

Obtenu de la même façon que le produit de l'exemple 13, mais en remplaçant le produit de la préparation 2 par le produit de la préparation 19, et le toluène par le dioxanne.
*Point de fusion* (M .K .) : 79-82°C.
*Pouvoir rotatoire:* solvant: MeOH, C=0.01g/cm³, T=20°C, L=589nm, α_{D} =-10.54
*IR* (cm⁻¹): 1701 et 1635 (C=O), 3000 à 2500 (-OH).

### EXEMPLE 28 : N-(Cyclopentylméthyl)-N-{[(7S)-3,4-diméthoxybicyclo[4.2.0]octa-1,3,5-trién-7-yl]méthyl}-3-(7,8-diméthoxy-1,2,4,5-tétrahydro-3H-3-benzazépin-3-yl)-3-oxopropan-1-amine, chlorhydrate

Obtenu de la même façon que le produit de l'exemple 13, mais en remplaçant le produit de la préparation 2 par le produit de la préparation 15, et le toluène par le dioxanne.
*Point de fusion* (M.K.): 87-90°C.
*Pouvoir rotatoire:* solvant: MeOH, C=0.008g/cm³, T=20°C, L=589nm, α_{D} =-19.07
*IR* (cm⁻¹) : 2454 (-NH⁺), 1633 (C=O), 1279-1205 (C-O-C).

### EXEMPLE 29 : N-(Cyclobutylméthyl)-N-{[(7S)-3,4-diméthoxybicyclo[4.2.0]octa-1,3,5-trién-7-yl]méthyl}-3-(7,8-diméthoxy-1,2,4,5-tétrahydro-3H-3-benzazépin-3-yl)-3-oxopropan-1-amine, fumarate

Obtenu de la même façon que le produit de l'exemple 13, mais en remplaçant le produit de la préparation 2 par le produit de la préparation 16, et le toluène par le dioxanne. La salification se fait en présence d'acide fumarique.
*Point de fusion* (M .K.): 68-71°C.
*Pouvoir rotatoire:* solvant: MeOH, C=0.01g/cm³, T=20°C, L=589nm, α_{D} =-12.04
*IR* (cm⁻¹): 1703 et 1634 (C=O), 1279-1072 (C-O-C).

### EXEMPLE 30 : N-Cyclobutyl)-N-{[(7S)-3,4-diméthoxybicyclo[4.2.0]octa-1,3,5-trién-7-yl]méthyl}-3-(7,8-diméthoxy-1,2,4,5-tétrahydro-3H-3-benzazépin-3-yl)-3-oxopropan-1-amine, chlorhydrate

Dans 7 ml de dichlorométhane, à température ambiante, on mélange 1 g (2,2 mmol.) de *N-*{[(7*S*)-3,4-diméthoxybicyclo[4.2.0]octa-1,3,5-trién-7-yl]méthyl}-3-(7,8-diméthoxy-1,2,4,5-tétrahydro-3H-3-benzazépin-3-yl)-3-oxopropan-1-amine (obtenu par retour à la base libre du composé de l'exemple 4) et 0,17 mL (2,2 mmol./ 1 éq.) de cyclobutanone. On ajoute ensuite 0,7 g (3,3 mmol./1,5 éq.) de triacétoxyborohydrure de sodium, puis 0,13 mL (2,2 mmol./ 1 éq.) d'acide acétique, et on laisse agiter à température ambiante pendant 5 h. La réaction étant incomplète, on rajoute 0,08 mL (0,5 éq.) de cyclobutanone et 350 mg (0,75 éq.) de triacétoxyborohydrure de sodium, et laisse agiter à température ambiante pendant 1h45 de plus. On ajoute alors 10 ml d'une solution aqueuse normale de soude, puis extrait 2 fois par 15 mL d'éther. Les phases organiques jointes sont lavées par 10 mL d'eau, puis 10 mL d'une solution aqueuse saturée de chlorure de sodium, puis séchées sur sulfate de magnésium. Après filtration et concentration, le résidu (1 g) est chromatographié sur une colonne de 50 g de silice (éluant : dichlorométhane / éthanol / ammoniaque : 98/2/0,2) pour donner le produit désiré sous forme base. Celle-ci est solubilisée dans 9 mL d'acétate d'éthyle et 12 mL d'éther, et salifiée par 0,63 mL d'éther chlorhydrique 2 N pour conduire au produit attendu.
*Point de fusion* (M .K.): 93-95°C.
*Pouvoir rotatoire:* solvant: MeOH, C=0.01g/cm³, T=20°C, L=589nm, α_{D} =-5.63
*IR* (cm⁻¹): 2424 (-NH⁺), 1633 (C=O), 1205-1107 (C-O-C).

### EXEMPLE 31 : N-{[(7R)-3,4-Diméthoxybicyclo[4.2.0]octa-1,3,5-trién-7-yl]méthyl} N-méthyl-3-(6,7,8-triméthoxy-1,2,4,5-tétrahydro-3H-3-benzazépin-3-yl)- 3-oxopropan-1-amine, chlorhydrate

Obtenu de la même façon que le produit de l'exemple 13, mais en remplaçant le produit du stade 3 de l'exemple 1 par la 3-acryloyl-6,7,8-triméthoxy-2,3,4,5-tétrahydro-1*H-*3-benzazépine, le produit de la préparation 2 par la {[(7R)-3,4-diméthoxybicyclo[4.2.0]octa-1,3,5-trién-7-yl]méthyl}méthylamine, et le toluène par le THF.
*Point de fusion* (M.K.): 83-86°C.

### EXEMPLE 32 : N-{[(7S)-3,4-Diméthoxybieyclo[4.2.0]octa-1,3,5-trién-7-yl]méthyl} N-méthyl-3-(6,7,8-triméthoxy-1,2,4,5-tétrahydro-3H-3-benzazépin-3-yl)- 3-oxopropan-1-amine, chlorhydrate

Obtenu de la même façon que le produit de l'exemple 13, mais en remplaçant le produit du stade 3 de l'exemple 1 par la 3-acryloyl-6,7,8-triméthoxy-2,3,4,5-tétrahydro-1*H-*3-benzazépine, le produit de la préparation 2 par la {[(7*S*)-3,4-diméthoxybicyclo[4.2.0]octa-1,3,5-trién-7-yl]méthyl}méthylamine, et le toluène par le THF.
*Point de fusion* (M .K.): 83-87°C.
*Pouvoir rotatoire:* solvant: MeOH, C=0.01g/cm³, T=20°C, L=589nm, α_{D} =-3.00
*IR* (cm⁻¹): 1633 (C=O), 3100 à 2000 (-NH⁺), 3600 à 3100 (-OH).

### EXEMPLE 33 : N-{[(7R)-3,4-Diméthoxybicyclo[4.2.0]octa-1,3,5-trién-7-yl]méthyl} 3-(7-méthoxy-1,2,4,5-tétrahydro-3H-3-benzazépin-3-yl)-N-méthyl-3-oxopropan-1-amine, chlorhydrate

Obtenu de la même façon que le produit de l'exemple 13, mais en remplaçant le produit du stade 3 de l'exemple 1 par la 3-acryloyl-7-méthoxy 2,3,4,5-tétrahydro-1*H*-3-benzazépine, le produit de la préparation 2 par la {[(7*R*)-3,4-diméthoxybicyclo[4.2.0]octa-1,3,5-trién-7-yl]méthyl}méthylamine, et le toluène par le THF.
*Point de fusion* (M .K.): 126-129°C.
*Pouvoir rotatoire:* solvant: MeOH, C=0.0126g/cm³, T=20°C, L=589nm, α_{D} =+2.67

### EXEMPLE 34 : N-{[(7S)-3,4-Diméthoxybieyelo[4.2.0]oeta-1,3,5-trién-7-yl]méthyl} 3-(7-méthoxy-1,2,4,5-tétrahydro-3H-3-benzazépin-3-yl)-N-méthyl-3-oxopropan-1-amine, chlorhydrate

Obtenu de la même façon que le produit de l'exemple 13, mais en remplaçant le produit du stade 3 de l'exemple 1 par la 3-acryloyl-7-méthoxy 2,3,4,5-tétrahydro-1*H-*3-benzazépine, le produit de la préparation 2 par la {[(7*S*)-3,4-diméthoxybicyclo[4.2.0]octa-1,3,5-trién-7-yl]méthyl}méthylamine, et le toluène par le THF.
*Point de fusion* (M.K.): 127-129°C.
*Pouvoir rotatoire :* solvant: MeOH, C=0.01g/cm³, T=20°C, L=589nm, α_{D} =-2. 7
*IR* (cm⁻¹) : 1629 (C=O), 3000 à 1800 (-NH⁺), 3500 (-OH) (faible).

### EXEMPLE 35 : 7-({[3-(7,8-Diméthoxy-1,2,4,5-tétrahydro-3H-3-benzazépin-3-yl)-3-oxopropyl]amino}méthyl)bicyclo[4.2.0]octa-1,3,5-trién-3-ol, chlorhydrate

On met en solution dans 19 g de PEG 300, 10 g (38,3 mmol./ 0,95 éq.) du produit du stade 3 de l'exemple 1, 6 g (40,2 mmol.) du produit de la préparation 3 et 42 mg (0,2 mmol./0,05 éq.) de trichlorure de ruthénium. On chauffe à 40°C pendant 4 h. Le milieu réactionnel est repris par 600 mL de dichlorométhane et lavé 2 fois par 500 mL, puis 2 fois par 800 mL d'eau. La phase organique est séchée sur sulfate de magnésium. Après concentration, le résidu est chromatographié sur 500 g de silice (éluant : dichlorométhane / éthanol / ammoniaque 95/5/0,5) pour donner le produit attendu sous forme base. 1 g de la base pure est concrétisé dans 9 mL d'éthanol, puis salifié par 1,1 éq. d'éther chlorhydrique 2N dans 9 mL d'acétonitrile, puis enfin recristallisé dans un mélange d'éthanol (27 mL) et d'eau (4 mL) pour conduire, après filtration et séchage, au produit attendu sous forme de chlorhydrate.
*Point de fusion* (M.K.): 212-215°C.
*IR* (cm⁻¹): 1633 (C=O), 3400 à 2400 (-NH₂⁺/OH).

### EXEMPLE 36 : 8-({[3-(7,8-Diméthoxy-1,2,4,5-tétrahydro-3H-3-benzazépin-3-yl)-3 oxopropyl]amino}méthyl)bicyclo[4.2.0]octa-1,3,5-trién-3-ol

Obtenu de la même façon que le produit de l'exemple 35, mais en remplaçant le produit de la préparation 3 par celui de la préparation 4.
*Point de fusion* (M.K.): 193-195°C
*IR* (cm⁻¹): 1627 (C=O), 3700 à 2200 (-NH₂⁺/OH).

### EXEMPLE 37 : N-[(Bicyclo[4.2.0]octa-1,3,5-trién-7-yl)méthyl] 3-(7,8-diméthoxy-1,2,4,5-tétrahydro-3H-3-benzazépin-3-yl)-3-oxopropan-1-amine, chlorhydrate

Obtenu de la même façon que le produit de l'exemple 35, mais en remplaçant le produit de la préparation 3 par la (bicyclo[4.2.0]octa 1,3,5-trién-7-yl méthyl)amine.
*Point de fusion* (M.K.) :189-193°C

### EXEMPLE 38 : 3-(7,8-Diméthoxy-1,2,4,5-tétrahydro-3H-3-benzazépin-3-yl)-N-{[4-méthoxy-3-méthyl-bicyclo[4.2.0]octa-1,3,5-trién-7-yl]méthyl}-3-oxopropan-1-amine, chlorhydrate

Obtenu de la même façon que le produit de l'exemple 35, mais en remplaçant le produit de la préparation 3 par celui de la préparation 29.
*Point de fusion* (M.K.): 166-168°C

### EXEMPLE 39 : 3-(7,8-Diméthoxy-1,2,4,5-tétrahydro-3H-3-benzazépin-3-yl)-N-{[3-méthoxy-4-méthyl-bicyclo[4.2.0]octa-1,3,5-trién-7-yl]méthyl}-3-oxopropan-1-amine, chlorhydrate

Obtenu de la même façon que le produit de l'exemple 35, mais en remplaçant le produit de la préparation 3 par celui de la préparation 30.
*Point de fusion* (M .K.): 211-214°C

### EXEMPLE 40 : N-{[(7S)-3,4-Diméthoxybieyclo[4.2.0]oeta-1,3,5-trién-7-yl]méthyl} 3-(7,8-diméthoxy-1,2,4,5-tétrahydro-3H-3-benzazépin-3-yl) N-méthyl-4-oxobutan-1-amine, chlorhydrate

### Stade 1 : 4-(7,8-Diméthoxy 1,2,4,5-tétrahydro-3H-3-benzazépin-3yl) 4-oxobutan-1-ol

A 6,0 g (29 mmol.) de 7,8-diméthoxy 2,3,4,5-tétrahydro-1*H-*3-benzazépine dans 30 mL de THF, à 0°C, on ajoute goutte à goutte en 15 mn, 11,6 mL (29 mmol./ l éq.) d'une solution de n-butyl lithium 2,6 M dans l'hexane. On refroidit ensuite vers -78°C et coule goutte à goutte en 10 mn une solution de 2,7 mL (34,8 mmol./ 1,2 éq.) de gamma-butyrolactone dans 15 mL de THF. On laisse agiter 30 mn à -78°C, puis amène et maintient 1h à -30°C, puis laisse revenir à température ambiante et agite 48h à cette température. On verse dans 75 mL d'une solution aqueuse saturée de chlorure d'ammonium, puis extrait 2 fois par 120 mL d'acétate d'éthyle. Les phases organiques jointes sont lavées par 150 mL d'une solution aqueuse saturée de chlorure de sodium, séchées sur sulfate de magnésium et concentrées. Le résidu est repris par 300 mL d'acétate d'éthyle et lavé 2 fois par 75 mL d'une solution aqueuse molaire d'acide chlorhydrique. Les phases aqueuses acides jointes sont ré-extraites 2 fois par 100 mL de dichlorométhane. Les phases organiques sont jointes, séchées sur sulfate de magnésium et concentrées pour donner le produit désiré.

### Stade 2 : 4-(7,8-Diméthoxy 1,2,4,5-tétrahydro-3H-3-benzazépin-3yl) 4-oxobutanal

Sur 2,0 mL (23 mmol./ 3 éq.) de chlorure d'oxalyle dans 45 mL de dichlorométhane , à - 60°C, on ajoute goutte à goutte 3,3 mL (46 mmol./ 6 éq.) de DMSO. On amène vers -25°C et ajoute goutte à goutte une solution de 2,25 g (7,7 mmol.) du composé du stade 1 dans 22 mL de dichlorométhane. On maintient 15 mn sous agitation à -25°C, puis ajoute goutte à goutte 6,5 mL (46 mmol./ 6 éq.) de triéthylamine et laisse revenir doucement à température ambiante. On ajoute alors 75 mL de dichlorométhane et 75 mL d'eau, agite, sépare les phases et lave la phase organique successivement par 75 mL d'une solution aqueuse normale d'acide chlorhydrique, 75 mL d'eau, 75 mL d'une solution aqueuse normale de soude et 75 mL d'eau. Après séchage sur sulfate de magnésium et concentration, on recueille le produit désiré.

### Stade 3 : N-{[(7S)-3,4-Diméthoxybicyclo[4.2.0]octa-1,3,5-trién-7-yl]méthyl} 3-(7,8-diméthoxy-1,2,4,5-tétrahydro-3H-3-benzazépin-3-yl) N-méthyl-4-oxobutan-1-amine, chlorhydrate

Dans 90 mL de dichlorométhane, à température ambiante, on mélange 2,53 g (12,2 mmol.) de {[(7*S*)-3,4-diméthoxybicyclo[4.2.0]octa-1,3,5-trién-7-yl] méthyl} méthylamine et 3,95 g (12,2 mmol./ 1 éq.) du composé stade 2. On ajoute ensuite 3,88 g (18,3 mmol./ 1,5 éq.) de triacétoxyborohydrure de sodium et laisse sous agitation à température ambiante pendant 4 h. On ajoute alors 60 mL d'une solution aqueuse normale de soude, puis extrait 2 fois par 150 mL d'acétate d'éthyle. Les phases organiques jointes sont lavées par 100 mL d'eau, puis 100 mL d'une solution aqueuse saturée de chlorure de sodium, puis séchées sur sulfate de magnésium. Après filtration et concentration, le résidu est chromatographié sur 300 g de silice (éluant : dichlorométhane / éthanol / ammoniaque : 97/3/0,3) pour donner le produit désiré sous forme base. Celle-ci est solubilisée dans 13 mL d'isopropanol et salifiée par 0,67 mL d'éthanol chlorhydrique 4,8 N, puis recristallisée de 25 mL d'acétate d'éthyle pour donner le produit attendu.
*Point de fusion* (M.K.): 132-136°C.
*Pouvoir rotatoire:* solvant: MeOH, C=0.012g/cm³, T=20°C, L=578nm, α_{D} =+4.28.
*IR* (cm⁻¹): 3500 (OH), 2441 (NH⁺), 1610 (C=O), 1278-1234-1204 (C-O-C), 865-845 (CH-Ar).

### EXEMPLE 41 : N-{[(7R)-3,4-Diméthoxybicyclo[4.2.0]octa-1,3,5-trién-7-yl]méthyl} 3-(7,8-diméthoxy-1,2,4,5-tétrahydro-3H-3-benzazépin-3-yl) N-méthyl-4-oxobutan-1-amine, chlorhydrate

Obtenu de la même manière que le produit de l'exemple 40 mais en remplaçant au stade 3 la {[(7*S*)-3,4-diméthoxybicyclo[4.2.0]octa-1,3,5-trién-7-yl] méthyl} méthylamine par la {[(7*R*) 3,4-diméthoxybicyclo[4.2.0]octa-1,3,5-trién-7-yl] méthyl}méthylamine (2 équivalents).
*Point de fusion* (M .K.): 106-137°C.

### EXEMPLE 42 : N-{[(7S)-3,4-Diméthoxybicyclo[4.2.0]octa-1,3,5-trién-7-yl]méthyl} 3-(7,8-diméthoxy-1,2,4,5-tétrahydro-3H-3-benzazépin-3-yl)-4-oxobutan-1-amine, chlorhydrate

Obtenu de la même manière que le produit de l'exemple 40 mais en remplaçant au stade 3 la {[(7*S*)-3,4-diméthoxybicyclo[4.2.0]octa-1,3,5-trién-7-yl] méthyl} méthylamine par la {[(7*S*) 3,4-diméthoxybicyclo[4.2.0]octa-1,3,5-trién-7-yl] méthyl}amine.
*Point de fusion* (M .K .) : 169-171°C.
*Pouvoir rotatoire:* solvant: MeOH, C=0,009g/cm³, T=20°C, L=589nm, α_{D} =-0,9.
*IR* (cm⁻¹) : 2719 (NH₂⁺), 1630 (C=O), 1276-1202-1177 (C-O-C), 862-832-781 (CH-Ar).

### EXEMPLE 43 : 7-({[3-(7,8-Diméthoxy-1,2,4,5-tétrahydro-3H-3-benzazépin-3-yl)-3-oxopropyl](méthyl)amino}méthyl)bicyclo[4.2.0]octa-1,3,5-trién-3-ol

Dans 145 mL d'acétonitrile à température ambiante, on mélange 3,2 g (7,8 mmol.) du produit de l'exemple 35, 6,4 mL (78 mmol./ 10 éq.) d'une solution aqueuse à 37% de formaldéhyde et 0,98 g (15,6 mmol.) de cyanoborohydrure de sodium. On maintient 1h40 sous agitation à température ambiante, puis évapore à sec. On reprend le résidu par 250 mL d'eau et extrait la phase aqueuse 2 fois par 150 mL de dichlorométhane. Les phases organiques jointes sont lavées à l'eau et séchées sur sulfate de magnésium. Après filtration, concentration et chromatographie sur 200 g de silice (éluant : dichlorométhane / éthanol / ammoniaque : 95/5/0,5), on obtient le produit attendu sous forme base.
Par salification par l'éther chlorhydrique de la base en solution dans l'acétonitrile, on obtient le chlorhydrate du produit attendu.
*Point de fusion* (M .K .) : 167-170°C
*IR* (cm⁻¹): 3600 à 2000 (OH/NH⁺), 1616 (C=O).

### EXEMPLE 44 : 7-{[[3-(7,8-Diméthoxy-1,2,4,5-tétrahydro-3H-3-benzazépin-3-yl)-3-oxopropyl](méthyl)amino]méthyl}bicyclo[4.2.0]octa-1,3,5-trién-3-yl méthylcarbamate, chlorhydrate

A 0,7 g (1,65 mmol.) du composé de l'exemple 43 dans 30 mL de toluène, à température ambiante, en autoclave, on ajoute 0,2 mL (3,3 mmol./ 2 éq.) d'isocyanate de méthyle et on chauffe à 90°C pendant 48h. Après concentration, le résidu est chromatographié sur 130 g de silice (éluant : dichlorométhane / éthanol / ammoniaque 95/5/0,5) pour donner le produit attendu sous forme base. Elle est solubilisée dans 6 mL d'éthanol et traitée par 0,5 mL d'éthanol chlorhydrique 3N pour donner le produit attendu.
*Point de fusion* (M .K .) : 185-188°C.

### EXEMPLE 45 : 8-({[3-(7,8-Diméthoxy-1,2,4,5-tétrahydro-3H-3-benzazépin-3-yl)-3-oxopropyl](méthyl)amino}méthyl)bicyclo[4.2.0]octa-1,3,5-trién-3-ol

Obtenu de la même façon que le produit de l'exemple 43, mais en remplaçant le produit de l'exemple 35 par celui de l'exemple 36.
*Point de fusion* (M .K .): 201-205°C
*IR* (cm⁻¹) : 3071 (OH), 2672 (NH⁺), 1634 (C=O), 1250-1228-1196 (C-O-C), 885-749-741 (CH-Ar).

### EXEMPLE 46 : 8-{[[3-(7,8-Diméthoxy-1,2,4,5-tétrahydro-3H-3-benzazépin-3-yl)-3-oxopropyl](méthyl)amino]méthyl}bicyclo[4.2.0]octa-1,3,5-trién-3-yl méthylcarbamate, chlorhydrate

Obtenu de la même façon que le composé de l'exemple 44 mais en utilisant le composé de l'exemple 45 à la place du composé de l'exemple 43.
*Point de fusion* (M .K .) : 107-110°C.

### EXEMPLE 47 : 7-{[[3-(7,8-Diméthoxy-1,2,4,5-tétrahydro-3H-3-benzazépin-3-yl)-3-oxopropyl](méthyl)amino]méthyl}bicyclo[4.2.0]octa-1,3,5-trién-3-yl diméthylcarbamate, chlorhydrate

A température ambiante, à 0,7 g (1,65 mmol.) du composé de l'exemple 43 dans 5 ml de pyridine, on ajoute d'un trait 0,3 mL (2,06 mmol./1,25 éq.) de triéthylamine, puis goutte à goutte 0 ,2 mL (2,06 mmol./ 1,25 éq.) de diméthylcarbamoyl chlorure. On maintient sous agitation à température ambiante pendant la nuit, puis concentre. Le résidu est chromatographié sur 130 g de silice (éluant : dichlorométhane / éthanol / ammoniaque : 95/5/0,5) pour donner le produit attendu sous forme base. Il est solubilisé dans 7 mL d'éthanol et traité par 0,5 mL d'éthanol chlorhydrique 3N pour donner le chlorhydrate.
*Point de fusion* (M .K .) : 176-180°C.

### EXEMPLE 48 : Enantiomère (-) du 7-{[[3-(7,8-diméthoxy-1,2,4,5-tétrahydro-3H-3-benzazépin-3-yl)-3-oxopropyl](méthyl)amino]méthyl}bieyclo[4.2.0]octa-1,3,5-trién-3-yl diméthylcarbamate, chlorhydrate

1,9 g du composé de l'exemple 47 est séparé par chromatographie sur phase chirale, phase mobile: MeOH/DEA : 1000/1. Le premier produit élué correspond au produit attendu qui est salifié par de l'éthanol chlorhydrique 3N.
*Point de fusion* (M.K.) : 132-137°C.
*Pouvoir rotatoire:* solvant: MeOH, C=0.012g/cm³, T=20°C, L=589nm, α_{D} =-6.72

### EXEMPLE 49 : Enantiomère (+) du 7-{[[3-(7,8-diméthoxy-1,2,4,S-tétrahydro-3H-3-benzazépin-3-yl)-3-oxopropyl](méthyl)amino]méthyl}bicyclo[4.2.0]oeta-1,3,5-trién-3-yl diméthylcarbamate, chlorhydrate

Le second produit élué à l'exemple 48 correspond au produit attendu, qui est salifié dans les mêmes conditions que le produit de l'exemple 48.
*Point de fusion* (M .K.): 164-167°C.
*Pouvoir rotatoire*: solvant: MeOH, C=0.01g/cm³, T=20°C, L=589nm, α_{D} =+7.15.

### EXEMPLE 50 : 8-{[[3-(7,8-Diméthoxy-1,2,4,5-tétrahydro-3H-3-benzazépin-3-yl)-3-oxopropyl](méthyl)amino]méthyl}bicyclo[4.2.0]octa-1,3,5-trién-3-yl diméthylcarbamate, chlorhydrate

Obtenu de la même façon que le composé de l'exemple 47 mais en utilisant le composé de l'exemple 45 à la place du composé de l'exemple 43.
*Point de fusion* (M .K.): 108-112°C.

### EXEMPLE 51 : 7-{[[3-(7,8-Diméthoxy-1,2,4,5-tétrahydro-3H-3-benzazépin-3-yl)-3-oxopropyl](méthyl)amino]méthyl}bicyclo[4.2.0]octa-1,3,5-trién-3-yl éthylcarbonate, chlorhydrate

A 0,95 g (2,24 mmol.) du composé de l'exemple 43 dans 20 mL de dichlorométhane, à température ambiante, on ajoute d'un trait 0,33 mL (2,35 mmol./ 1,05 éq.) de triéthylamine. On amène vers 0°C et coule goutte à goutte 0,23 mL (2,46 mmol./ 1,1 éq.) de chloroformiate d'éthyle. On agite à 0°C pendant 1h30, puis laisse remonter à température ambiante. La phase organique est lavée 2 fois par 20 mL d'eau et séchée sur sulfate de magnésium. Après concentration, le résidu (0,96 g) est chromatographié sur 130 g de silice (éluant : dichlorométhane / éthanol / ammoniaque 95/5/0,5) pour donner le produit attendu sous forme base, qui solubilisée dans 9 mL d'éthanol et traitée par 0,65 mL d'éthanol chlorhydrique 3N donne le chlorhydrate.
*Point de fusion* (M .K.): 149-152°C.

### EXEMPLE 52 : 8-{[[3-(7,8-Diméthoxy-1,2,4,5-tétrahydro-3H-3-benzazépin-3-yl)-3-oxopropyl]amino]méthyl}bicyclo[4.2.0]octa-1,3,5-trién-3-yl méthylcarbamate, chlorhydrate

### Stade 1 : [3-(7,8-Diméthoxy-1,2,4,5-tétrahydro-3H-3-benzazépin-3-yl-3-oxopropyl]-[(4-hydroxybicyclo[4.2.0]oeta-1,3,5-trién-7-yl)méthyl] carbamate de tert-butyle

Dans 20 mL de dichlorométhane, à température ambiante, on mélange 1,93 g (4,7 mmol.) du produit de l'exemple 36 et 1,03 g (4,7 mmol./ 1 éq.) de dicarbonate de di-tert-butyle. Après 2h sous agitation à température ambiante, on ajoute 50 mL de dichlorométhane, lave 2 fois par 40 mL d'eau et sèche sur sulfate de magnésium. Après concentration, on recueille le produit attendu.
*Point de fusion*(M .K.): 150-154°C.

### Stade 2 : 8-({(tert-Butoxycarbonyl [3-(7,8-diméthoxy 1,2,4,5-tétrahydro-3H-3-benzazépin-3-yl) 3-oxopropyl]amino}méthyl) bicyclo[4.2.0]octa-1,3,5-trién-3-yl) méthylcarbamate

On procède comme à l'exemple 44 mais en remplaçant le produit de l'exemple 43 par le produit du stade 1 ci-dessus.

### Stade 3 : 8-{[[3-(7,8-Diméthoxy-1,2,4,5-tétrahydro-3H-3-benzazépin-3-yl)-3-oxopropyl]-amino]méthyl}bicyclo[4.2.0]octa-1,3,5-trién-3-yl méthylcarbamate, chlorhydrate

Sur 0,48 g (0,84 mmol.) du composé du stade 2 ci-dessus en solution dans 8 mL d'acétate d'éthyle, on ajoute 2,8 mL (8,4 mmol./ 10 éq.) d'acétate d'éthyle chlorhydrique 3N. On maintient sous agitation à température ambiante pendant la nuit, puis filtre le solide formé, le rince à l'acétate d'éthyle et le sèche sous vide pour obtenir le produit attendu.
*Point de fusion* (M .K.): 150-154°C.

### EXEMPLE 53 : 8-{[[3-(7,8-Diméthoxy-1,2,4,5-tétrahydro-3H-3-benzazépin-3-yl)-3-oxopropyl]amino]méthyl}bicyclo[4.2.0]octa-1,3,5-trién-3-yl diméthylcarbamate, chlorhydrate

### Stade 1 : 8-({(tert-Butoxycarbonyl [3-(7,8-diméthoxy 1,2,4,5-tétrahydro-3H-3-benzazépin-3-yl) 3-oxopropyl]amino}méthyl) bicyclo[4.2.0]octa-1,3,5-trién-3-yl) diméthylcarbamate

On procède selon le mode opératoire du composé de l'exemple 47 mais en remplaçant le produit de l'exemple 43 par le produit du stade 1 de l'exemple 52.

### Stade 2 : 8-{[[3-(7,8-Diméthoxy-1,2,4,5-tétrahydro-3H-3-benzazépin-3-yl)-3-oxopropyl]-amino]méthyl}bicyclo[4.2.0]oeta-1,3,5-trién-3-yl diméthylcarbamate, chlorhydrate

Sur 0,93 g ( 1,6 mmol.) du composé du stade 1 en solution dans 9 mL d'éthanol, on ajoute 2,4 mL (7,2 mmol./ 4,5 éq.) d'éthanol chlorhydrique 3N. On maintient sous agitation à température ambiante pendant la nuit, puis filtre le solide formé et le sèche sous vide pour obtenir le produit attendu.
*Point de fusion* (M .K.): 220-222°C.

### EXEMPLE 54 : 7-{[[3-(7,8-Diméthoxy-1,2,4,5-tétrahydro-3H-3-benzazépin-3-yl)-3-oxopropyl]amino]méthyl}bicyclo[4.2.0]octa-1,3,5-trién-3-yl diméthylcarbamate, chlorhydrate

### Stade 1 : 7-({(tert-Butoxycarbonyl [3-(7,8-diméthoxy 1,2,4,5-tétrahydro-3H-3-benzazépin-3-yl) 3-oxopropyl]amino}méthyl) bicyclo[4.2.0]octa-1,3,5-trién-3-yl) diméthylcarbamate

On solubilise 3,6 g (8,8 mmoles) de *tert*-butyl [3-(7,8-diméthoxy 1,2,4,5-tetrahydro-3*H*-3-benzazépin-3-yl)-3-oxopropyl] [(3-hydroxybicyclo[4.2.0]octa-1,3,5-trién-7-yl)méthyl] carbamate dans 50 mL de dichorométhane. On y ajoute à 0°C 2,1 g (1,1 équivalent) de chlorure de N,N-diméthylcarbamoyle. On agite 3 jours à 25°C. On verse sur l'eau, on extrait au dichlorométhane, décante, sèche sur MgSO₄, filtre et évapore à sec. On obtient un résidu qui est purifié par flash-chromatographie sur 70 g de silice (éluant : CH₂Cl₂ / Éthanol : 98/2 à 90/10) pour conduire au produit attendu.

### Stade 2 : 7-{[[3-(7,8-Diméthoxy-1,2,4,5-tétrahydro-3H-3-benzazépin-3-yl)-3-oxopropyl]amino]méthyl}bicyclo[4.2.0]octa-1,3,5-trién-3-yl diméthylcarbamate, chlorhydrate

On solubilise 0,8 g (1,37 mmoles) du produit obtenu au stade 1 dans 4 mL d'éthanol et on ajoute 2 mL d'une solution 3,2 N d'Éthanol-HCl. On chauffe à 60°C pendant 2 heures. On laisse refroidir à température ambiante et on agite 2 heures. On filtre un solide qui est recristallisé de 6 mL d'éthanol à reflux. On laisse cristalliser à 25°C, filtre et sèche. On obtient le produit attendu sous la forme d'un solide blanc.
*Point de fusion* (M.K.) = 217-230°C

### EXEMPLE 55 : 7-{[[3-(7,8-Diméthoxy-1,2,4,5-tétrahydro-3H-3-benzazépin-3-yl)-3-oxopropyl]amino]méthyl}bieyclo[4.2.0]octa-1,3,5-trién-3-yl 4-morpholinecarboxylate, chlorhydrate

On procède selon le mode opératoire du composé de l'exemple 54, mais en remplaçant au stade 1 le chlorure de N,N-diméthylcarbamoyle par le chlorure de morpholinyl 4-carbonyle.
*Point de fusion* (M.K.) = 217-220°C

### EXEMPLE 56 : 7-{[[3-(7,8-Diméthoxy-1,2,4,5-tétrahydro-3H-3-benzazépin-3-yl)-3-oxopropyl]amino]méthyl}bicyclo[4.2.0]octa-1,3,5-trién-3-yl diéthylcarbamate, chlorhydrate

On procède selon le mode opératoire du composé de l'exemple 54, mais en remplaçant au stade 1 le chlorure de N,N-diméthylcarbamoyle par le chlorure de N,N-diéthylcarbamoyle.
*Point de fusion* (M.K.) = 197-200°C

### EXEMPLE 57 : 7-{[[3-(7,8-Diméthoxy-1,2,4,5-tétrahydro-3H-3-benzazépin-3-yl)-3-oxopropyl]amino]méthyl}bicyclo[4.2.0]octa-1,3,5-trién-3-yl 1-pyrrolidinecarboxylate, chlorhydrate

On procède selon le mode opératoire du composé de l'exemple 54, mais en remplaçant au stade 1 le chlorure de N,N-diméthylcarbamoyle par le chlorure de pyrrolidinyl 1-carbonyle.
*Point de fusion* (M.K.) = 220-222°C

### EXEMPLE 58 : 8-{[[3-(7,8-Diméthoxy-1,2,4,5-tétrahydro-3H-3-benzazépin-3-yl)-3-oxopropyl]amino]m\éthyl}bicyclo[4.2.0]octa-1,3,5-trién-3-yl éthylcarbonate, chlorhydrate

### Stade 1 : 8-({(tert-Butoxycarbonyl [3-(7,8-diméthoxy 1,2,4,5-tétrahydro-3H-3-benzazépin-3-yl) 3-oxopropyl]amino}méthyl) bicyclo[4.2.0]octa-1,3,5-trién-3-yl) éthylcarbonate

Obtenu comme pour la synthèse de l'exemple 51, mais en remplaçant le produit de l'exemple 43 par le [3-(7,8-diméthoxy-1,2,4,5-tétrahydro-3*H*-3-benzazépin-3-yl)-3-oxopropyl] [(4-hydroxybicyclo[4.2.0]octa-1,3,5-trién-7-yl)méthyl] carbamate de *tert-*butyle obtenu au stade 1 de l'exemple 52.

### Stade 2 : 8-{[[3-(7,8-Diméthoxy-1,2,4,5-tétrahydro-3H-3-benzazépin-3-yl)-3-oxopropyl]amino]méthyl}bicyclo[4.2.0]octa-1,3,5-trién-3-yl éthylcarbonate, chlorhydrate

Sur 2,55 g( 4,4 mmol.) du composé du stade 1 en solution dans 50 mL d'acétate d'éthyle, on ajoute 14,5 mL (44 mmol./ 10 éq.) d'acétate d'éthyle chlorhydrique 3N. On maintient sous agitation à température ambiante pendant 4 jours, puis filtre le solide formé, le rince à l'acétate d'éthyle et le sèche sous vide pour obtenir 2,15 g d'un résidu qui est purifié sur silice (éluant : dichlorométhane / éthanol / ammoniaque : 95/5/0,5). Après salification par de l'éthanol chlorhydrique dans l'éthanol, on obtient le produit attendu.
*Point de fusion* (M .K.): 164-167°C.

### EXEMPLE 59 : 7-{[[3-(7,8-Diméthoxy-1,2,4,5-tétrahydro-3H-3-benzazépin-3-yl)-3-oxopropyl]amino]méthyl}bicyclo[4.2.0]octa-1,3,5-trién-3-yl éthylcarbonate, chlorhydrate

Obtenu comme le produit de l'exemple 58 mais en remplaçant au stade 1 le produit du stade 1 de l'exemple 52 par le [3-(7,8-diméthoxy 1,2,4,5-tétrahydro-3*H*-3-benzazépin-3-yl) 3-oxopropyl] [3-hydroxybicyclo[4.2.0] octa 1,3,5-trién-7-yl) méthyl] carbamate de *tert-*butyle.
*Point de fusion* (M .K.): 208-212°C.

### EXEMPLE 60 : 8-{[[3-(7,8-Diméthoxy-1,2,4,5-tétrahydro-3H-3-benzazépin-3-yl)-3-oxopropyl]amino]méthyl}bicyclo[4.2.0]octa-1,3,5-trién-3-yl acétate, chlorhydrate

### Stade 1 : 8-({(tert-Butoxycarbonyl [3-(7,8-diméthoxy 1,2,4,5-tétrahydro-3H-3-benzazépin-3-yl) 3-oxopropyl]amino}méthyl) bicyclo[4.2.0]oeta-1,3,5-trién-3-yl) acétate

A 3,25 g (6,4 mmol.) de [3-(7,8-diméthoxy 1,2,4,5-tétrahydro-3*H*-3-benzazepin-3-yl) 3-oxopropyl] [4-hydroxybicyclo[4.2.0] octa 1,3,5-trién-7-yl) méthyl] carbamate de *tert-*butyle obtenu au stade 1 de l'exemple 52 dans 20 mL de dichlorométhane, à température ambiante, on ajoute d'un trait 0,33 mL (6,7 mmol./ 1,05 éq.) de triéthylamine. On amène vers 0°C et coule goutte à goutte 0,45 mL (6,4 mmol./ 1 éq.) de chlorure d'acétyle. On agite à 0°C pendant 1h, puis laisse remonter à température ambiante. La phase organique est lavée 2 fois par 20 mL d'eau et séchée sur sulfate de magnésium. Après concentration, le résidu est chromatographié sur 200 g de silice (éluant : dichlorométhane / acétate d'éthyle : 97/3) pour donner le produit recherché.

### Stade 2 : 8-{[[3-(7,8-Diméthoxy-1,2,4,5-tétrahydro-3H-3-benzazépin-3-yl)-3-oxopropyl]amino]méthyl}bicyclo[4.2.0]octa-1,3,5-trién-3-yl acétate, chlorhydrate

Sur 2,2 g (4,0 mmol.) du composé obtenu au stade 1 en solution dans 45 mL d'acétate d'éthyle, on ajoute 13,5 mL (40 mmol./ 10 éq.) d'acétate d'éthyle chlorhydrique 3N. On maintient sous agitation à température ambiante pendant 4 jours, verse le milieu réactionnel sur 300 mL d'éther, puis filtre le solide formé, le rince à l'éther et le sèche sous vide pour obtenir un résidu qui est chromatographié sur silice (éluant : dichlorométhane / éthanol / ammoniaque : 95/5/0,5). Après salification par l'éthanol chlorhydrique dans l'éthanol, on obtient le produit attendu sous forme de chlorhydrate.
*Point de fusion* (M .K.): 176-179°C.

### EXEMPLE 61 : 7-({[3-(7,8-Diméthoxy-1,2,4,5-tétrahydro-3H-3-benzazépin-3-yl)-3-oxopropyl]amino}méthyl)bicyclo[4.2.0]octa-1,3,5-trién-3-yl hexahydrocyclopenta[c]pyrrole-2(1H)-carboxylate (composé cis)

### Stade 1 : 7-({(tert-Butoxycarbonyl) [3-(7,8-diméthoxy 1,2,4,5-tétrahydro-3H-3-benzazépin-3-yl) 3-oxopropyl]amino}méthyl)bicyclo[4.2.0] octa 1,3,5-trién-3-yl hexahydrocyclopenta [c] pyrrole-2(1H)carboxylate

Dans 210 mL de THF on dissout 1,5 g (2,94 mmol) de [3-(7,8-diméthoxy 1,2,4,5-tétrahydro-3H-3-benzazépin-3-yl)-3-oxopropyl] [3-hydroxybicyclo[4.2.0] octa 1,3,5-trién-7-yl) méthyl] carbamate de tert-butyle, 4,1 mL de triéthylamine et 2,37 g (11,74 mmol) de 4-nitrophényl chloridocarbonate. On agite 1 h à température ambiante puis additionne 0.65 g (5.87 mmol) d' octahydropenta[c]pyrrole et 2,9 mL de triéthylamine. Après 2 h à température ambiante on rajoute à nouveau 0,65g d'octahydropenta[c]pyrrole et abandonne à température ambiante pendant 1 h. On dilue le milieu réactionnel par 700 mL d'acétate d'éthyle puis lave à l'eau, décante, séche sur MgSO4 et évapore. Le résidu est chromatographié sur silice (éluant: CH₂Cl₂/Éthanol/NH₄OH : 99/1/0.1) pour conduire au produit attendu.

### Stade 2 : 7-({[3-(7,8-Diméthoxy-1,2,4,5-tétrahydro-3H-3-benzazépin-3-yl)-3-oxopropyl]-amino}méthyl)bicyclo[4.2.0]octa-1,3,5-trién-3-yl hexahydrocyclopenta[c]pyrrole-2(1H)-carboxylate (composé cis)

On dissout le produit obtenu au stade 1 dans 10 volumes d'éthanol et 10 volumes d'une solution 3N d'HCl éthanolique. On laisse sous agitation 24h à température ambiante et filtre le précipité obtenu qui correspond au produit attendu.
*Point de fusion* (M .K.): 214-217°C.

### EXEMPLE 62 : 7-({[3-(7,8-Diméthoxy-1,2,4,5-tétrahydro-3H-3-benzazépin-3-yl)-3-oxopropyl]amino}méthyl)bicyclo[4.2.0]octa-1,3,5-trién-3-yl 4,4-difluoro-1-pipéridinecarboxylate

Obtenu comme à l'exemple 61 mais en utilisant au stade 1 la 4,4-difluoropipéridine à la place de l'octahydropenta[c]pyrrole.
*Point de fusion* (M .K.): 212-216°C.

### EXEMPLE 63 : 3-(7,8-Diméthoxy-1,2,4,5-tétrahydro-3H-3-benzazépin-3-yl)- N-[(3-isopropoxybicyclo[4.2.0]octa-1,3,5-trién-7-yl)méthyl] 3-oxopropan-1-amine, hémifumarate

### Stade 1 : [3-(7,8-Diméthoxy-1,2,4,5-tétrahydro-3H-3-benzazépin-3-yl)-3-oxopropyl]-[(3-hydroxybicyclo[4.2.0]oeta-1,3,5-trién-7-yl)méthyl) carbamate de tert-butyle

On solubilise 3,6 g (8,8 mmoles) du produit de l'exemple 35 dans 60 mL de dichlorométhane. Puis on ajoute 2,1g (1,1 équivalent) de dicarbonate de ditertbutyle et on agite à température ambiante pendant 3 heures (jusqu'à la fin du dégagement gazeux). On évapore le dichlorométhane , on sèche sous le vide de la pompe à palettes et on obtient le produit attendu.

### Stade 2 : [3-(7,8-Diméthoxy-1,2,4,5-tétrahydro-3H-3-benzazépin-3-yl)-3-oxopropyl]-[(3-isopropoxybicyclo[4.2.0]octa-1,3,5-trién-7-yl)méthyl] carbamate de tert-butyle

On solubilise 1,4 g (2,7 mmoles) du produit du stade 1 dans 15 mL de DMF. Puis on ajoute 740 mg (2 équivalents) de carbonate de potassium, 160 mg (0,18 équivalent) de carbonate de césium et 0,54 mL (2 équivalents) d'iodure d'isopropyle. On chauffe ensuite à 40°C pendant 24 heures. On évapore la DMF à l'évaporateur rotatif. Puis le résidu obtenu est dilué à l'eau et extrait à CH₂Cl₂. La phase organique est ensuite séchée sur MgSO₄, filtrée et évaporée à sec. On obtient une huile qui est purifiée par flash-chromatographie sur 100g de silice (éluant : CH₂Cl₂ / Acétate d 'éthyle : 80/20) pour donner le produit attendu sous forme de meringue collante.

### Stade 3 : 3-(7,8-Diméthoxy-1,2,4,5-tétrahydro-3H-3-benzazépin-3-yl)- N-[(3-isopropoxybicyclo[4.2.0]octa-1,3,5-trién-7-yl)méthyl] 3-oxopropan-1-amine, hémifumarate

On solubilise 1,1 g (1,99 mmoles) du produit du stade 2 dans 10 mL d' éthanol. On ajoute 14 mL (18 équivalents) d'une solution d'éthanol chlorhydrique (2,6 N). On agite une nuit à température ambiante . Un produit cristallise. Il est filtré et séché pour donner 800 mg de produit. Ce solide est solubilisé dans l'eau, on ajoute NaOH (1 N) jusqu'à pH= 9, on extrait à CH₂Cl₂, sèche sur MgSO₄, filtre et évapore à sec pour obtenir 650mg d'une huile. Celle-ci est ensuite purifiée par flash-chromatographie sur 100g de silice (éluant : CH₂Cl₂ / Éthanol / NH₄OH : 95/5/0,5). On obtient le produit attendu sous forme base que l'on met en solution dans 10 mL d' éthanol. On ajoute 8,1 mL (1 équivalent) d'une solution à 2 % d'acide fumarique dans l'éthanol (M = 0,172). On agite 30 minutes à température ambiante, puis on évapore à sec. On cristallise le résidu obtenu de l'acétonitrile à 25°C. On filtre les cristaux, on obtient 314 mg d'un solide qui est recristallisé à reflux de l'acétonitrile. On filtre à chaud, puis on laisse cristalliser une heure à température ambiante. On filtre et sèche le solide pour obtenir le produit attendu sous la forme de cristaux blancs.
*Point de fusion* (M.K.) = 208-211°C

### EXEMPLE 64 : 2-{[((7S)-3,4-Diméthoxybicyclo[4.2.0]octa-1,3,5-trién-7-yl)-méthyl]-(méthyl)-amino}éthyl 7,8-diméthoxy-1,2,4,5-tétrahydro-3H-3-benzazépine-3-carboxylate, chlorhydrate

### Stade 1 : Chlorure de 7,8-diméthoxy 1,2,4,5-tetrahydro-3H-3-benzazépine 3-carbonyle

A 1,2 mL (10 mmol./l éq.) de diphosgène dans 20 mL de dichlorométhane, vers 0°C, on ajoute goutte à goutte une solution de 3,55 g (20 mmol.) de 7,8-diméthoxy 2,3,4,5-tetrahydro-1*H*-3-benzazépine dans 22 mL de dichlorométhane, puis 4,2 mL (30 mmol./1,5 éq.) de triéthylamine. On maintient la nuit sous agitation à température ambiante, puis concentre. Le résidu est repris au dichlorométhane, lavé à l'eau et séché sur sulfate de magnésium pour donner, après concentration, le produit recherché.

### Stade 2 : 2-{[(3,4-Diméthoxybicyclo[4.2.0]oeta-1,3,5-trién-7-yl)-méthyl]-(méthyl)-amino}éthyl 7,8-diméthoxy-1,2,4,5-tétrahydro-3H-3-benzazépine-3-carboxylate

A température ambiante, on dégraisse au pentane 0,23 g (5,7 mmol./1,25 éq.) d'hydrure de sodium (à 60%), puis ajoute 7 ml de THF. On ajoute ensuite goutte à goutte une solution de 1,14 g (4,5 mmol./ 1 éq.) du composé de la préparation 25 dans 7 mL de THF, maintient sous agitation pendant 1h, ajoute par fractions 1,02 g (3,8 mmol./ 0,85 éq.) du composé du stade 1 ci-dessus, et maintient sous agitation à température ambiante pendant la nuit. Le milieu réactionnel est versé sur 50 mL d'une solution aqueuse normale d'acide chlorhydrique. On extrait au dichlorométhane, puis les phases organiques jointes sont lavées par une solution normale de soude et séchées sur sulfate de magnésium. Après filtration et concentration, le résidu est chromatographié sur 150 g de silice (éluant : dichlorométhane / éthanol / ammoniaque : 98/2/0,2). La base ainsi obtenue est solubilisée dans l'isopropanol et salifiée par de l'éthanol chlorhydrique pour conduire au produit attendu.
*Point de fusion* (M.K.) : 147-150°C
*Pouvoir rotatoire:* solvant: MeOH, C=0.01g/cm³, T=20°C, L=589nm, α_{D} =+7.1.

### EXEMPLE 65 : 2-{[((7R)-3,4-Diméthoxybicyclo[4.2.0]octa-1,3,5-trién-7-yl)-méthyl]-(méthyl)-amino}éthyl 7,8-diméthoxy-1,2,4,5-tétrahydro-3H-3-benzazépine-3-carboxylate, chlorhydrate

Obtenu de la même manière qu'à l'exemple 64 mais en utilisant au stade 2 le produit de la préparation 26 au lieu du produit de la préparation 25.
*Point de fusion* (M.K.) : 144-147°C
*Pouvoir rotatoire:* solvant: MeOH, C=0.01g/cm³, T=20°C, L=589nm, α_{D} =-6.94.

### EXEMPLE 66 : N-{2-[{[(7S)-3,4-Diméthoxybicyclo[4.2.0]octa-1,3,5-trién-7-yl]méthyl}(méthyl)amino]éthyl}-7,8-diméthoxy-1,2,4,5-tetrahydro-3H-3-benzazépine-3-carboxamide, méthanesulfonate

A température ambiante, à 1,06 g (4,5 mmol.) du composé de la préparation 27 en solution dans 40 mL de dichlorométhane, on ajoute d'un trait 2,34 ml (13,4 mmol./ 3 éq.) de diisopropyléthylamine, puis par fractions 1,02 g (3,8 mmol./ 0,85 éq.) du composé du stade 1 de l'exemple 64, et maintient sous agitation à température ambiante pendant la nuit. Le milieu réactionnel est lavé à l'eau et concentré. Le résidu est chromatographié sur 140 g de silice (éluant : dichlorométhane / éthanol / ammoniaque : 95/5/0,5). Le produit purifié est salifié par l'acide méthanesulfonique dans l'acétate d'éthyle et, après évaporation, concrétisé de l'éther pour donner le produit attendu.
*Point de fusion* (M.K.) : 65-85°C

### EXEMPLE 67 : N-{2-[{[(7R)-3,4-Diméthoxybicyclo[4.2.0]octa-1,3,5-trién-7-yl]méthyl}(méthyl)amino]éthyl}-7,8-diméthoxy-1,2,4,5-tetrahydro-3H-3-benzazépine-3-carboxamide, méthanesulfonate

Obtenu de la même manière qu'à l'exemple 66 mais en utilisant le produit de la préparation 28 au lieu du produit de la préparation 27.
*Point de fusion* (M.K.) : 65-85°C

### EXEMPLE 68 : N-[(2,3-Diméthoxybicyclo[4.2.0]octa-1,3,5-trién-7-yl)méthyl] 3-(7,8-diméthoxy-1,2,4,5-tétrahydro-3H-3-benzazépin-3-yl)-N-méthyl-3-oxopropan-1-amine, chlorhydrate

Obtenu de la même façon que le produit de l'exemple 43, mais en remplaçant le produit de l'exemple 35 par celui de l'exemple 8.
*Point de fusion* (M .K.): 185-186°C.

### EXEMPLE 69 : N-[(2,3,4-Triméthoxybicyclo[4.2.0]octa-1,3,5-trién-7-yl)méthyl] 3-(7,8-diméthoxy-1,2,4,5-tétrahydro-3H-3-benzazépin-3-yl)- N-méthyl-3-oxopropan-1-amine, fumarate

Obtenu de la même façon que le produit de l'exemple 43, mais en remplaçant le produit de l'exemple 35 par celui de l'exemple 9. La salification est effectuée par l'acide fumarique.
*Point de fusion* (M .K.): 158-160°C

### EXEMPLE 70 : 3-(7,8-Diméthoxy-1,2,4,5-tétrahydro-3H-3-benzazépin-3-yl)-N-[(5-méthoxybicyclo[4.2.0]octa-1,3,5-trién-7-yl)méthyl-N-méthyl-3-oxopropan-1-amine, chlorhydrate

Obtenu de la même façon que le produit de l'exemple 43, mais en remplaçant le produit de l'exemple 35 par celui de l'exemple 10.
*Point de fusion* (M .K.): 130-132°C

### EXEMPLE 71 : 7-{[[3-(7,8-Diméthoxy-1,2,4,5-tétrahydro-3H-3-benzazépin-3-yl)-3-oxopropyl](méthyl)amino]méthyl}bicyclo[4.2.0]octa-1,3,5-trién-3-yl diéthylcarbamate, chlorhydrate

Obtenu de la même façon que le produit de l'exemple 43, mais en remplaçant le produit de l'exemple 35 par celui de l'exemple 56.
*Point de fusion* (M .K.): 175-178°C

### EXEMPLE 72 : 7-{[[3-(7,8-Diméthoxy-1,2,4,5-tétrahydro-3H-3-benzazépin-3-yl)-3-oxopropyl](méthyl)amino]méthyl}bicyclo[4.2.0]octa-1,3,5-trién-3-yl 4-morpholinecarboxylate, chlorhydrate

Obtenu de la même façon que le produit de l'exemple 43, mais en remplaçant le produit de l'exemple 35 par celui de l'exemple 55.
*Point de fusion* (M .K.): 196-198°C

### EXEMPLE 73 : 7-{[[3-(7,8-Diméthoxy-1,2,4,5-tétrahydro-3H-3-benzazépin-3-yl)-3-oxopropyl](méthyl)amino]méthyl}bicyclo[4.2.0]octa-1,3,5-trién-3-yl 1-pyrrolidinecarboxylate, chlorhydrate

Obtenu de la même façon que le produit de l'exemple 43, mais en remplaçant le produit de l'exemple 35 par celui de l'exemple 57.
*Point de fusion* (M .K.): 174-177°C

### EXEMPLE 74 : 3-(7,8-Diméthoxy-1,2,4,5-tétrahydro-3H-3-benzazépin-3-yl)-N-{[3-méthoxy-4-méthyl-bicyclo[4.2.0]octa-1,3,5-trién-7-yl]méthyl}-N méthyl-3-oxopropan-1-amine, chlorhydrate

Obtenu de la même façon que le produit de l'exemple 43, mais en remplaçant le produit de l'exemple 35 par celui de l'exemple 39.
*Point de fusion* (M .K.): 172-175°C

### EXEMPLE 75 : 8-{[[3-(7,8-Diméthoxy-1,2,4,5-tétrahydro-3H-3-benzazépin-3-yl)-3-oxopropyl](méthyl)amino]méthyl}bicyclo[4.2.0]octa-1,3,5-trién-3-yl éthylcarbonate, chlorhydrate

Obtenu de la même façon que le produit de l'exemple 43, mais en remplaçant le produit de l'exemple 35 par celui de l'exemple 58.
*Point de fusion* (M .K.): 156-159°C

### EXEMPLE 76 : 3-(7,8-Diméthoxy-1,2,4,5-tétrahydro-3H-3-benzazépin-3-yl)-N-{[4-méthoxy-3-méthyl-bicyclo[4.2.0]octa-1,3,5-trién-7-yl]méthyl}- N-méthyl-3-oxopropan-1-amine, chlorhydrate

Obtenu de la même façon que le produit de l'exemple 43, mais en remplaçant le produit de l'exemple 35 par celui de l'exemple 38.
*Point de fusion* (M .K.): 191-193°C

### EXEMPLE 77 : N-[(Bicydo[4.2.0]octa-1,3,5-trién-7-yl)méthyl] 3-(7,8-diméthoxy-1,2,4,5-tétrahydro-3H-3-benzazépin-3-yl)- N-méthyl-3-oxopropan-1-amine, chlorhydrate

Obtenu de la même façon que le produit de l'exemple 43, mais en remplaçant le produit de l'exemple 35 par celui de l'exemple 37.
*Point de fusion* (M .K.): 110-113°C

### EXEMPLE 78 : 7-({[3-(7,8-Diméthoxy-1,2,4,5-tétrahydro-3H-3-benzazépin-3-yl)-3-oxopropyl](méthyl)amino}méthyl)bicyclo[4.2.0]octa-1,3,5-trién-3-yl hexahydrocyclopenta[c]pyrrole-2(1H)-carboxylate (composé cis)

Obtenu de la même façon que le produit de l'exemple 43, mais en remplaçant le produit de l'exemple 35 par celui de l'exemple 61.
*Point de fusion* (M .K.): 110-113°C

### EXEMPLE 79 : 2-({[(7S)-3,4-Diméthoxybicyclo [4.2.0] octa-1,3,5-trien-7-yl]méthyl} [3-(7,8-diméthoxy-1,2,4,5-tétrahydro-3H-3-benzazépin-3-yl)-3-oxopropyl]-amino)-éthanol, chlorhydrate

On mélange 1 g (2,2 mmol) de (7*S*) *N-*[(3,4-diméthoxybicyclo [4.2.0] octa-1,3,5-trien-7-yl)méthyl] 3-(7,8-diméthoxy 1,2,4,5-tétrahydro-3*H-*3-benzazépin-3-yl) 3-oxopropane 1-amine (obtenue par retour base du composé de l'Exemple 4), 0,4 g (3,3 mmol) de 1,4-dioxane 2,5-diol, 0,7 g (3,3 mmol) de triacétoxyborohydrure de sodium et 8 mL de chlorure de méthylène. Après 45 minutes à température ambiante, le milieu réactionnel est basifié par 10 mL de soude 1N et agité une demi-heure. Les phases sont séparées, la phase aqueuse est extraite deux fois à l'acétate d'éthyle, les phases organiques sont réunies, lavées à l'eau puis avec une solution saturée de NaCl et séchées sur MgSO4. Après évaporation du solvant, on isole un résidu qui est chromatographié sur silice (éluant CH₂Cl₂/EtOH/NH₄OH 95/5/0,5) pour conduire au dérivé attendu qui est transformé en son chlorhydrate par une solution 2N d'éther chlorhydrique.
*Point de fusion* (M .K .) :75-79°C.

### EXEMPLE 80 : 3-(7,8-Diméthoxy-1,2,4,5-tétrahydro-3H-3-benzazépin-3-yl)-N-{[3-(3-méthoxypropoxybicyclo [4.2.0] octa-1,3,5-trien-7-yl]méthyl}-3-oxopropan 1-amine, chlorhydrate.

### Stade A. : [3-(7,8-Diméthoxy 1,2,4,5-tétrahydro-3H-3-benzazépin-3-yl) 3-oxopropyl] [3-hydroxy bicyclo [4.2.0] octa-1,3,5-trien 7-yl)méthyl] carbamate de tert-butyle

Sur 10g (24,4 mmol.) de 7-({[3-(7,8-diméthoxy 1,2,4,5-tétrahydro-3*H*-3-benzazépin-3-yl) 3-oxopropyl] amino}méthyl) bicyclo [4.2.0] octa-1,3,5-trién 3-ol, obtenu par retour base du composé de l'exemple 35, en suspension dans 120 mL de dichlorométhane, à température ambiante, on ajoute 20 mL d'éthanol pour solubiliser en partie le produit, puis 6,4 g (29,3 mmol) de dicarbonate de di-tert-butyle. On agite à température ambiante pendant 2h, puis ajoute 250 mL de dichlorométhane et lave 2 fois par 200 mL d'eau. Après séchage de la phase organique sur sulfate de magnésium, puis concentration, on obtient un résidu qui est purifié par filtration sur silice (éluant : dichlorométhane/méthanol 97/3).

### Stade B : 3-(7,8-Diméthoxy-1,2,4,5-tétrahydro-3H-3-benzazépin-3-yl)-N-{[3-(3-méthoxypropoxybicyclo [4.2.0] octa-1,3,5-trien-7-yl]méthyl}-3-oxopropan 1-amine, chlorhydrate

On mélange à température ambiante 1,33g (2,6 mmol.) du dérivé obtenu au stade A, 43 mg (0,26 mmol) d'iodure de potassium, 1,27g (3,9 mmol.) de carbonate de césium et 0,40 mL (3,7 mmol) de chlorure de méthoxypropyle dans 2,6 mL de DMF, puis on porte à 60°C pendant 24h. On verse alors sur 50 mL d'eau glacée et extrait 2 fois par 50 mL d'acétate d'éthyle. Les phases organiques jointes sont lavées par 30 mL d'eau, séchées sur sulfate de magnésium et concentrées. Le résidu est chromatographié sur 100g d'alumine (éluant : cyclohexane / acétate d'éthyle 75/25) pour donner un dérivé qui est solubilisé dans 8 mL d'éthanol anhydre et traité à température ambiante par 1,55 mL (5,0 mmol) d'éthanol chlorhydrique 3,7N. On maintient ensuite sous agitation à température ambiante pendant 45h. Le solide qui se forme est filtré pour donner le composé recherché sous forme de chlorhydrate.
*Point de fusion* (M .K.): 155-159°C.

### EXEMPLE 81 : 3-(7,8-Diméthoxy-1,2,4,5-tétrahydro-3H-3-benzazépin-3-yl)-N-{[3-(3-méthoxypropoxybicyclo [4.2.0] octa-1,3,5-trien-7-yl]méthyl} N-méthyl-3-oxopropan 1-amine, chlorhydrate.

On mélange à température ambiante 1,06 g (2,5 mmol) de 7-({[3-(7,8-diméthoxy 1,2,4,5-tetrahydro-3*H*-3-benzazépin-3-yl) 3-oxopropyl] (méthyl) amino}méthyl) bicyclo [4.2.0] octa-1,3,5-trien 3-ol (obtenu par retour base du composé de l'exemple 43), 42 mg (0,25 mmol) d'iodure de potassium, 0,98 g (3,0 mmol) de carbonate de césium et 0,35 mL (3,25 mmol) de chlorure de méthoxypropyle dans 2,5 mL de DMF, puis on porte à 50°C pendant 3 jours. On verse alors dans 50 mL d'eau glacée et extrait 2 fois par 50 mL d'acétate d'éthyle. Les phases organiques jointes sont lavées par 30 mL d'eau, séchées sur sulfate de magnésium et concentrées. Le résidu (1,21 g) est chromatographié sur silice (éluant : dichlorométhane / méthanol 95/5) pour donner 1,07 g du composé recherché sous forme de base. Il est solubilisé dans 5 mL d'acétonitrile et traité à température ambiante par 1,2 mL d'éther chlorhydrique 2N. On maintient ensuite sous agitation à température ambiante pendant 2h. Le solide qui se forme est filtré pour donner 0,91 g du composé recherché sous forme de chlorhydrate.
*Point de fusion* (M.K.): 142-147°C.

### EXEMPLE 82 : 3-(7,8-Diméthoxy-1,2,4,5-tétrahydro-3H-3-benzazépin-3-yl)-N-{[3-(3-méthoxyéthoxybicyclo [4.2.0] octa-1,3,5-trien-7-yl]méthyl}-3-oxopropan 1-amine, chlorhydrate.

On procède comme à l'exemple à l'exemple 80 mais en utilisant au stade B le chlorure de méthoxyéthyle au lieu de chlorure de méthoxypropyle.
*Point de fusion* (M .K.): 159-170°C.

### EXEMPLE 83 : 3-(7,8-Diméthoxy-1,2,4,5-tétrahydro-3H-3-benzazépin-3-yl)-N-{[3-(3-méthoxyéthoxybicyclo [4.2.0] octa-1,3,5-trien-7-yl]méthyl} N-méthyl-3-oxopropan 1-amine, chlorhydrate.

On procède comme à l'exemple 81 mais en utilisant le chlorure de méthoxyéthyle au lieu de chlorure de méthoxypropyle.
*Point de fusion* (M .K.): 129-131°C.

### EXEMPLE 84 : 2-{[7-({[3-(7,8-Diméthoxy-1,2,4,5-tétrahydro-3H-3-benzazépin-3-yl)-3-oxopropyl]amino}méthyl) bicyclo [4.2.0] octa-1,3,5-trien-3-yl]oxy} N-méthylacétamide, chlorhydrate.

### Stade A : Ester éthylique de l'acide {[7-({(tert-butoxycarbonyl) [3-(7,8-diméthoxy 1,2,4,5-tétrahydro-3H-3-benzazépin-3-yl) 3-oxopropyl] amino} méthyl) bicyclo [4.2.0] octa-1,3,5-trien 3-yl)oxy] carboxylique

On mélange 6,8 g (13,3 mmol) du composé obtenu au stade A de l'exemple 80, 2,3g (16,6mmol) de carbonate de potassium et 2,21 mL (20,0mmol) de bromoacétate d'éthyle dans 13,3 mL de DMF. On maintient le milieu réactionnel sous agitation à température ambiante pendant 25h, puis verse sur 250 mL d'eau glacée et extrait deux fois par 150 mL d'acétate d'éthyle. Les phases organiques jointes sont lavées par 100 mL d'eau, séchées sur sulfate de magnésium et concentrées pour conduire au composé recherché.

### Stade B : [3-(7,8-Diméthoxy-1,2,4,5-tétrahydro-3H-3-benzazépin-3-yl)-3-oxopropyl]-({3-[2-(méthylamino) 2-oxoéthoxy] bicyclo [4.2.0] octa-1,3,5-trien 7-yl}méthyl) carbamate de tert-butyle

On mélange à température ambiante 2,6 g (4,4 mmol.) du composé obtenu au stade A ci-dessus en solution dans 20 mL d'éthanol, avec 20 mL d'une solution aqueuse à 40% de monométhylamine. On maintient sous agitation à température ambiante pendant 3 jours, puis concentre. Le résidu est repris successivement au toluène à l'éthanol, puis au toluène et concentré pour conduire au produit recherché.

### Stade C : 2-{[7-({[3-(7,8-Diméthoxy-1,2,4,5-tétrahydro-3H-3-benzazépin-3-yl)-3-oxopropyl]-amino}méthyl) bicyclo [4.2.0] octa-1,3,5-trien-3-yl]oxy} N-méthylacétamide, chlorhydrate

A température ambiante, on mélange 2,63g (4,5 mmol.) du composé obtenu au stade B ci-dessus dans 120 mL d'éthanol avec 25 mL d'éthanol chlorhydrique 3,7 N. Après 1 journée sous agitation à température ambiante, on filtre le solide formé, le rince à l'éthanol et à l'éther et le sèche sous vide pour obtenir le composé désiré sous forme de chlorhydrate.
*Point de fusion* (M .K.): 145-146°C.

### EXEMPLE 85 : 2-{[7-({[3-(7,8-Diméthoxy-1,2,4,5-tétrahydro-3H-3-benzazépin-3-yl)-3-oxopropyl](méthyl)amino}méthyl) bicyclo [4.2.0] octa-1,3,5-trien-3-yl]oxy} N-méthylacétamide, chlorhydrate.

A température ambiante, à 0,83g (1,72 mmol) du composé obtenu au stade C de l'exemple 84 en solution dans 32 mL d'acétonitrile, on ajoute d'un trait 1,4 mL (17,2 mmol) d'une solution aqueuse à 37% de formaldéhyde, puis 0,22g (3,44 mmol) de cyanoborohydrure de sodium. Après agitation pendant 45 mn à température ambiante, on concentre et le résidu est repris par 30 mL d'eau, la phase aqueuse est extraite 2 fois par 30 mL de dichlorométhane, les phases organiques sont jointes, lavées par 30 mL d'eau et séchées sur sulfate de magnésium. Après concentration, le résidu est chromatographié sur silice (éluant : dichlorométhane / éthanol / ammoniaque 95/5/0,5) pour donner le composé recherché sous forme de base.
Cette base est solubilisée dans 4 mL d'éthanol et traitée par 0,3 mL d'éthanol chlorhydrique 3,7N. Après 1h sous agitation à température ambiante, on filtre le solide formé, le rince à l'éthanol et le sèche sous vide pour obtenir le composé recherché sous forme de chlorhydrate.
*Point de fusion* (M .K.): 147-153°C

### ETUDE PHARMACOLOGIQUE

### EXEMPLE 86 : Effet des composés sur la fréquence spontanée d'oreillette droite de rat

Les rats WISTAR mâles de 325 - 375 g sont anesthésiés par injection ip de pentobarbital sodique (30mg/kg). Le coeur est rapidement prélévé et placé dans une solution physiologique à 4°C contenant (en mM): NaCl 120.3, KCl 4.8, CaCl₂ 2.5, KH₂PO₄ 1.0, MgSO₄ 1.3, NaHCO₃ 24.2, glucose 11.1, Ca-EDTA 0.016, oxygénée (carbogène 95%O₂ + 5% CO₂), pH 7.4. L'oreillette droite (OD) qui bat spontanément est isolée, placée dans une cuve thermostatée à 35°C contenant 20 ml de solution physiologique et reliée à un capteur de tension isométrique (model IT-25, EMKA Technologies, Paris, France). La tension initiale imposée est de 0.4 g. La fréquence des battements spontanés est mesurée par le logiciel IOX (EMKA Technologies, Paris, France). Les préparations dont la fréquence spontanée n'est pas comprise entre 200 et 300 battements par minute sont exclues.

Les produits sont quotidiennement dissous à 10⁻² M. Les dilutions suivantes sont conservées dans la glace pendant la durée de l'expérience.

Après 30 minutes de stabilisation le produit à tester est additionné au milieu de façon cumulative toutes les 15 minutes (4 concentrations). Les diminutions de fréquence sont exprimées en pourcentage par rapport à la fréquence initiale. La concentration réduisant de 30 % la fréquence initiale (IC30) est calculée et exprimée en molaire (µM).

### Résultats

| **Composé** | **IC₃₀ (µM)** |
|---|---|
| Exemple 3a | 1,3 |
| Exemple 4 | 1,4 |
| Exemple 7 | 1,7 |
| Exemple 14 | 1 |
| Exemple 22 | 0,8 |
| Exemple 23 | 1,4 |
| Exemple 27 | 1,2 |
| Exemple 28 | 1,2 |
| Exemple 29 | 0,4 |
| Exemple 34 | 1,3 |
| Exemple 44 | 0,8 |
| Exemple 46 | 0,5 |
| Exemple 47 | 0,6 |
| Exemple 48 | 0,5 |
| Exemple 50 | 0,5 |
| Exemple 53 | 1,8 |
| Exemple 54 | 0,8 |
| Exemple 71 | 1,8 |
| Exemple 73 | 2,4 |
| Exemple 74 | 1,0 |
| Exemple 76 | 1,6 |
| Exemple 78 | 2,0 |

Le tableau ci-dessus démontre que les composés de l'invention réduisent de façon directe l'activité pacemaker cardiaque.

### EXEMPLE 87 : Composition pharmaceutique

| Formule de préparation pour 1000 comprimés dosés à 10 mg : | |
|---|---|
| Composé de l'un des exemples 1 à 85 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) : 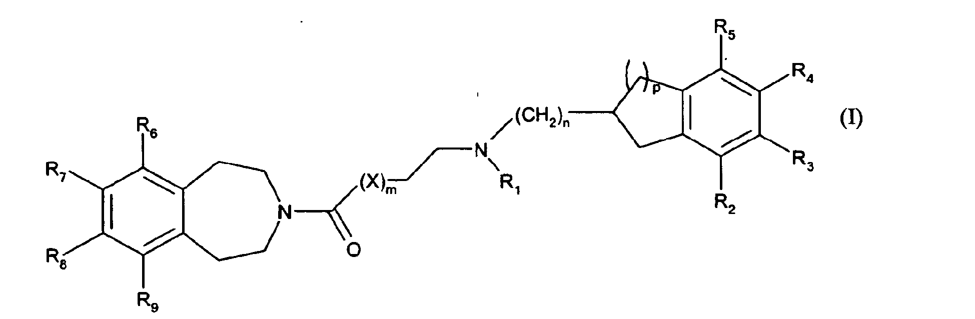 dans laquelle :
- R₁ représente un atome d'hydrogène ou un groupement choisi parmi cycloalkyle C₃-C₇, benzyle et alkyle C₁-C₆ linéaire ou ramifié, le groupement alkyle étant saturé
ou insaturé et éventuellement substitué par un groupement hydroxy, cycloalkyle C₃-C₇ ou par un ou plusieurs atomes d'halogène,
- R₂, R₃, R₄ et R₅, identiques ou différents, représentent chacun un atome d'hydrogène ou un groupement hydroxy, méthyle, -OSO₂R₁₀, -OCOR₁₀, alkoxy C₁-C₆ linéaire ou ramifié saturé ou insaturé éventuellement substitué par un groupement méthoxy ou -(CO)-N R₁₂R_{'12}, ou bien R₂ et R₃, ou R₃ et R₄, ou R₄ et R₅ forment ensemble un groupement -O-(CH₂)_{q}-O-, -O-CH=CH-O- ou -O-CH=CH-,
- R₆, R₇, R₈ et R₉, identiques ou différents, représentent chacun un atome d'hydrogène ou un groupement alkoxy C₁-C₆ linéaire ou ramifié saturé ou insaturé, ou bien R₆ et R₇, ou R₇ et R₈, ou R₈ et R₉ forment ensemble un groupement -0-(CH₂)_{q}-O-,
- R₁₀ représente un groupement choisi parmi alkoxy C₁-C₆ linéaire ou ramifié, NR₁₁R'₁₁, et alkyle C₁-C₆ linéaire ou ramifié éventuellement substitué par un ou plusieurs atomes d'halogène,
- R₁₁ et R'₁₁, identiques ou différents, représentent chacun un atome d'hydrogène ou un groupement alkyle C₁-C₆ linéaire ou ramifié, ou bien R₁₁ et R'₁₁ forment ensemble, avec l'atome d'azote qui les porte, un hétérocycle azoté mono-ou bicyclique comportant de 5 à 8 chaînons et comportant éventuellement un autre hétéroatome choisi parmi O et N, ledit hétérocycle étant éventuellement substitué par un ou plusieurs atomes d'halogène,
- R₁₂ et R'₁₂, identiques ou différents, représentent chacun un atome d'hydrogène ou un groupement alkyle C₁-C₆ linéaire ou ramifié,
- X représente O, NH ou CH₂,
- m et p, identiques ou différents, représentent chacun 0 ou 1,
- n et q, identiques ou différents, représentent chacun 1 ou 2,
ses isomères optiques lorsqu'ils existent, ainsi que ses sels d'addition à un acide pharmaceutiquement acceptable.

2. Composé de formule (I) selon la revendication 1, pour lequel R₁ représente un atome d'hydrogène ou un groupement alkyle C₁-C₆ linéaire ou ramifié, ses isomères optiques lorsqu'ils existent, ainsi que ses sels d'addition à un acide pharmaceutiquement acceptable.

3. Composé de formule (I) selon la revendication 1, pour lequel R₁ représente un groupement cycloalkyle C₃-C₇ ou cycloalkylalkyle dans lequel la partie cycloalkyle comprend de 3 à 7 atomes de carbone et la partie alkyle comprend de 1 à 6 atomes de carbone et est linéaire ou ramifié, ses isomères optiques lorsqu'ils existent, ainsi que ses sels d'addition à un acide pharmaceutiquement acceptable.

4. Composé de formule (I) selon l'une quelconque des revendications 1 à 3, pour lequel R₂, R₃, R₄ et R₅, identiques ou différents, représentent chacun un atome d'hydrogène ou un groupement alkoxy C₁-C₆ linéaire ou ramifié saturé ou insaturé ou -OCOR₁₀ dans lequel R₁₀ représente un groupement NR₁₁R'₁₁ tel que défini dans la revendication 1, ses isomères optiques lorsqu'ils existent, ainsi que ses sels d'addition à un acide pharmaceutiquement acceptable.

5. Composé de formule (I) selon l'une quelconque des revendications 1 à 4, pour lequel R₆, R₇, R₈ et R₉, identiques ou différents, représentent chacun un atome d'hydrogène ou un groupement alkoxy C₁-C₆ linéaire ou ramifié saturé ou insaturé, ses isomères optiques lorsqu'ils existent, ainsi que ses sels d'addition à un acide pharmaceutiquement acceptable.

6. Composé de formule (I) selon l'une quelconque des revendications 1 à 5, pour lequel m représente 0, ses isomères optiques lorsqu'ils existent, ainsi que ses sels d'addition à un acide pharmaceutiquement acceptable.

7. Composé de formule (I) selon l'une quelconque des revendications 1 à 5, pour lequel m représente 1 et X représente CH₂, ses isomères optiques lorsqu'ils existent, ainsi que ses sels d'addition à un acide pharmaceutiquement acceptable.

8. Composé de formule (I) selon l'une quelconque des revendications 1 à 7, pour lequel p représente 0, ses isomères optiques, ainsi que ses sels d'addition à un acide pharmaceutiquement acceptable.

9. Composé de formule (I) selon l'une quelconque des revendications 1 à 7, pour lequel p représente 1, ses isomères optiques lorsqu'ils existent, ainsi que ses sels d'addition à un acide pharmaceutiquement acceptable.

10. Composé de formule (I) selon la revendication 1, pour lequel R₁ représente un atome d'hydrogène ou un groupement alkyle C₁-C₆ linéaire ou ramifié, R₂, R₃, R₄ et R₅, identiques ou différents, représentent chacun un atome d'hydrogène ou un groupement alkoxy C₁-C₆ linéaire ou ramifié saturé ou insaturé, R₆, R₇, R₈ et R₉, identiques ou différents, représentent chacun un atome d'hydrogène ou un groupement alkoxy C₁-C₆ linéaire ou ramifié saturé ou insaturé, m représente 0, n représente 1 et p représente 0, ses isomères optiques, ainsi que ses sels d'addition à un acide pharmaceutiquement acceptable.

11. Composé de formule (I) selon la revendication 1, choisi parmi :
- la N-{[3,4-diméthoxybicyclo[4.2.0]octa-1,3,5-trién-7-yl]méthyl} 3-(7,8-diméthoxy-1,2,4,5-tétrahydro-3H-3-benzazépin-3-yl) N-méthyl-3-oxopropan-1-amine, ses isomères optiques, ainsi que ses sels d'addition à un acide pharmaceutiquement acceptable;
- la N-{[3,4-diméthoxybicyclo[4.2.0]octa-1,3,5-trién-7-yl]méthyl}-3-(7,8-diméthoxy-1,2,4,5-tétrahydro-3H-3-benzazépin-3-yl)-3-oxopropan-1-amine, ses isomères optiques, ainsi que ses sels d'addition à un acide pharmaceutiquement acceptable;
- la N-[2-(5,6-diméthoxy-2,3-dihydro-1*H*-indén-2-yl)méthyl]-3-(7,8-diméthoxy-1,2,4,5-tétrahydro-3*H-*3-benzazépin-3-yl)-3-oxopropan-1-amine, ainsi que ses sels d'addition à un acide pharmaceutiquement acceptable;
- la N-{[3,4-diméthoxybicyclo[4.2.0]octa-1,3,5-trién-7-yl]méthyl} 3-(7,8-diméthoxy-1,2,4,5-tétrahydro-3H-3-benzazépin-3-yl) N-méthyl-4-oxobutan-1-amine, ses isomères optiques, ainsi que ses sels d'addition à un acide pharmaceutiquement acceptable;
- la N-{[3,4-diméthoxybicyclo[4.2.0]octa-1,3,5-trién-7-yl]méthyl} 3-(7,8-diméthoxy-1,2,4,5-tétrahydro-3H-3-benzazépin-3-yl)-4-oxobutan-1-amine, ses isomères optiques, ainsi que ses sels d'addition à un acide pharmaceutiquement acceptable; et
- le 7-{[[3-(7,8-diméthoxy-1,2,4,5-tétrahydro-3*H*-3-benzazépin-3-yl)-3-oxopropyl]-(méthyl)amino]méthyl}bicyclo[4.2.0]octa-1,3,5-trién-3-yl diméthylcarbamate, ses isomères optiques, ainsi que ses sels d'addition à un acide pharmaceutiquement acceptable.

12. Procédé de synthèse des composés de formule (I) selon la revendication 1 à partir d'un composé de formule (II) : 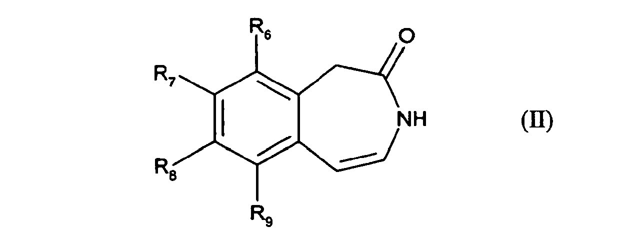 dans laquelle R₆, R₇, R₈ et R₉ sont tels que définis dans la revendication 1, que l'on soumet à une réaction d'hydrogénation, pour conduire au composé de formule (III) : 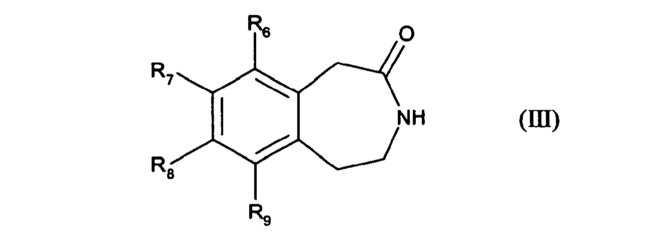 dans laquelle R₆, R₇, R₈ et R₉ sont tels que définis dans la revendication 1,
que l'on réduit, pour conduire au composé de formule (IV) : 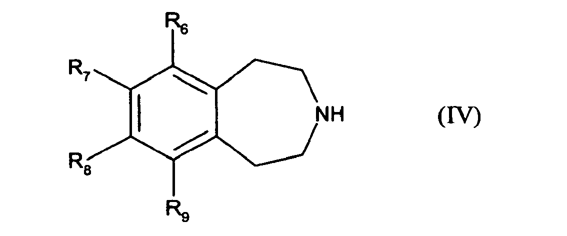 dans laquelle R₆, R₇, R₈ et R₉ sont tels que définis dans la revendication 1,
que l'on met en réaction :
• soit, lorsque l'on souhaite accéder aux composés de formule (I) pour lesquels m représente zéro, avec le chlorure d'acryloyle, pour conduire au composé de formule (V): 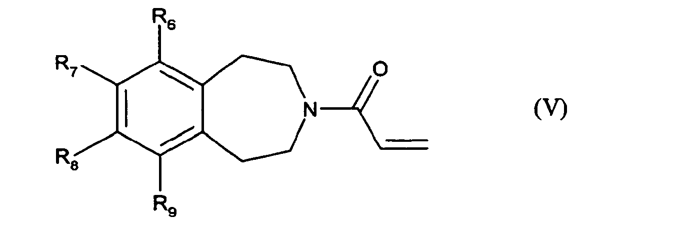 dans laquelle R₆, R₇, R₈ et R₉ sont tels que définis dans la revendication 1,
que l'on soumet à une réaction de couplage avec un composé de formule (VI) : 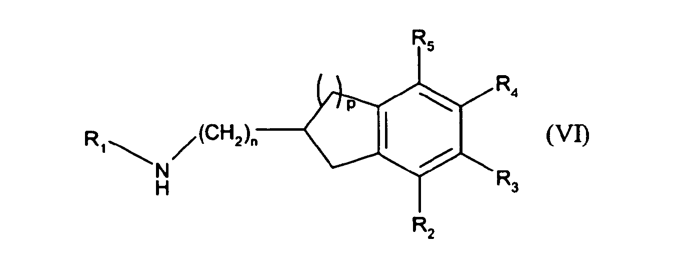 dans laquelle n, p, R₁, R₂, R₃, R₄ et R₅ sont tels que définis dans la revendication 1,
pour conduire aux composés de formule (Ia), cas particulier des composés de formule (I) pour lesquels m représente zéro : 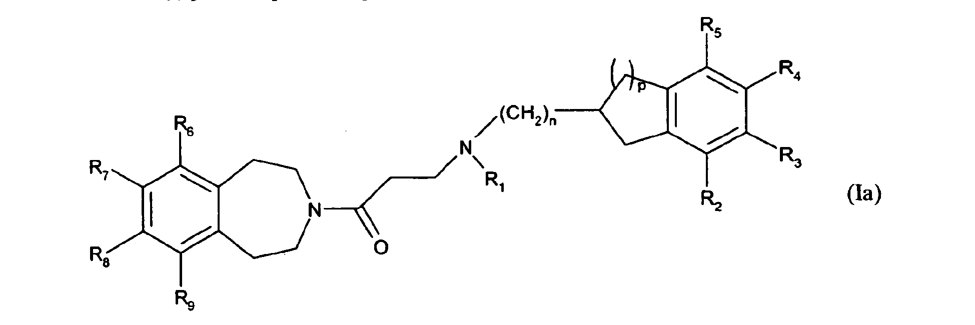 dans laquelle n, p, R₁, R₂, R₃, R₄, R₅, R₆, R₇, R₈ et R₉ sont tels que définis dans la revendication 1,
• soit, lorsque l'on souhaite accéder aux composés de formule (I) pour lesquels (X)ₘ représente O ou NH, avec du diphosgène, pour conduire au composé de formule (VII) : 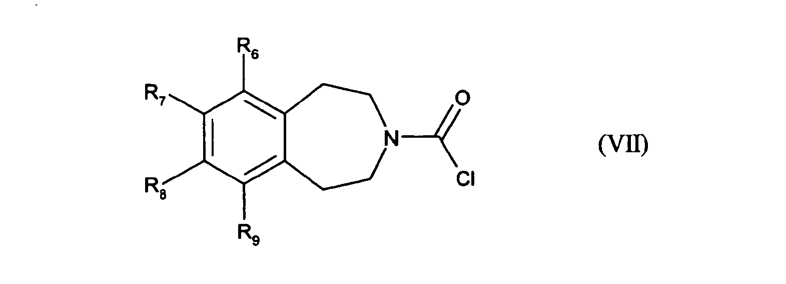 dans laquelle R₆, R₇, R₈ et R₉ sont tels que définis dans la revendication 1,
que l'on met en réaction avec un composé de formule (VIII) : 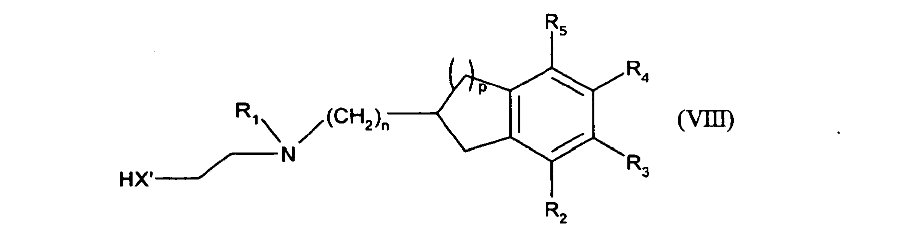 dans laquelle n, p, R₁, R₂, R₃, R₄ et R₅ sont tels que définis dans la revendication 1, et X' représente O ou NH,
pour conduire aux composés de formule (Ib), cas particulier des composés de formule (I) pour lesquels m représente 1 et X représente O ou NH : 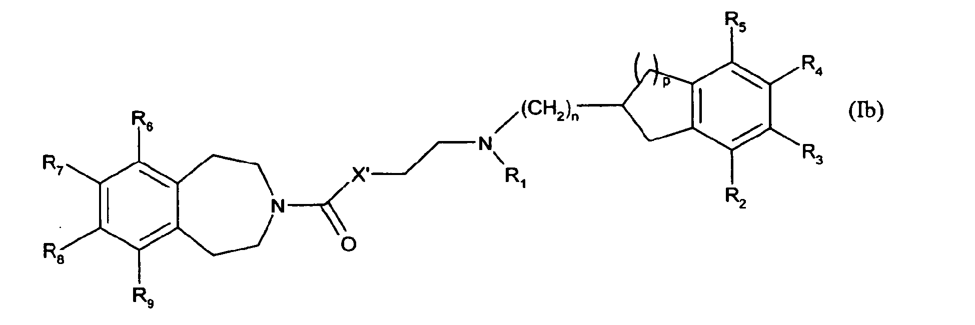 dans laquelle n, p, R₁, R₂, R₃, R₄, R₅, R₆, R₇, R₈ et R₉ sont tels que définis dans la revendication 1, et X' représente O ou NH,
• soit, lorsque l'on souhaite accéder aux composés de formule (I) pour lesquels (X)ₘ représente CH₂, avec de la gamma-butyrolactone, pour conduire au composé de formule (IX) : 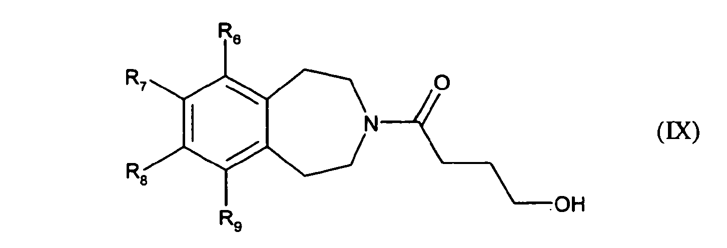
dans laquelle R₆, R₇, R₈ et R₉ sont tels que définis dans la revendication 1,
qui est oxydé en composé de formule (X) : 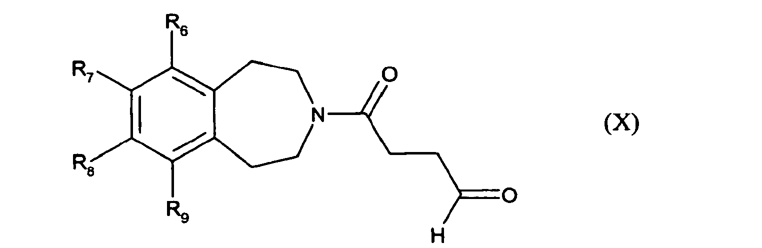 dans laquelle R₆, R₇, R₈ et R₉ sont tels que définis dans la revendication 1,
qui est mis en réaction avec un composé de formule (VI), pour conduire aux composés de formule (Ic), cas particulier des composés de formule (I) pour lesquels m représente 1 et X représente CH₂ : 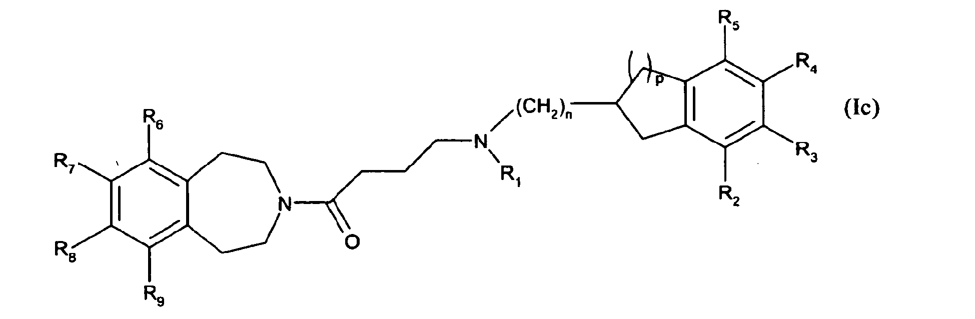 dans laquelle n, p, R₁, R₂, R₃, R₄, R₅, R₆, R₇, R₈ et R₉ sont tels que définis dans la revendication 1.

13. Composition pharmaceutique contenant comme principe actif un composé de formule (I) selon l'une quelconque des revendications 1 à 11, en combinaison avec un ou plusieurs excipients ou véhicules inertes, non toxiques, pharmaceutiquement acceptables.

14. Composition pharmaceutique selon la revendication 13 utile pour le traitement ou la prévention des pathologies dans lesquelles une accélération de la fréquence cardiaque joue un rôle déclenchant ou aggravant.

15. Composition pharmaceutique selon la revendication 14 utile pour le traitement ou la prévention des cardiopathies ischémiques, des insuffisances cardiaques tant systolique que diastolique, dans leurs formes chronique ou aiguë, des troubles du rythme ventriculaires ou supra ventriculaires, ou des pathologies constituant un facteur de risque vasculaire.

16. Composition pharmaceutique selon la revendication 15 utile pour le traitement ou la prévention de l'angor stable, de l'angor instable, des syndromes de menace, de l'infarctus du myocarde, ou du post infarctus.

17. Composition pharmaceutique selon la revendication 15 utile pour le traitement ou la prévention de l'hypertension artérielle, du diabète ou de l'hypercholestérolémie.

18. Composition pharmaceutique selon la revendication 13 utile pour le traitement curatif ou préventif des douleurs, de la vessie hyperactive ou de la sensation de sécheresse oculaire.

## Claims

1. Compound of formula (I) : 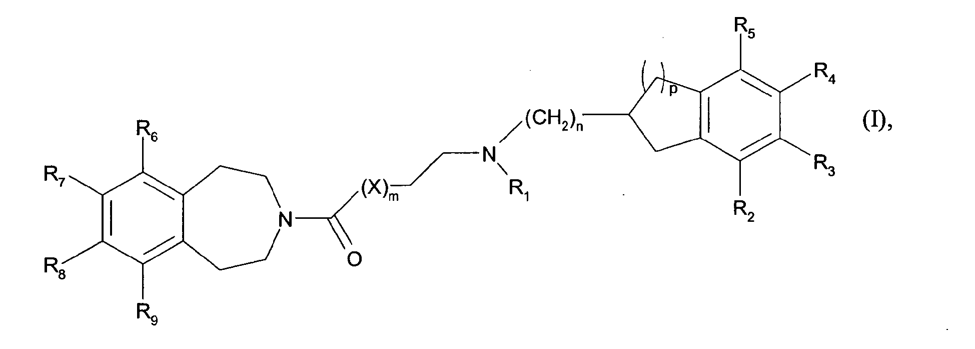 wherein:
- R₁ represents a hydrogen atom or a group selected from C₃-C₇cycloalkyl, benzyl and linear or branched C₁-C₆alkyl, the alkyl group being saturated or unsaturated and optionally substituted by a hydroxy or C₃-C₇cycloalkyl group or by one or more halogen atoms,
- R₂, R₃, R₄ and R₅, which may be the same or different, each represent a hydrogen atom or a hydroxy group; methyl group; -OSO₂R₁₀ group; -OCOR₁₀ group; or linear or branched, saturated or unsaturated C₁-C₆alkoxy group optionally substituted by a methoxy or -(CO)-NR₁₂R'₁₂ group, or R₂ and R₃, or R₃ and R₄, or R₄ and R₅ together form a group -O-(CH₂)_{q}-O-, -O-CH=CH-O- or -O-CH=CH-,
- R₆, R₇, R₈ and R₉, which may be the same or different, each represent a hydrogen atom or a linear or branched, saturated or unsaturated C₁-C₆alkoxy group, or R₆ and R₇, or R₇ and R₈, or R₈ and R₉ together form a group -O-(CH₂)_{q}-O-,
- R₁₀ represents a group selected from linear or branched C₁-C₆alkoxy, NR₁₁R'₁₁ and linear or branched C₁-C₆alkyl which is optionally substituted by one or more halogen atoms,
- R₁₁ and R'₁₁, which may be the same or different, each represent a hydrogen atom or a linear or branched C₁-C₆alkyl group, or R₁₁ and R'₁₁ together with the nitrogen atom carrying them form a monocyclic or bicyclic, 5- to 8-membered, nitrogen-containing heterocycle optionally containing another hetero atom selected from O and N, said heterocycle being optionally substituted by one or more halogen atoms,
- R₁₂ and R'₁₂, which may be the same or different, each represent a hydrogen atom or a linear or branched C₁-C₆alkyl group,
- X represents O, NH or CH₂,
- m and p, which may be the same or different, each represent 0 or 1,
- n and q, which may be the same or different, each represent 1 or 2,
its optical isomers when they exist, and also addition salts thereof with a pharmaceutically acceptable acid.

2. Compound of formula (I) according to claim 1, wherein R₁ represents a hydrogen atom or a linear or branched C₁-C₆alkyl group, its optical isomers when they exist, and also addition salts thereof with a pharmaceutically acceptable acid.

3. Compound of formula (I) according to claim 1, therein R₁ represents a C₃-C₇cycloalkyl group or a cycloalkylalkyl group wherein the cycloalkyl moiety contains from 3 to 7 carbon atoms and the alkyl moiety contains from 1 to 6 carbon atoms and is linear or branched, its optical isomers when they exist, and also addition salts thereof with a pharmaceutically acceptable acid.

4. Compound of formula (I) according to any one of claims 1 to 3, wherein R₂, R₃, R₄ and R₅, which may be the same or different, each represent a hydrogen atom or a linear or branched, saturated or unsaturated C₁-C₆alkoxy group or -OCOR₁₀ wherein R₁₀ represents a group NR₁₁R'₁₁ as defined in claim 1, its optical isomers when they exist, and also addition salts thereof with a pharmaceutically acceptable acid.

5. Compound of formula (I) according to any one of claims 1 to 4, wherein R₆, R₇, R₈ and R₉, which may be the same or different, each represent a hydrogen atom or a linear or branched, saturated or unsaturated C₁-C₆alkoxy group, its optical isomers when they exist, and also addition salts thereof with a pharmaceutically acceptable acid.

6. Compound of formula (I) according to any one of claims 1 to 5, wherein m represents 0, its optical isomers when they exist, and also addition salts thereof with a pharmaceutically acceptable acid.

7. Compound of formula (I) according to any one of claims 1 to 5, wherein m represents 1 and X represents CH₂, its optical isomers when they exist, and also addition salts thereof with a pharmaceutically acceptable acid.

8. Compound of formula (I) according to any one of claims 1 to 7, wherein p represents 0, its optical isomers, and also addition salts thereof with a pharmaceutically acceptable acid.

9. Compound of formula (I) according to any one of claims 1 to 7, wherein p represents 1, its optical isomers when they exist, and also addition salts thereof with a pharmaceutically acceptable acid.

10. Compound of formula (I) according to claim 1, wherein R₁ represents a hydrogen atom or a linear or branched C₁-C₆alkyl group, R₂, R₃, R₄ and R₅, which may be the same or different, each represent a hydrogen atom or a linear or branched, saturated or unsaturated C₁-C₆alkoxy group, R₆, R₇, R₈ and R₉, which may be the same or different, each represent a hydrogen atom or a linear or branched, saturated or unsaturated C₁-C₆alkoxy group, m represents 0, n represents 1 and p represents 0, its optical isomers, and also addition salts thereof with a pharmaceutically acceptable acid.

11. Compound of formula (I) according to claim 1 selected from:
- N-{[3,4-dimethoxybicyclo[4.2.0]octa-1,3,5-trien-7-yl]methyl}-3-(7,8-dimethoxy-1,2,4,5-tetrahydro-3H-3-benzazepin-3-yl)-N-methyl-3-oxopropan-1-amine, its optical isomers, and also addition salts thereof with a pharmaceutically acceptable acid;
- N-{[3,4-dimethoxybicyclo[4.2.0]octa-1,3,5-trien-7-yl]methyl]-3-(7,8-dimethoxy-1,2,4,5-tetrahydro-3H-3-benzazepin-3-yl)-3-oxopropan-1-amine, its optical isomers, and also addition salts thereof with a pharmaceutically acceptable acid;
- N-[2-(5,6-dimethoxy-2,3-dihydro-1*H*-inden-2-yl)methyl]-3-(7,8-dimethoxy-1,2,4,5-tetrahydro-3*H*-3-benzazepin-3-yl)-3-oxopropan-1-amine, and also addition salts thereof with a pharmaceutically acceptable acid;
- N-{[3,4-dimethoxybicyclo[4.2.0]octa-1,3,5-trien-7-yl]methyl}-3-(7,8-dimethoxy-1,2,4,5-tetrahydro-3H-3-benzazepin-3-yl)-N-methyl-4-oxobutan-1-amine, its optical isomers, and also addition salts thereof with a pharmaceutically acceptable acid;
- N-{[3,4-dimethoxybicyclo[4.2.0]octa-1,3,5-trien-7-yl]methyl}-3-(7,8-dimethoxy-1,2,4,5-tetrahydro-3*H*-3-benzazepin-3-yl)-4-oxobutan-1-amine, its optical isomers, and also addition salts thereof with a pharmaceutically acceptable acid; and
- 7-{[[3-(7,8-dimethoxy-1,2,4,5-tetrahydro-3*H*-3-benzazepin-3-yl)-3-oxopropyl]-(methyl)amino]methyl}bicyclo[4.2.0]octa-1,3,5-trien-3-yl dimethylcarbamate, its optical isomers, and also addition salts thereof with a pharmaceutically acceptable acid.

12. Process for the synthesis of compounds of formula (I) according to claim 1 starting from a compound of formula (II): 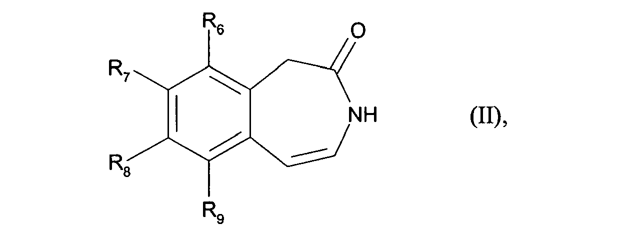 wherein R₆, R₇, R₈ and R₉ are as defined in claim 1,
which is subjected to a hydrogenation reaction to yield the compound of formula (III): 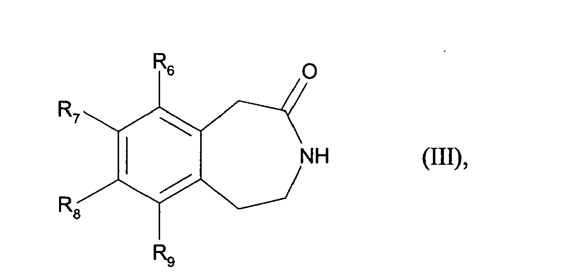 wherein R₆, R₇, R₈ and R₉ are as defined in claim 1,
which is reduced to yield the compound of formula (IV): 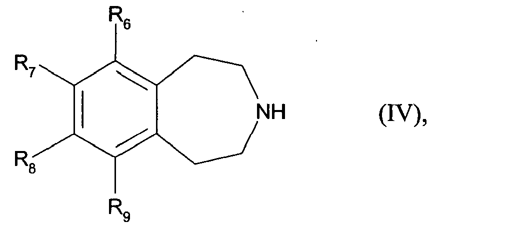 wherein R₆, R₇, R₈ and R₉ are as defined in claim 1,
which is reacted
• either, when it is desired to obtain compounds of formula (I) wherein m represents zero, with acryloyl chloride to yield the compound of formula (V): 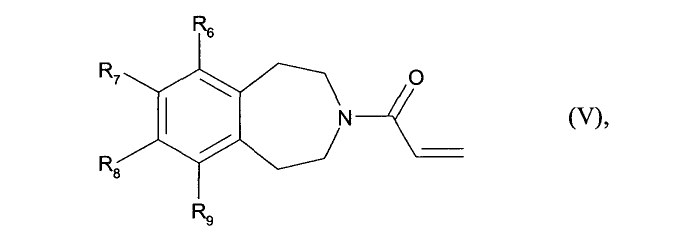 wherein R₆, R₇, R₈ and R₉ are as defined in claim 1,
which is subjected to a coupling reaction with a compound of formula (VI): 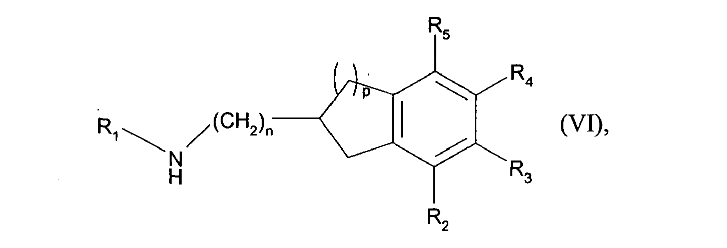 wherein n, p, R₁, R₂, R₃, R₄ and R₅ are as defined in claim 1,
to yield the compounds of formula (Ia), a particular case of compounds of formula (I) wherein m represents zero: 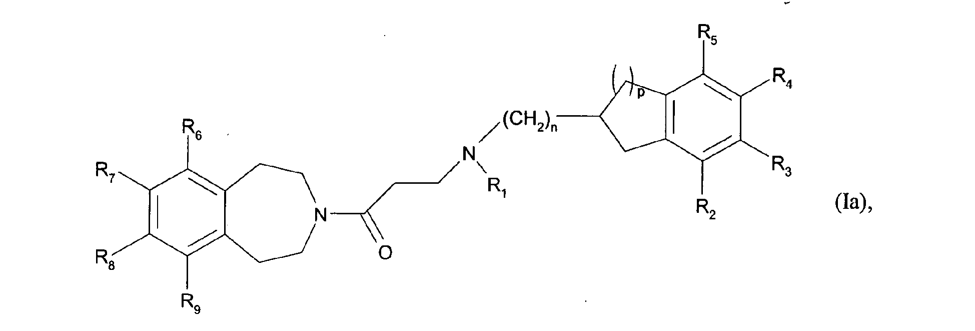 wherein n, p, R₁, R₂, R₃, R₄, R₅, R₆, R₇, R₈ and R₉ are as defined in claim 1,
• or, when it is desired to obtain compounds of formula (I) wherein (X)ₘ represents O or NH, with diphosgene to yield the compound of formula (VII): 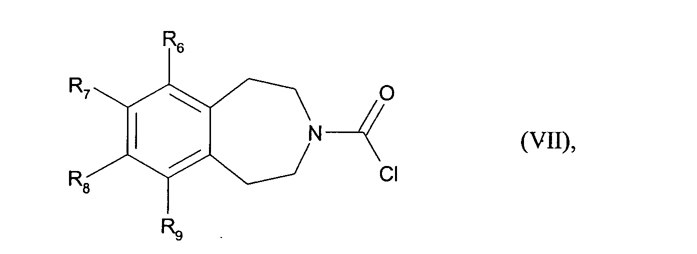 wherein R₆, R₇, R₈ and R₉ are as defined in claim 1,
which is reacted with a compound of formula (VIII): 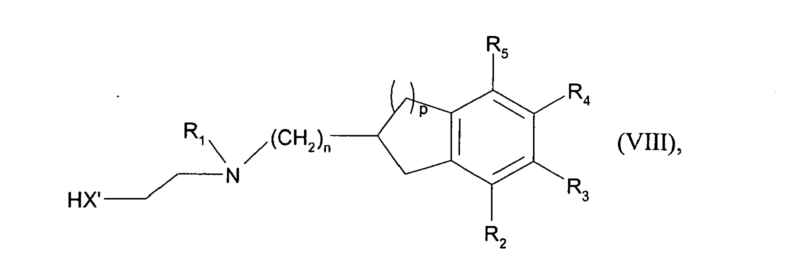 wherein n, p, R₁, R₂, R₃, R₄ and R₅ are as defined in claim 1 and X' represents O or NH,
to yield the compounds of formula (Ib), a particular case of compounds of formula (I) wherein m represents 1 and X represents O or NH: 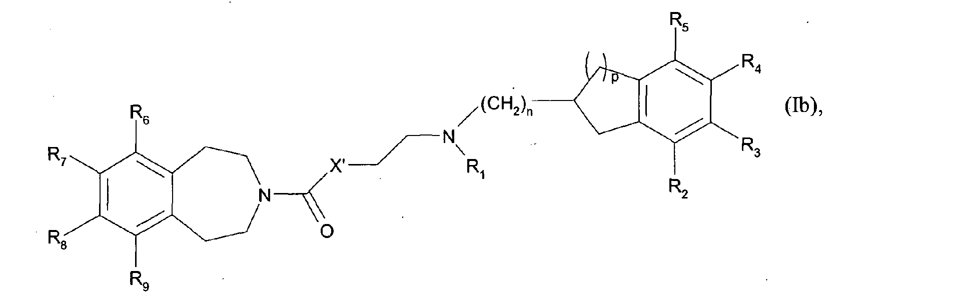 wherein n, p, R1, R₂, R₃, R₄, R₅, R₆, R₇, R₈ and R₉ are as defined in claim 1 and X' represents O or NH,
• or, when it is desired to obtain compounds of formula (I) wherein (X)ₘ represents CH₂, with gamma-butyrolactone to yield the compound of formula (IX): 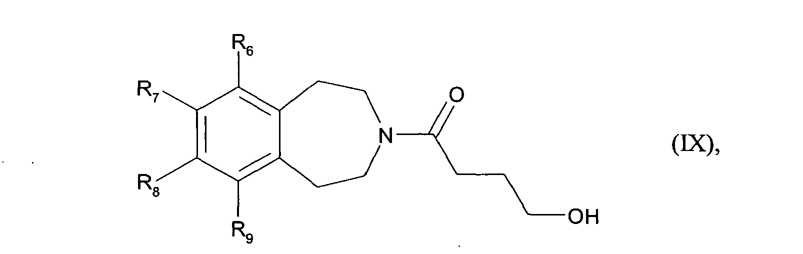
wherein R₆, R₇, R₈ and R₉ are as defined in claim 1,
which is oxidised to form the compound of formula (X): 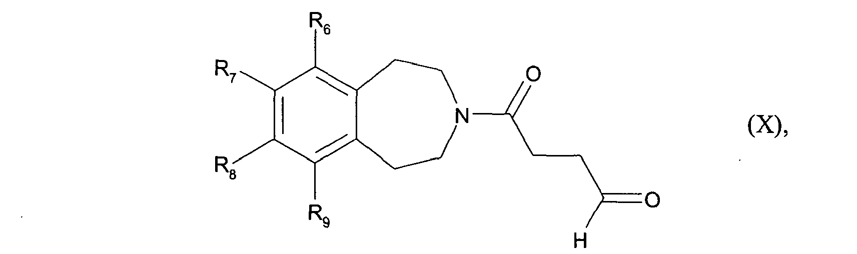 wherein R₆, R₇, R₈ and R₉ are as defined in claim 1,
which is reacted with a compound of formula (VI) to yield the compounds of formula (Ic), a particular case of compounds of formula (I) wherein m represents 1 and X represents CH₂: 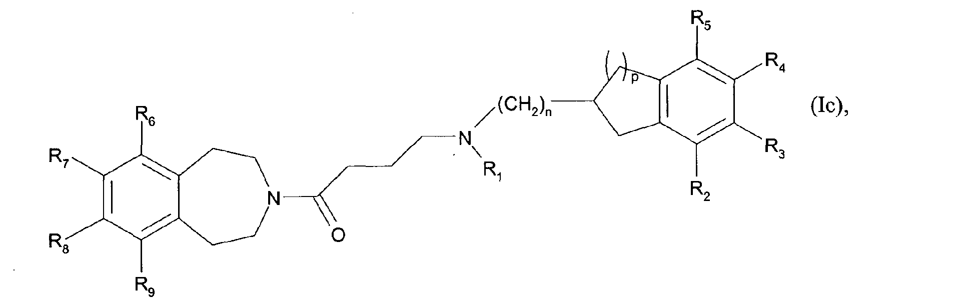 wherein n, p, R₁, R₂, R₃, R₄, R₅, R₆, R₇, R₈ and R₉ are as defined in claim 1.

13. Pharmaceutical composition comprising as active ingredient a compound of formula (I) according to any one of claims 1 to 11, in combination with one or more pharmaceutically acceptable, inert, non-toxic excipients or carriers.

14. Pharmaceutical composition according to claim 13 for use in the treatment or prevention of pathologies in which accelerated heart rate acts as a trigger or has an aggravating role.

15. Pharmaceutical composition according to claim 14 for use in the treatment or prevention of ischaemic cardiopathies, heart failure whether systolic or diastolic and whether in the chronic or acute forms thereof, ventricular or supraventricular rhythm disturbances, or pathologies constituting a vascular risk factor.

16. Pharmaceutical composition according to claim 15 for use in the treatment or prevention of stable angina, unstable angina, threat syndromes, myocardial infarction or post infarction.

17. Pharmaceutical composition according to claim 15 for use in the treatment or prevention of arterial hypertension, diabetes or hypercholesterolaemia.

18. Pharmaceutical composition according to claim 13 for use in the curative or preventative treatment of pain, bladder overactivity or eye dryness sensations.

## Patentansprüche

1. Verbindung der Formel (I): 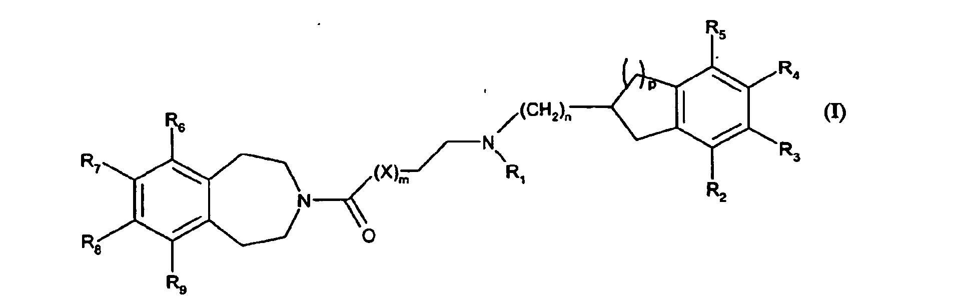 in der:
- R₁ ein Wasserstoffatom oder eine Gruppe ausgewählt aus C₃-C₇-Cycloalkyl, Benzyl und geradkettigem oder verzweigtem C₁-C₆-Alkyl bedeutet, wobei die Alkylgruppe gesättigt oder ungesättigt ist und gegebenenfalls durch eine Hydroxy-, C₃-C₇-Cycloalkylgruppe oder ein oder mehrere Halogenatome substituiert ist,
- R₂, R₃, R₄ und R₅, die identisch oder verschieden sind, jeweils ein Wasserstoffatom oder eine Hydroxygruppe, Methylgruppe, -OSO₂R₁₀, -OCOR₁₀, geradkettige oder verzweigte, gesättigte oder ungesättigte, gegebenenfalls durch eine Methoxygruppe oder -(CO)-N R₁₂R'₁₂ substituierte C₁-C₆-Alkoxygruppe bedeuten oder R₂ und R₃ oder R₃ und R₄ oder R₄ und R₅ gemeinsam eine Gruppe -O-(CH₂)_{q}-O-, -O-CH=CH-O- oder -O-CH=CH- bilden,
- R₆, R₇, R₈ und R₉, die identisch oder verschieden sind, jeweils ein Wasserstoffatom oder eine geradkettige oder verzweigte, gesättigte oder ungesättigte C₁-C₆-Alkoxygruppe bedeuten oder R₆ und R₇ oder R₇ und R₈ oder R₈ und R₉ gemeinsam eine Gruppe -O-(CH₂)_{q}-O- bilden,
- R₁₀ eine Gruppe bedeutet ausgewählt aus geradkettigem oder verzweigtem C₁-C₆-Alkoxy, NR₁₁R'₁₁ und geradkettigem oder verzweigtem, gegebenenfalls durch ein oder mehrere Halogenatome substituiertem C₁-C₆-Alkyl,
- R₁₁ und R'₁₁, die identisch oder verschieden sind, jeweils ein Wasserstoffatom oder eine geradkettige oder verzweigte C₁-C₆-Alkylgruppe bedeuten oder R₁₁ und R'₁₁ gemeinsam mit dem sie tragenden Stickstoffatom einen mono- oder bicyclischen Heterocyclus bilden, der 5 bis 8 Kettenglieder aufweist und gegebenenfalls ein weiteres Heteroatom ausgewählt aus O und N aufweist, wobei der Heterocyclus gegebenenfalls durch ein oder mehrere Halogenatome substituiert ist,
- R₁₂ und R'₁₂, die identisch oder verschieden sind, jeweils ein Wasserstoffatom oder eine geradkettige oder verzweigte C₁-C₆-Alkylgruppe bedeuten,
- X O, NH oder CH₂ bedeutet,
- m und p, die identisch oder verschieden sind, jeweils 0 oder 1 bedeuten,
- n und q, die identisch oder verschieden sind, jeweils 1 oder 2 bedeuten,
ihre optischen Isomeren, falls sie existieren, sowie ihre Additionssalze mit einer pharmazeutisch annehmbaren Säure.

2. Verbindung der Formel (I) nach Anspruch 1, worin R₁ ein Wasserstoffatom oder eine geradkettige oder verzweigte C₁-C₆-Alkylgruppe bedeutet, ihre optischen Isomeren, falls sie existieren, sowie ihre Additionssalze mit einer pharmazeutisch annehmbaren Säure.

3. Verbindung der Formel (I) nach Anspruch 1, worin R₁ eine C₃-C₇-Cycloalkylgruppe oder eine Cycloalkylalkylgruppe, worin der Cycloalkylrest 3 bis 7 Kohlenstoffatome aufweist und der Alkylrest 1 bis 6 Kohlenstoffatome besitzt und geradkettig oder verzweigt ist, bedeutet, ihre optischen Isomeren, falls sie existieren, sowie ihre Additionssalze mit einer pharmazeutisch annehmbaren Säure.

4. Verbindung der Formel (I) nach einem der Ansprüche 1 bis 3, worin R₂, R₃, R₄ und R₅, die identisch oder verschieden sind, jeweils ein Wasserstoffatom oder eine geradkettige oder verzweigte, gesättigte oder ungesättigte C₁-C₆-Alkoxygruppe oder eine Gruppe der Formel -OCOR₁₀ bedeuten, worin R₁₀ eine Gruppe NR₁₁R'₁₁ darstellt, wie sie in Anspruch 1 definiert ist, ihre optischen Isomeren, falls sie existieren, sowie ihre Additionssalze mit einer pharmazeutisch annehmbaren Säure.

5. Verbindung der Formel (I) nach einem der Ansprüche 1 bis 4, worin R₆, R₇, R₈ und R₉, die identisch oder verschieden sind, jeweils ein Wasserstoffatom oder eine geradkettige oder verzweigte, gesättigte oder ungesättigte C₁-C₆-Alkoxygruppe bedeuten, ihre optischen Isomeren, falls sie existieren, sowie ihre Additionssalze mit einer pharmazeutisch annehmbaren Säure.

6. Verbindung der Formel (I) nach einem der Ansprüche 1 bis 5, worin m den Wert 0 besitzt, ihre optischen Isomeren, falls sie existieren, sowie ihre Additionssalze mit einer pharmazeutisch annehmbaren Säure.

7. Verbindung der Formel (I) nach einem der Ansprüche 1 bis 5, worin m 1 und X CH₂ bedeuten, ihre optischen Isomeren, falls sie existieren, sowie ihre Additionssalze mit einer pharmazeutisch annehmbaren Säure.

8. Verbindung der Formel (I) nach einem der Ansprüche 1 bis 7, worin p 0 bedeutet, ihre optischen Isomeren, falls sie existieren, sowie ihre Additionssalze mit einer pharmazeutisch annehmbaren Säure.

9. Verbindung der Formel (I) nach einem der Ansprüche 1 bis 7, worin p 1 bedeutet, ihre optischen Isomeren, falls sie existieren, sowie ihre Additionssalze mit einer pharmazeutisch annehmbaren Säure.

10. Verbindung der Formel (I) nach Anspruch 1, worin R₁ ein Wasserstoffatom oder eine geradkettige oder verzweigte C₁-C₆-Alkylgruppe bedeutet, R₂, R₃, R₄ und R₅, die identisch oder verschieden sind, jeweils ein Wasserstoffatom oder eine geradkettige oder verzweigte, gesättigte oder ungesättigte C₁-C₆-Alkoxygruppe bedeuten, R₆, R₇, R₈ und R₉, die identisch oder verschieden sind, jeweils ein Wasserstoffatom oder eine geradkettige oder verzweigte, gesättigte oder ungesättigte C₁-C₆-Alkoxygruppe bedeuten, m 0 bedeutet, n 1 bedeutet und p 0 bedeutet, ihre optischen Isomeren sowie ihre Additionssalze mit einer pharmazeutisch annehmbaren Säure.

11. Verbindung der Formel (I) nach Anspruch 1, ausgewählt aus:
- N-{[3,4-Dimethoxybicyclo[4.2.0]octa-1,3-5-trien-7-yl]-methyl}-3-(7,8-dimethoxy-1,2,4,5-tetrahydro-3H-3-benzazepin-3-yl)-N-methyl-3-oxopropan-1-amin, seine optischen Isomeren sowie seine Additionssalze mit einer pharmazeutisch annehmbaren Säure;
- N-{[3,4-Dimethoxybicyclo[4.2.0]octa-1,3,5-trien-7-yl]-methyl}-3-(7,8-dimethoxy-1,2,4,5-tetrahydro-3H-3-benzazepin-3-yl)-3-oxopropan-1-amin, seine optischen Isomeren sowie seine Additionssalze mit einer pharmazeutisch annehmbaren Säure;
- N-[2-(5,6-Dimethoxy-2,3-dihydro-1*H*-inden-2-yl)-methyl]-3-(7,8-dimethoxy-1,2,4,5-tetrahydro-3H-3-benzazepin-3-yl)-3-oxopropan-1-amin sowie seine Additionssalze mit einer pharmazeutisch annehmbaren Säure;
- N-{[3,4-Dimethoxybicyclo[4.2.0]octa-1,3,5-trien-7-yl]-methyl}-3-(7,8-dimethoxy-1,2,4,5-tetrahydro-3H-3-benzazepin-3-yl)-N-methyl-4-oxobutan-1-amin, seine optischen Isomeren sowie seine Additionssalze mit einer pharmazeutisch annehmbaren Säure;
- N-{[3,4-Dimethoxybicyclo[4.2.0]octa-1,3,5-trien-7-yl]-methyl}-3-(7,8-dimethoxy-1,2,4,5-tetrahydro-3H-3-benzazepin-3-yl)-4-oxobutan-1-amin, seine optischen Isomeren sowie seine Additionssalze mit einer pharmazeutisch annehmbaren Säure; und
- 7-{[[3-(7,8-Dimethoxy-1,2,4,5-tetrahydro-3*H*-3-benzazepin-3-yl)-3-oxopropyl]-(methyl)-amino]-methyl}-bicyclo[4.2.0]octa-1,3,5-trien-3-yl-dimethylcarbamat, seine optischen Isomeren sowie seine Additionssalze mit einer pharmazeutisch annehmbaren Säure.

12. Verfahren zur Synthese der Verbindungen der Formel (I) nach Anspruch 1, ausgehend von einer Verbindung der Formel (II): 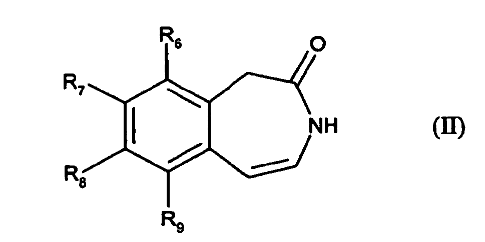 in der R₆, R₇, R₈ und R₉ die in Anspruch 1 angegebenen Bedeutungen besitzen, welche man einer Hydrierungsreaktion unterwirft zur Bildung der Verbindung der Formel (III): 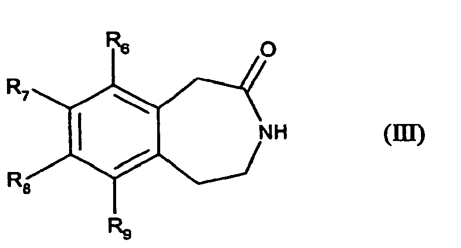 in der R₆, R₇, R₈ und R₉ die in Anspruch 1 angegebenen Bedeutungen besitzen, welche man reduziert zur Bildung der Verbindung der Formel (IV): 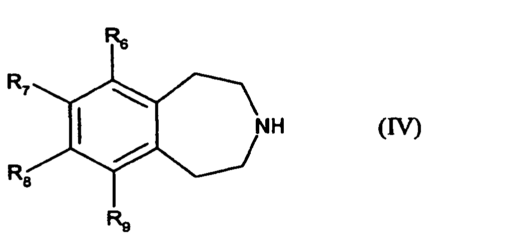 in der R₆, R₇, R₈ und R₉ die in Anspruch 1 angegebenen Bedeutungen besitzen, welche man:
• entweder, wenn man die Verbindungen der Formel (I) erhalten will, worin m Null bedeutet, mit Acryloylchlorid umsetzt zur Bildung der Verbindung der Formel (V): 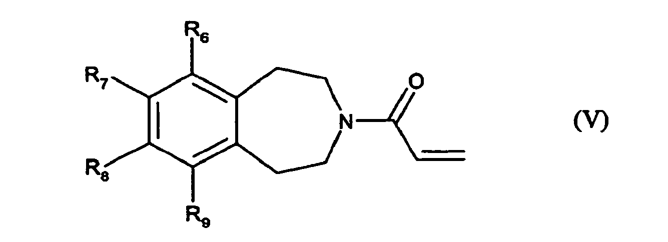 in der R₆, R₇, R₈ und R₉ die in Anspruch 1 angegebenen Bedeutungen besitzen,
die man einer Kupplungsreaktion mit einer Verbindung der Formel (VI) unterwirft: 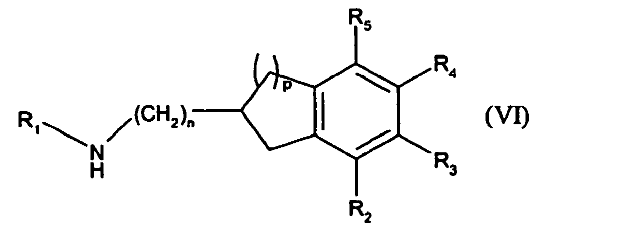 in der n, p, R₁, R₂, R₃, R₄ und R₅ die in Anspruch 1 angegebenen Bedeutungen besitzen,
zur Bildung der Verbindungen der Formel (Ia), einem Sonderfall der Verbindungen der Formel (I), worin m Null bedeutet: 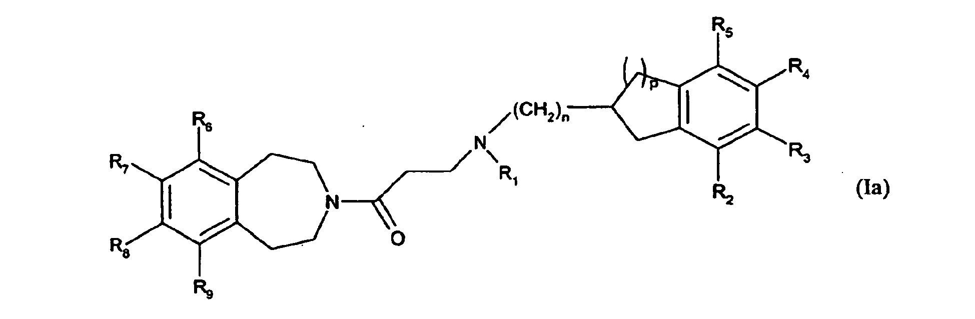 in der n, p, R₁, R₂, R₃, R₄, R₅, R₆, R₇, R₈ und R₉ die in Anspruch 1 angegebenen Bedeutungen besitzen,
• oder, wenn man die Verbindungen der Formel (I) erhalten will, worin (X)ₘ O oder NH bedeutet, mit Diphosgen umsetzt zur Bildung der Verbindung der Formel (VII): 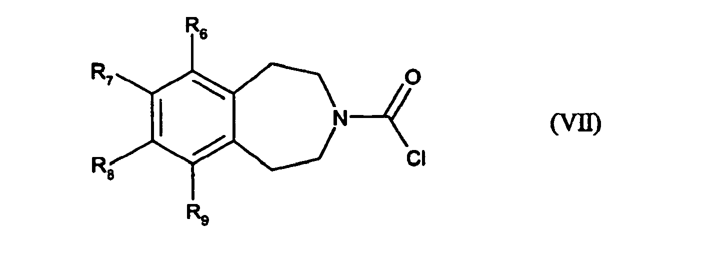 in der R₆, R₇, R₈ und R₉ die in Anspruch 1 angegebenen Bedeutungen besitzen,
welche man mit einer Verbindung der Formel (VIII) umsetzt: 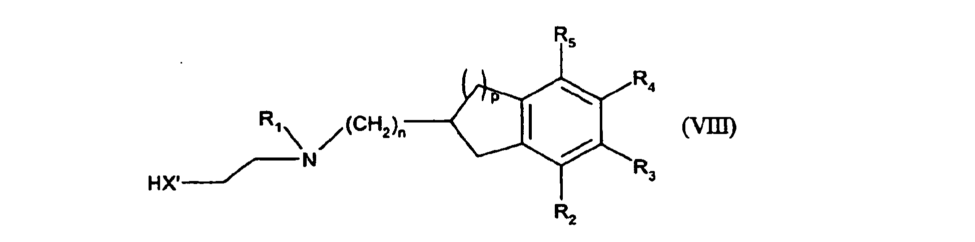 in der n, p, R₁, R₂, R₃, R₄ und R₅ die in Anspruch 1 angegebenen Bedeutungen besitzen und X' O oder NH bedeutet,
zur Bildung der Verbindungen der Formel (Ib), einem Sonderfall der Verbindungen der Formel (I), worin m 1 und X O oder NH bedeuten: 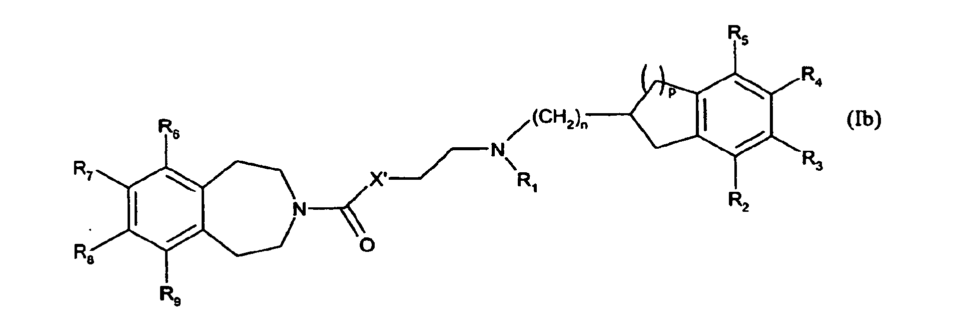 in der n, p, R₁, R₂, R₃, R₄, R₅, R₆, R₇, R₈ und R₉ die in Anspruch 1 angegebenen Bedeutungen besitzen und X' O oder NH bedeutet,
• oder, wenn man die Verbindungen der Formel (I) erhalten will, worin (X)ₘ CH₂ bedeutet, mit Gamma-Butyrolacton umsetzt zur Bildung der Verbindung der Formel (IX): 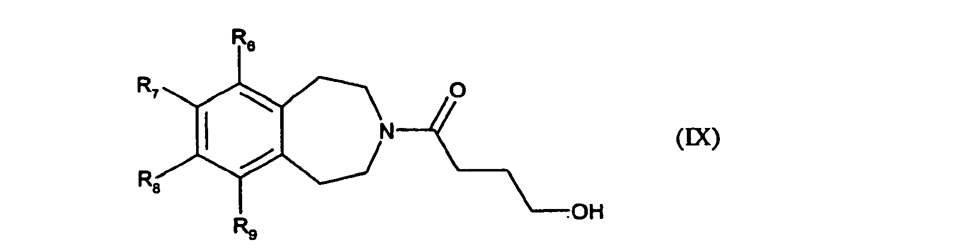 in der R₆, R₇, R₈ und R₉ die in Anspruch 1 angegebenen Bedeutungen besitzen,
welche zu der Verbindung der Formel (X) oxidiert wird: 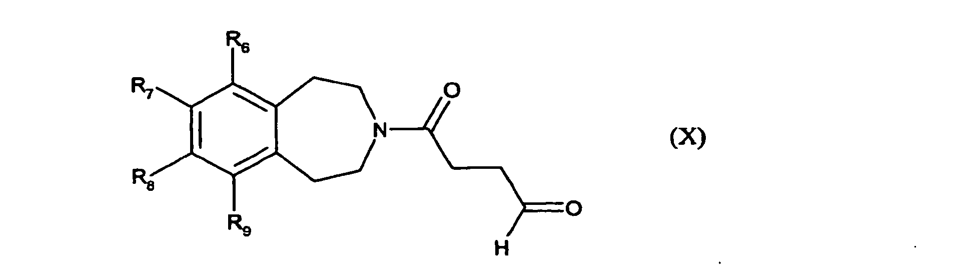 in der R₆, R₇, R₈ und R₉ die in Anspruch 1 angegebenen Bedeutungen besitzen,
die man mit einer Verbindung der Formel (VI) umsetzt zur Bildung der Verbindungen der Formel (Ic), einem Sonderfall der Verbindungen der Formel (I),
worin m 1 und X CH₂ bedeuten: 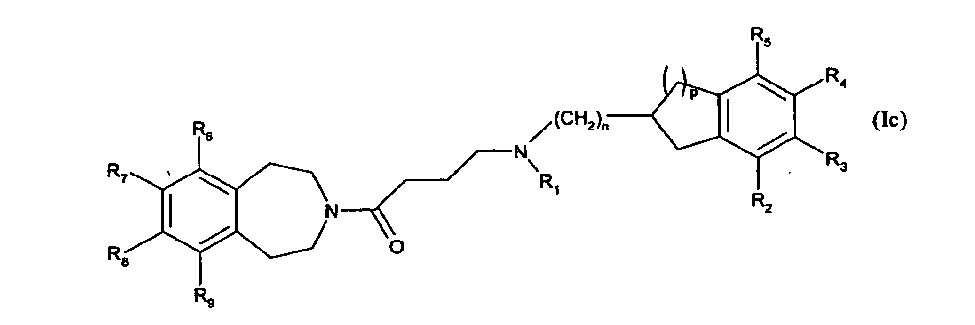 in der n, p, R₁, R₂, R₃, R₄, R₅, R₆, R₇, R₈ und R₉ die in Anspruch 1 angegebenen Bedeutungen besitzen.

13. Pharmazeutische Zubereitung, enthaltend als Wirkstoff eine Verbindung der Formel (I) nach einem der Ansprüche 1 bis 11 in Kombination mit einem oder mehreren inerten, nichttoxischen, pharmazeutisch annehmbaren Trägermaterialien oder Hilfsstoffen.

14. Pharmazeutische Zubereitung nach Anspruch 13 zur Behandlung oder Vorbeugung von Erkrankungen, bei denen eine Beschleunigung der Herzfrequenz eine auslösende oder erschwerende Rolle spielt.

15. Pharmazeutische Zubereitung nach Anspruch 14 zur Behandlung oder Vorbeugung von ischämischen Kardiopathien, systolischen oder diastolischen Herzinsuffizienzen in chronischer oder akuter Form, ventrikulären oder supraventrikulären Rhythmusstörungen oder Erkrankungen, die einen Gefäßrisikofaktor bilden.

16. Pharmazeutische Zubereitung nach Anspruch 15 zur Behandlung oder Vorbeugung von stabiler Angina pectoris, instabiler Angina pectoris, von Bedrohungs-Syndromen, Myokardinfarkt oder Postinfarkt.

17. Pharmazeutische Zubereitung nach Anspruch 15 zur Behandlung oder Vorbeugung der arteriellen Hypertension, des Diabetes oder der Hypercholesterolämie.

18. Pharmazeutische Zubereitung nach Anspruch 13 zur heilenden oder vorbeugenden Behandlung von Schmerzen, hyperaktiver Blase oder dem Gefühl des trockenen Auges.
